# EUROPÄISCHE PATENTSCHRIFT

(11) **EP 2 058 323 B1**
(45) Veröffentlichungstag und Bekanntmachung des Hinweises auf die Patenterteilung: **16.05.2012**
(21) Anmeldenummer: 09000315.3
(22) Anmeldetag: 24.05.2005
(51) Int. Cl.: C07K 7/06, C07K 14/47

(54) **An MHC-Moleküle bindende Tumor-assoziierte Peptide**
Tumour-associated peptides which bind to MHC molecules
Peptides spécifiques aux tumeurs se fixant sur des molécules CMH

(30) Priorität: 25.05.2004 DE 102004026135
(43) Veröffentlichungstag der Anmeldung: 13.05.2009
(62) Teilanmeldung aus: 05752606.3
(73) Patentinhaber: Immatics Biotechnologies GmbH, 72076 Tübingen (DE)
(72) Erfinder: Trautwein, Claudia, 42489 Wülfrath (DE); Rammensee, Hans-Georg, 72076 Tübingen (DE); Stevanovic, Stefan, 72076 Tübingen (DE); Weinschenk, Toni, 73773 Aichwald (DE)
(74) Vertreter: Krauss, Jan

(56) Entgegenhaltungen:
- DE-A1- 10 225 144
- US-A1- 2005 063 967
- LEMMEL, CLAUDIA: "Dissertation: Qualitative und quantitative Analyse krankheitsassoziierter MHC-Klasse-I-Liganden durch massenspektrometrische Verfahren" [Online] 24. Juni 2004 (2004-06-24), , XP002344158 Gefunden im Internet: URL:http://w210.ub.uni-tuebingen.de/dbt/vo lltexte/2004/1287/pdf/Lemmel_Dissertation. pdf> [gefunden am 2005-09-08] * Seite 119, letzte Zeile * Peptid VPDSSGPERIL * Seite 80; Tabelle 3.16 *
- LEMMEL C ET AL: "Differential quantitative analysis of MHC ligands by mass spectrometry using stable isotope labeling" NATURE BIOTECHNOLOGY, NATURE PUBLISHING GROUP, NEW YORK, NY, US, Bd. 22, Nr. 4, 1. April 2004 (2004-04-01), Seiten 450-454, XP002359085 ISSN: 1087-0156
- PLUSCHKE G ET AL: "Molecular cloning of a human melanoma-associated chondroitin sulfate proteoglycan" PROCEEDINGS OF THE NATIONAL ACADEMY OF SCIENCES OF USA, NATIONAL ACADEMY OF SCIENCE, WASHINGTON, DC.; US, Bd. 93, Nr. 18, 1. September 1996 (1996-09-01), Seiten 9710-9715, XP003019023 ISSN: 0027-8424
- DATABASE Geneseq [Online] 5. November 2001 (2001-11-05), "Human brain expressed single exon probe encoded protein SEQ ID NO: 25597." XP002521875 gefunden im EBI accession no. GSP:AAM53492 Database accession no. AAM53492
- DATABASE Geneseq [Online] 27. Februar 2002 (2002-02-27), "Propionibacterium acnes immunogenic protein #19260." XP002521876 gefunden im EBI accession no. GSP:AAU58364 Database accession no. AAU58364
- FLAD T ET AL: "DIRECT IDENTIFICATION OF MAJOR HISTOCOMPATIBILITY COMPLEX CLASS I-BOUND TUMOR-ASSOCIATED PEPTIDE ANTIGENS OF A RENAL CARCINOMA CELL LINE BY A NOVEL MASS SPECTROMETRIC METHOD" CANCER RESEARCH, AMERICAN ASSOCIATION FOR CANCER RESEARCH, BALTIMORE, MD, US, Bd. 58, Nr. 24, 15. Dezember 1998 (1998-12-15), Seiten 5803-5811, XP001161018 ISSN: 0008-5472
- KRÜGER TOBIAS ET AL: "Lessons to be learned from primary renal cell carcinomas: novel tumor antigens and HLA ligands for immunotherapy." CANCER IMMUNOLOGY, IMMUNOTHERAPY : CII. SEP 2005, Bd. 54, Nr. 9, September 2005 (2005-09), Seiten 826-836, XP002344048 ISSN: 0340-7004
- STEVANOVIC STEFAN ET AL: "Generating data for databases--the peptide repertoire of HLA molecules" NOVARTIS FOUNDATION SYMPOSIUM, WILEY, CHICHESTER, GB, Bd. 254, 1. Januar 2003 (2003-01-01), Seiten 143-252, XP009105681 ISSN: 1528-2511
- SCHIRLE MARKUS ET AL: "Identification of tumor-associated MHC class I ligands by a novel T cell-independent approach" EUROPEAN JOURNAL OF IMMUNOLOGY, WEINHEIM, DE, Bd. 30, Nr. 8, August 2000 (2000-08), Seiten 2216-2225, XP002246625 ISSN: 0014-2980
- MELIEF CORNELIS J M ET AL: "Peptide-based cancer vaccines" CURRENT OPINION IN IMMUNOLOGY, Bd. 8, Nr. 5, 1996, Seiten 651-657, XP002344045 ISSN: 0952-7915
- PURCELL A W ET AL: "Immunoproteomics: Mass spectrometry-based methods to study the targets of the immune response." MOLECULAR & CELLULAR PROTEOMICS, Bd. 3, Nr. 3, März 2004 (2004-03), Seiten 193-208, XP002344046 ISSN: 1535-9476

## Beschreibung

Die vorliegende Erfindung betrifft Tumor-assoziierte Peptide, die die Fähigkeit aufweisen, an ein Molekül des menschlichen Haupt-Histokompatibilitäts-Komplexes (MHC), Klasse I, zu binden.

Derartige Peptide werden bspw. in der Immuntherapie von Tumorerkrankungen eingesetzt.

Bei der Eliminierung von Tumorzellen durch das Immunsystem spielt die Erkennung von Tumor-assoziierten Antigenen (TAA) durch Komponenten des Immunsystems eine herausragende Rolle. Diesem Mechanismus liegt die Voraussetzung zugrunde, dass zwischen Tumorzellen und normalen Zellen qualitative oder quantitative Unterschiede bestehen. Um eine anti-Tumor-Antwort herbeizuführen, müssen die Tumorzellen Antigene exprimieren, gegen welche eine immunologische Antwort erfolgt, die für die Eliminierung des Tumors ausreicht.

Beteiligt bei der Abstoßung von Tumoren sind insbesondere CD8-exprimierende zytotoxische T-Lymphozyten (im folgenden CTL). Zur Auslösung einer derartigen Immunreaktion durch zytotoxische T-Zellen müssen den T-Zellen dazu fremde Proteine/Peptide präsentiert werden. T-Zellen erkennen Antigene als Peptidfragmente nur dann, wenn diese an Zelloberflächen von MHC-Molekülen präsentiert werden. Diese MHC-Moleküle ("major histocompatibility complex") sind Peptidrezeptoren, die normalerweise Peptide innerhalb der Zelle binden, um sie zu der Zelloberfläche zu transportieren. Dieser Komplex aus Peptid und MHC-Molekül kann durch die T-Zellen erkannt werden. Die MHC-Moleküle des Menschen werden auch als humane Leukozyten-Antigene (HLA) bezeichnet.

Es gibt zwei Klassen von MHC-Molekülen: MHC-Klasse-I-Moleküle, die auf den meisten Zellen mit Kern vorkommen, präsentieren Peptide, die durch proteolytischen Abbau endogener Proteine entstehen. MHC-Klasse-II-Moleküle kommen nur auf professionellen Antigen-präsentierenden Zellen (APC) vor und präsentieren Peptide exogener Proteine, die im Verlauf der Endozytose von APC aufgenommen und verarbeitet werden. Komplexe aus Peptid und MHC-Klasse-I werden von CD8-positiven zytotoxischen T-Lymphozyten erkannt, Komplexe aus Peptid und MHC-Klasse-II werden von CD4-Helfer-T-Zellen erkannt.

Damit ein Peptid eine zelluläre Immunantwort auslösen kann, muss es an ein MHC-Molekül binden. Dieser Vorgang ist vom Allel des MHC-Moleküls und von der Aminosäuresequenz des Peptids abhängig. MHC-Klasse-I-bindende Peptide sind in der Regel 8-10 Reste lang und enthalten in ihrer Sequenz zwei konservierte Reste ("Anker"), die mit der entsprechenden Bindungsfurche des MHC-Moleküls interagieren.

Damit das Immunsystem eine effektive CTL-Antwort gegen Tumor-abgeleitete Peptide starten kann, müssen diese Peptide nicht nur in der Lage sein, an die bestimmten MHC-Klasse-I-Moleküle zu binden, die von den Tumorzellen exprimiert werden, sondern sie müssen auch von T-Zellen mit spezifischen T-Zell-Rezeptoren (TCR, "T-cell receptor") erkannt werden.

Das Hauptziel zur Entwicklung einer Tumorvakzine ist die Identifizierung und Charakterisierung von Tumor-assoziierten Antigenen, die durch CD8⁺ CTL erkannt werden.

Die Antigene, die von den Tumor-spezifischen zytotoxischen T-Lymphozyten erkannt werden, bzw. deren Epitope, können Moleküle aus sämtlichen Proteinklassen sein, wie z.B. Enzyme, Rezeptoren, Transkriptionsfaktoren, etc. Eine andere wichtige Klasse Tumor-assoziierter Antigene sind Gewebe-spezifische Strukturen, wie bspw. CT ("cancer testis")-Antigene, die in verschiedenen Tumorarten und in gesundem Hodengewebe exprimiert werden.

Damit die Proteine durch die zytotoxischen T-Lymphozyten als Tumor-spezifisches Antigen erkannt werden, und um somit in einer Therapie eingesetzt werden zu können, müssen bestimmte Voraussetzungen vorliegen: Das Antigen soll hauptsächlich von Tumorzellen exprimiert werden und von Normalgeweben nicht oder nur in geringeren Mengen als in den Tumoren. Weiterhin ist wünschenswert, wenn das betreffende Antigen nicht nur in einer Tumorart, sondern auch in weiteren in hoher Konzentration vorliegt. Unbedingt essentiell ist auch das Vorliegen von Epitopen in der Aminosäuresequenz des Antigens, da solche von einem Tumor-assoziierten Antigen abgeleiteten Peptide ("immunogene Peptide") zu einer T-Zell-Antwort führen sollen, sei es *in vitro* oder *in vivo.*

TAAs stellen somit ein Ausgangspunkt für die Entwicklung einer Tumorvakzine dar. Die Methoden zur Identifizierung und Charakterisierung der TAAs beruhen zum einen auf dem Einsatz von in Patienten bereits induzierten CTL oder basieren auf der Erstellung differentieller Transkriptionsprofile zwischen Tumoren und Normalgeweben.

Das Auffinden von Genen, die in Tumorgeweben oder humanen Tumor-Zelllinien überexprimiert sind, oder die in derartigen Geweben oder Zelllinien selektiv exprimiert werden, liefert jedoch keine präzise Information für einen Einsatz der von diesen Genen transkribierten Antigene in der Immuntherapie. Dies hat die Ursache darin, dass jeweils nur einzelne Epitope dieser Antigene für einen derartigen Einsatz geeignet sind, da nur die Epitope der Antigene - nicht das gesamte Antigen - durch MHC-Präsentierung eine T-Zell-Antwort hervorrufen. Daher ist es wichtig, diejenigen Peptide von überexprimierten oder selektiv exprimierten Proteinen zu selektieren, die mit MHC-Molekülen präsentiert werden, wodurch Angriffspunkte für die spezifische Erkennung von primären Zellen oder von aus primären Tumorgewebszellen etablierten Tumor-Zelllinien durch zytotoxische T-Lymphozyten gewonnen werden könnten.

DE 102 25 144 A1 offenbart die Identifizierung von Tumor-assoziierten Peptiden anders als SEQ ID Nr. 303, die die Fähigkeit aufweisen, an ein Molekül des menschlichen MHC Klasse I zu binden.

Pluschke et al. (in Pluschke et al., Molecular cloning of a human melanoma-associated chondroitin sulfate proteoglycan" PNAS, Bd. 93, Nr. 18, 01.09.1996, Seiten 9710-9715) offenbaren Chondroitin sulfate proteoglycan 4 (CSPG4) umfassend die Sequenz TMLARLASA (SEQ ID Nr. 303). Eine entsprechende immunologische Verwendung als Tumor-assoziiertes Peptid wird nicht offenbart.

Vor diesem Hintergrund ist es Aufgabe der vorliegenden Erfindung, mindestens eine neue Aminosäuresequenz für ein derartiges Peptid bereitzustellen, welches die Fähigkeit aufweist, an ein Molekül des menschlichen Haupt-Histokompatibilitäts-Komplexes (MHC)-Klasse-I zu binden.

Diese Aufgabe wird erfindungsgemäß gelöst durch die Bereitstellung eines Tumor-assoziierten Peptids, wobei das Peptid die Fähigkeit aufweist, an ein Molekül des menschlichen Haupt-Histokompatibilitäts-Komplexes (MHC) Klasse-I zu binden, ausgewählt aus, a) einer Aminosäuresequenz bestehend aus SEQ ID Nr. 303 (TMLARLASA) aus dem beiliegenden Sequenzprotokoll, b) einem Peptid gemäß a), wobei eine Aminosäure durch eine andere Aminosäure mit ähnlichen chemischen Eigenschaften ersetzt ist, und c) einem Peptid gemäß a) oder b), wobei N- oder/und C-terminal eine weitere Aminosäure vorhanden ist.

Die der Erfindung zugrunde liegende Aufgabe wird auf diese Weise vollkommen gelöst.

Dabei versteht sich, dass die vom Tumor identifizierten Peptide zur Gewinnung größerer Mengen und zum Einsatz für die unten aufgeführten Zwecke synthetisiert oder in Zellen zur Expression gebracht werden.

Die Erfinder konnten die oben genannten Peptide als spezifische Liganden von MHC-Klasse-I-Molekülen aus Tumorgewebe isolieren und identifizieren. Dabei werden hierin mit dem Begriff "Tumor-assoziiert" Peptide bezeichnet, die aus Tumormaterial isoliert und identifiziert wurden. Diese Peptide, die auf echten (primären) Tumoren präsentiert werden, unterliegen also der Antigenprozessierung in einer Tumor-Zelle.

Die spezifischen Liganden können in der Krebstherapie eingesetzt werden, z.B. um eine Immunantwort gegen Tumorzellen zu induzieren, die die entsprechenden Antigene exprimieren, von denen die Peptide abstammen.

Eine solche Immunantwort in Form einer Induktion von CTL kann zum einen *in vivo* erreicht werden. Dazu wird einem Patienten, der an einer mit dem TAA assoziierten Tumorerkrankung leidet, das Peptid bspw. in Form einer pharmazeutischen Zusammensetzung verabreicht.

Andererseits kann eine CTL-Antwort auf einen Tumor, der die Antigene, von denen die Peptide abstammen, exprimiert, auch *ex vivo* ausgelöst werden. Dazu inkubiert man die CTL-Vorläuferzellen zusammen mit Antigen-präsentierenden Zellen und den Peptiden. Anschließend kultiviert man die dadurch stimulierten CTL und verabreicht diese aktivierten CTL dem Patienten.

Weiterhin besteht die Möglichkeit, APC *ex vivo* mit den Peptiden zu beladen und diese beladenen APC dem Patienten zu verabreichen, der im Tumorgewebe das Antigen exprimiert, von dem das Peptid abstammt. Die APC können dann wiederum *in vivo* den CTL das Peptid präsentieren und sie aktivieren.

Die erfindungsgemäßen Peptide können aber auch als diagnostische Reagenzien eingesetzt werden.

So kann mit den Peptiden herausgefunden werden, ob in einer CTL-Population spezifisch gegen ein Peptid gerichtete CTL vorliegen oder durch eine Therapie induziert wurden.

Außerdem kann mit den Peptiden die Zunahme von Vorläufer T-Zellen getestet werden, die eine Reaktivität gegen das definierte Peptid aufweisen.

Ferner kann das Peptid als Marker dazu verwendet werden, um den Krankheitsverlauf eines Tumors zu verfolgen, der das Antigen exprimiert, von dem das Peptid abstammt.

In der beigefügten Tabelle 1 sind die identifizierten Peptide aufgeführt. In der Tabelle sind außerdem die Proteine angegeben, von denen die Peptide abstammen und die jeweilige Positionen der Peptide in den betreffenden Proteinen, die durch anerkannte Gensymbole gemäß dem "HUGO Gene Nomenclature Committee" (http://www.gene.ucl.ac.uk/nomenclature/ bzw. http://www.ncbi.nlm.nih.gov/LocusLink/) benannt bzw. abgekürzt werden. Dabei sind die englischen Bezeichnungen der Proteine beibehalten worden, um missverständliche Übersetzungen zu vermeiden. Ferner sind jeweils die Acc-Nummern angegeben, die in der Genbank des "National Center for Biotechnology Information" des National Institute of Health geführt werden (siehe http:\\www.ncbi.nlm.nih.gov).

Die Erfinder konnten die Peptide (oder Liganden) aus 8 Nierenzelltumoren, und 2 Glioblastomen von zusammen 10 Patienten, RCC75, RCC98, RCC100, RCC103, RCC112, RCC115, RCC116, RCC130 und NCH359, sowie NCH361, sowie aus einer Tumorzelllinie (J-Y) isolieren.

Aus den Tumoren der Patienten und der Zelllinie J-Y konnten 577 Liganden identifiziert werden, welche an die HLA-Subtypen A*03, B*07, B*40 (RCC75), A*01, A*03, B*07, B*18 (RCC98), A*02, A*03, B*07, B*18 (RCC100), A*11, A*25, B*15, B*44 (RCC103), A*01, A*31, B*08, B*27 (RCC112), A*02, A*03, B*15, B*18 (RCC115), A*01, A*02, B*27, B*37 (RCC116), A*02, A*24, B*07, B*44 (RCC130), A*03, A*32, B*07, B*35 (NCH359), A*26, B*38 (NCH361) und A*02, B*07 (J-Y) gebunden waren.

Einige der Liganden stammten von stark exprimierten sogenannten "Housekeeping" Genen ab, die in den meisten Geweben gleichmäßig exprimiert werden, viele zeichneten sich aber durch gewebespezifische und tumorspezifische Expression aus.

So konnten einige Peptide identifiziert werden, die von Proteinen abstammen, die insbesondere in Tumorgewebe überexprimiert werden. So konnten bspw. Fragmente von Tenascin-C (GLAPSIRTK, SEQ ID-Nr. 2;) identifiziert werden. (Herold-Mende et al., Clinical Impact and Functional Aspects of Tenascin-C Expression during Glioma Progression, 2002, Int. J. Cancer, 98: 362-369)

Die Erfinder konnten außerdem u.a. Liganden identifizieren, die von SOX9, (YPHLHNAEL, SEQ ID-Nr. 7) und RGS5 (LAALPHSCL, SEQ ID-Nr. 448), abstammen.

Da primäre Tumorzellen sich nicht für die Kultivierung *in vitro* eignen, wählten die Erfinder beispielhaft eine humane Tumor-Zelllinie, um zusätzlich zu demonstrieren, dass sich von dieser Zelllinie identifizierte Peptide dazu eignen, *in vitro* zytotoxische T-Lymphozyten zu aktivieren. Im speziellen konnten die Erfinder zeigen, dass es unter Verwendung eines beispielhaft ausgewählten Peptids von der etablierten Tumor-Zelllinie JY möglich war, *in vitro* zytotoxische T-Lymphozyten (CTL) zu generieren, die spezifisch für das ausgewählte Peptid mit der Sequenz FPSLREAAL (MAGEA1, Position 294-302) und der SEQ ID-Nr. 114 und das HLA-Allel B*0702 waren. Mit diesen CTL konnten gezielt KM22-Zielzellen, welche mit dem Peptid mit der SEQ ID-Nr. 114 beladen worden waren, abgetötet werden. Dahingegen wurden die zur Kontrolle verwendeten, nicht mit dem Peptid mit der SEQ ID-Nr. 114 beladenen KM22-Zielzellen von den zytotoxischen T-Zellen nicht erkannt. Somit konnte beispielhaft gezeigt werden, dass mit den Peptiden als Epitope humane T-Zellen *in vitro* aktiviert werden konnten. Ferner konnte gezeigt werden, dass die zytotoxischen T-Lymphozyten, welche die mit dem Peptid mit der SEQ ID-Nr. 114 beladenen T2-Zellen lysierten, auch Interferon-gamma exprimieren, welches als zuverlässiger Marker für die Aktivierung von T-Zellen beschrieben worden ist.

In einer bevorzugten Ausführungsform kann ein Peptid zur Stimulierung einer Immunantwort eingesetzt werden, das die Sequenz ID-Nr. 303 aufweist und bei denen zumindest eine Aminosäure durch eine andere Aminosäure mit ähnlichen chemischen Eigenschaften ersetzt ist.

Dies sind mit Bezug auf die jeweiligen MHC-Subtypen bspw. die Ankeraminosäuren, die durch Aminosäuren mit ähnlichen chemischen Eigenschaften ersetzt werden können. So kann bspw. bei Peptiden, die mit dem MHC-Subtyp HLA-A*02 assoziiert sind, an Position 2 Leucin mit Isoleucin, Valin oder mit Methionin und umgekehrt ausgetauscht werden, und am C-Terminus Leucin mit Valin, Isoleucin und Alanin, die allesamt unpolare Seitenketten aufweisen.

Weiterhin ist es möglich, ein Peptid mit der Sequenz ID-Nr. 303 einzusetzen, das N-oder/und C-terminal zumindest eine weitere Aminosäure aufweist oder bei dem zumindest eine Aminosäure deletiert ist.

Erfindungsgemäß kann das Peptid zur Behandlung von Tumorerkrankungen und/oder adenomatöser Erkrankungen verwendet werden.

Die zu behandelnden Tumorerkrankungen umfassen dabei bspw. Nieren-, Gehirn-, Brust-, Pankreas-, Magen-, Hoden- und/oder Hautkrebs und Tumorerkrankungen des Nervensystems. Die Aufzählung der Tumorerkrankungen ist dabei lediglich beispielhaft und soll den Verwendungsbereich nicht eingrenzen. Dass die erfindungsgemäßen Peptide für eine solche Verwendung geeignet sind, konnten die Erfinder in eigenen Versuchen zeigen. Darin wurde nachgewiesen, dass eigens generierte CTL, die spezifisch für bestimmte Peptide waren, effektiv und selektiv Tumorzellen abtöten konnten.

Für einen Einsatz von Tumor-assoziierten Antigenen in einer Tumorvakzine sind grundsätzlich mehrere Applikationsformen möglich. So beschrieben Tighe et al., 1998, Gene vaccination: plasmid DNA is more than just a blueprint, Immunol. Today 19(2):89-97, dass das Antigen entweder als rekombinantes Protein mit geeigneten Adjuvantien bzw. Trägersystemen oder als die für das Antigen kodierende cDNA in Plasmidvektoren verabreicht werden kann. In diesen Fällen muss das Antigen im Körper des Patienten von Antigen-präsentierenden Zellen (APC) verarbeitet und präsentiert werden, damit eine Immunantwort ausgelöst wird.

Melief et al., 1996, Peptide-based cancer vaccines, Curr. Opin. Immunol. 8:651-657, zeigen eine weitere Möglichkeit, nämlich die Verwendung von synthetischen Peptiden als Vakzine.

Das Peptid kann dabei in einer bevorzugten Ausführungsform mit Zugabe von Adjuvantien verwendet werden, oder aber auch in Alleinstellung.

Als Adjuvans kann bspw. der Granulocyte-macrophage-colony-stimulating-factor (GM-CSF) eingesetzt werden. Weitere Beispiele für solche Adjuvantien sind Aluminiumhydroxid, Emulsionen von Mineralölen, wie bspw. das Freund'sche Adjuvans, Saponine oder Siliciumverbindungen.

Die Verwendung mit Adjuvantien bietet den Vorteil, dass die durch das Peptid ausgelöste Immunantwort verstärkt werden kann und/oder dass das Peptid stabilisiert wird.

In einer anderen bevorzugten Ausführungsform wird das Peptid gebunden auf einer Antigen-präsentierenden Zelle eingesetzt.

Diese Maßnahme hat den Vorteil, dass die Peptide dem Immunsystem, insbesondere den zytotoxischen T-Lymphozyten (CTL), präsentiert werden können. Dadurch können die CTL die Tumorzellen erkennen und spezifisch abtöten. Als Antigen-präsentierende Zellen sind bspw. dendritische Zellen, Monozyten oder B-Lymphozyten für einen solchen Einsatz geeignet.

Dabei werden die Zellen *ex vivo* mit den Peptiden beladen werden. Andererseits besteht auch die Möglichkeit, die Zellen mit der für die Peptide kodierenden DNA oder der entsprechenden RNA zu transfizieren, um dann die Peptide auf den Zellen zur Expression zu bringen.

Die Erfinder konnten in eigenen Versuchen zeigen, dass es möglich ist, dendritische Zellen (DC) mit spezifischen Peptiden zu beladen, und dass diese beladenen dendritischen Zellen Peptid-spezifische CTL aktivieren. Dies bedeutet, dass das Immunsystem stimuliert werden kann, CTL gegen die Tumore zu entwickeln, welche die entsprechenden Peptide exprimieren.

Die das Peptid tragenden Antigen-präsentierenden Zellen können dabei entweder direkt verwendet werden oder aber vor einem Einsatz bspw. mit dem Hitzeschock-Protein gp96 aktiviert werden. Dieses Hitzeschock-Protein induziert die Expression von MHC-Klasse I-Molekülen und von costimulierenden Molekülen wie B7 und stimuliert außerdem die Produktion von Zytokinen. Dadurch wird insgesamt die Auslösung von Immunantworten gefördert.

In einer weiteren Ausführungsform werden die Peptide zur Herstellung eines Antikörpers eingesetzt.

Polyklonale Antikörper können in herkömmlicher Weise durch Immunisierung von Tieren mittels Injektion der Peptide und anschließender Reinigung des Immunglobulins gewonnen werden.

Monoklonale Antikörper können nach Standardprotokollen hergestellt werden, wie bspw. in Methods Enzymol. (1986), 121, Hybridoma technology and monoclonal antibodies, beschrieben.

Die Erfindung betrifft außerdem in einem weiteren Aspekt eine pharmazeutische Zusammensetzung, die eines oder mehrere der Peptide enthält.

Diese Zusammensetzung dient bspw. der parenteralen Verabreichung, bspw. subkutan, intradermal oder intramuskulär, oder der oralen Verabreichung. Dabei sind die Peptide in einem pharmazeutisch annehmbaren, vorzugsweise wässrigen, Träger gelöst oder suspendiert. Darüber hinaus kann die Zusammensetzung Hilfsstoffe, wie bspw. Puffer, Bindemittel, Verdünnungsmittel, etc. enthalten.

Die Peptide können auch zusammen mit immunstimulierenden Substanzen, z.B. Zytokinen, verabreicht werden. Eine umfassende Darstellung von Hilfsstoffen, wie sie bei einer derartigen Zusammensetzung verwendet werden können, ist bspw. in A. Kibbe, Handbook of Pharmaceutical Excipients, 3. Ed., 2000, American Pharmaceutical Association and pharmaceutical press, dargestellt.

Das Mittel kann dabei zur Prävention, Prophylaxe und/oder Therapie von Tumorerkrankungen und/oder adenomatöser Erkrankungen eingesetzt werden.

Das pharmazeutische Mittel, das zumindest das Peptid mit der Sequenz ID-Nr. 303 enthält, wird einem Patienten verabreicht, der an einer Tumorerkrankung leidet, mit der das betreffende Peptid bzw. Antigen assoziiert ist. Dadurch kann eine Tumor-spezifische Immunantwort auf Basis Tumor-spezifischer CTL ausgelöst werden.

Die in der pharmazeutischen Zusammensetzung vorliegende Menge des Peptids oder der Peptide liegt dabei in einer therapeutisch effektiven Menge vor. Dabei können die in der Zusammensetzung enthaltenen Peptide auch an mindestens zwei verschiedene HLA-Typen binden.

Die vorliegende Erfindung betrifft in einem weiteren Aspekt Nukleinsäuremoleküle, die für das Peptid mit der Sequenz ID-Nr. 303 kodiert.

Die Nukleinsäuremoleküle können dabei DNA- oder RNA-Moleküle sein und ebenfalls für die Immuntherapie von Krebserkrankungen eingesetzt werden. Dabei induziert das von dem Nukleinsäuremolekül exprimierte Peptid eine Immunantwort gegen Tumorzellen, die das Peptid exprimieren.

Erfindungsgemäß können die Nukleinsäuremoleküle auch in einem Vektor vorliegen.

Darüber hinaus betrifft die Erfindung Zellen, die mit Hilfe des Nukleinsäuremoleküls, welches für die Peptide kodiert, genetisch derart verändert wurde, dass sie ein Peptid mit der Sequenz ID-Nr. 303 produzieren.

Die Zellen werden hierfür mit der für die Peptide kodierenden DNA oder der entsprechenden RNA transfiziert, wodurch die Peptide auf den Zellen zur Expression gebracht werden. Für einen solchen Einsatz sind als Antigen-präsentierende Zellen bspw. dendritische Zellen, Monozyten oder B-Lymphozyten geeignet.

Es versteht sich, dass die vorstehend genannten und die nach-stehend noch zu erläuternden Merkmale nicht nur in der jeweils angegebenen Kombination, sondern auch in Alleinstellung verwendbar sind, ohne den Rahmen der vorliegenden Erfindung zu verlassen.

Ausführungsbeispiele der Erfindung werden in den nachfolgenden Beispielen und den beigefügten Figuren dargestellt und erläutert. Es zeigen:
- Fig. 1a: die Negativkontrolle bezüglich CTL-spezifischer Lyse von KM22- Zielzellen (ohne Peptid);

- Fig. 1b: die CTL-spezifische Lyse von mit Peptid-gepulsten und mit Interferon- Gamma behandelten KM22-Zielzellen,
- Fig. 2: die Interferon-Gamma-Bildung durch spzifische T-Lymphozyten nach Stimulation durch Peptid-beladene KM22- oder JY-Zellen,
- Fig. 3: die Peptid-spezifische Stimulierung humaner CD8-positiver T-Zellen, und
- Fig. 4: den Nachweis von Peptid-spezifischen T-Zellen aus dem Blut von Nieren- zellkrebs-Patienten.
- Fig. 5: die Tetrameranalyse der Mikrospären-angetriebene Proliferation von B*0702/LARNLTQQL spezifischen CD8⁺ Lymphozyten aus peripherem Blut. 4 von 6 getesteten gesunden Spendern (HD155, -159, -161, -177) hat- ten signifikante (über der roten Linie befindliche) T-Zell-Antworten, die spezifisch gegen das getestete Peptid-Antigen mit SEQ-ID-Nr. 233 gerichtet waren, wie durch Nachweis von für den Peptid/EILA-B*0702-Komplex spe- zifischen T-Zell-Rezeptoren durch Tetramer-Färbung gezeigt wird.

### Beispiel 1

### 1.1. Patientenproben

Von der Abteilung für Urologie, Universität Tübingen, wurden acht Proben erhalten, die von Patienten stammten, die histologisch bestätigte Nierenzelltumore aufwiesen. Der mit RCC75 bezeichnete Patient besaß die folgende HLA-Typisierung: A*03, B*07, B*40. Der mit RCC98 bezeichnete Patient besaß die folgende HLA-Typisierung: A*01, A*03, B*07, B*18, der mit RCC100 bezeichnete Patient die HLA-Typisierung A*02, A*03 B*07, B*18. Der mit RCC103 bezeichnete Patient besaß die folgende HLA-Typisierung: A*11, A*25, B*15, B*44. Der mit RCC112 bezeichnete Patient besaß die folgende HLA-Typisierung: A*01, A*31, B*08, B*27. Der mit RCC115 bezeichnete Patient besaß die folgende HLA-Typisierung: A*02, A*03, B*15, B*18, der mit RCC116 bezeichnete Patient die HLA-Typisierung A*01, A*02, B*27, B*37 und der mit RCC130 bezeichnete Patient die HLA-Typisierung A*02, A*24, B*07, B*44.

Von der Abteilung für Neurochirurgie, Universität Heidelberg, wurden zwei Proben erhalten, die von Patienten stammten, die histologisch bestätigte Glioblastome aufwiesen. Der mit NCH359 bezeichnete Patient besaß die folgende HLA-Typisierung: A*03, A*32, B*07, B*35. Der mit NCH361 bezeichnete Patient besaß die folgende HLA-Typisierung: A*26 und B*38.

### 1.2. Isolierung der MHC-Klasse-I-gebundenen Peptide

Die schockgefrorenen Tumorproben wurden prozessiert wie bereits beschrieben in Schirle, M. et al., Identification of tumor-associated MHC class I ligands by a novel T cell-independent approach, 2000, European Journal of Immunology, 30:2216-2225. Die Peptide wurden nach Standardprotokollen isoliert, und zwar unter Verwendung des monoklonalen Antikörpers W6/32, der spezifisch für die HLA-Klasse-I-Moleküle ist, oder des monoklonalen Antikörpers BB7.2, der für HLA-A2 spezifisch ist. Barnstable, C.J. et al., Production of monoclonal antibodies to group A erythrocytes, HLA and other human cell surface antigens-new tools for genetic analysis, 1978, Cell, 14:9-20 und Parham, P. & Brodsky, F.M., Partial purification and some properties of BB7.2. A cytotoxic monoclonal antibody with specificity for HLA-A2 and a variant of HLA-A28, 1981, Hum. Immunol., 3:277-299, beschrieben die Herstellung und Anwendung dieser Antikörper.

### 1.3. Massenspektroskopie

Die Peptide wurden durch "reversed phase HPLC" getrennt (SMART-System, µRPC C2/C18 SC 2.1/19, Amersham Pharmacia Biotech) und die erhaltenen Fraktionen durch nanoESI MS analysiert. Dabei wurde vorgegangen, wie beschrieben in Schirle, M. et al., Identification of tumor-associated MHC class I ligands by a novel T cell-independent approach, 2000, European Journal of Immunology, 30:2216-2225.

Die Peptide, die aus Tumorgewebe gewonnen wurden, wurden wie eben erwähnt durch Kapillar-LC-MS identifiziert, allerdings mit geringfügigen Änderungen: 100 µl der Proben wurden jeweils geladen, entsalzt und auf einer 300 µm* 5 mm C18 µ-Vorsäule (LC Packings) vorkonzentriert. Das Lösungsmittel und die Probe wurden mittels einer Spritzenpumpe (PHD 2000, Harvard Apparatur, Inc.) mit einer abgedichteten 100 µl-Spritze (1710 RNR, Hamilton) mit einer Geschwindigkeit von 2 µl/min zugeführt. Zur Trennung der Peptide wurde die Vorkonzentrierungssäule vor eine 75 µm * 250 mm C-18-Säule (LC Packings) geschaltet. Anschließend wurde ein binärer Gradient mit 25-60% B innerhalb von 70 min gefahren, wobei die Flussrate von 12 µl/min auf ungefähr 300 nl/min reduziert wurde, und zwar unter Verwendung eines TEE-Anschlusses (ZT1C, Valco) und einer 300 µm * 150 mm C-18-Säule.

Um sicherzustellen, dass das System frei von restlichen Peptiden war, wurde jeweils eine Leer-Probe gemessen. Online-Fragmentierung wurde wie beschrieben durchgeführt, und die Spektren der Fragmente wurden manuell analysiert. Die Datenbankrecherchen (NCBInr, EST) wurden unter Verwendung von MASCOT durchgeführt (http://www.matrixscience.com).

### 1.4. Identifizierung der MHC-Klasse-I-Liganden

In dem beigefügten Sequenzprotokoll und in der beigefügten Tabelle 1 sind die Liganden aufgeführt, die an die HLA-Moleküle der Patienten RCC75, RCC98, RCC100, RCC103, RCC112, RCC115, RCC116, RCC130, NCH359 und NCH361 gebunden waren. Die Peptide, die mit HLA-A*02 assoziiert waren, wiesen das Allel-spezifische Peptidmotiv auf: So waren an Position 2 Leucin, Valin, Isoleucin, Alanin oder Methionin und am C-Terminus Leucin, Valin, Isoleucin, oder Alanin zu finden. Die meisten der Liganden stammten von sogenannten "housekeeping"-Proteinen ab, jedoch konnten auch Liganden von Proteinen identifiziert werden, die mit Tumoren assoziiert sind. So konnten bspw. Fragmente von Tenascin-C identifiziert werden (GLAPSIRTK, SEQ ID-Nr. 2; GVLKKVIRH, SEQ ID-Nr. 20). Herold-Mende et al., Clinical Impact and Functional Aspects of Tenascin-C Expression during Glioma Progression, 2002, Int. J. Cancer, 98: 362-369 zeigen, dass im allgemeinen die Höhe der Expression des extrazellulären Matrix-Proteins Tenascin-C mit dem Schweregrad der Erkrankung und der Einwanderung von Tumorzellen in gesundes Gewebe korreliert.

### 1.5. Nachweis von Peptid-spezifischen T-Zellen im normalen CD8+-T-Zell-Repertoir

Zum Nachweis von Peptid-spezifischen T-Zellen, beispielsweise für das Peptid mit der Sequenz FPSLREAAL (SEQ ID-Nr. 114), wurden mononukleäre Zellen aus peripherem Blut gesunder Probanden mit den betreffenden HLA-A*Subtypen-Tetrameren gefärbt, welche mit betreffenden Peptiden konstituiert waren: Zur Herstellung der Tetramere wurden rekombinante HLA-B*Subtypen-Moleküle *in vitro* mit den Peptiden konstituiert, durch Gelfiltration gereinigt, biotinyliert und mit Streptavidin zur Verknüpfung der Monomere gemischt, wie von Walter S et al., 2003, Cutting Edge: Predetermined Avidity of Human CD8 T Cells Expanded on Calibrated MHC/Anti-CD28-Coated Microspheres, J. Immunol. 171: 4974-4978, beschrieben.

Grundsätzlich werden die Ergebnisse der Doppelfärbungen durch Analyse mittels FACS ausgewertet und die spezifische Bindungen der Peptid-Tetramere nachgewiesen (Walter S et al., 2003, Cutting Edge: Predetermined Avidity of Human CD8 T Cells Expanded on Calibrated MHC/Anti-CD28-Coated Microspheres, J. Immunol. 171: 4974-4978).

### Beispiel 2

Um die Präsentation der ausgewählten Peptide durch Tumorzellen und die Erkennung der Peptide durch CTL zu analysieren, wurden *in vitro* CTL induziert, die spezifisch für die ausgewählten Peptide waren. Dazu wurden KM22- und JY-Zielzelllinien eingesetzt.

### 2.1 Gewinnung der spezifischen T-Lymphozyten

Spezifische T-Lymphozyten wurden wie unter 1.5 beschrieben aus dem Blut gesunder Probanden isoliert und durch FACS-Sorting angereichert.

### 2.2. Synthese der Peptide

Die beispielhaft ausgewählten Peptide wurden durch Verwendung von F-moc (9-Fluorenylmethyloxycarbonyl) -Schutzgruppen auf einem Peptid-Synthetisierer (432A, Applied Biosystems, Weiterstadt, Deutschland) synthetisiert und durch "reversed phase" HPLC und Massenspektroskopie analysiert. Auf diese Weise können ausreichende Mengen an den identifizierten Peptiden hergestellt werden.

### 2.3. Induktion einer Antigen-spezifischen CTL-Antwort unter Einsatz von restringierten synthetischen Peptiden

Zur Induktion von CTL wurden die in Schritt 2.1 gewonnenen T-Lymphozyten (5 x 10⁶ T-Lymphozyten pro well) durch *in vitro* Restimulation in 24-well Platten mit 1 x 10⁶ bestrahlten Zielzellen pro well in 1.5 ml T-Zell-Medium [bestehend aus RPMI 1640, 25 mM HEPES (Life Technologies/Invitrogen, Karlsruhe, Germany)] mit 10% Hitze-inaktiviertem humanen AB serum (CC Pro, Neustadt/Weinstraße, Germany), 2 mM L-Glutamin, 50 U/ml Penicillin, 50 µg/ml Streptomycin, and 20 µg/ml Gentamicin (alles von BioWhittaker/Cambrex, Verviers, Belgium) koinkubiert. Zusätzlich wurden 5 ng/ml human IL-12 p70 (R&D Systems) zugegeben. Nach ca. 4 Tagen Ko-Inkubation bei 37°C wurde frisches Medium mit 20 U/ml human IL-2 (R&D Systems) zugegeben und die Zellen für weitere 3 bis 4 Tage inkubiert. Dieser Stimulationszyklus wurde zweimal wiederholt.

### 2.4. CTL-Assay

Für die CTL-Assays wurden als Target-Zellen KM22 und JY-Tumor-Zelllinien verwendet. Peptid-gepulste Zellen wurden mit 50 µg/ml Peptid 2 Stunden lang gepulst. Alle Target-Zellen wurden in RP10Medium (RPMI 1640, ergänzt mit 10 % Hitze-inaktiviertem fetalem Kälberserum und mit Antibiotika) 1 Stunde lang bei 37°C mit [⁵¹Cr]Natriumchromat (⁵¹Cr) markiert. Anschließend wurden jeweils 10⁴ Zellen/pro well auf eine 96-well-Platte mit abgerundetem Boden gegeben. Verschiedene Mengen an CTL wurden hinzugefügt, um ein Endvolumen von 200 µl zu erreichen, mit anschließender Inkubation für 4 Stunden bei 37°C. Danach wurden die Überstände (50µl/well) geerntet und in einem beta-Platten-Zähler ausgezählt. Die spezifische Lyse wurde in Prozent folgendermaßen berechnet: 100 x (experimentelle Freisetzung - spontane Freisetzung / maximale Freisetzung - spontane Freisetzung). Die spontane und die maximale Freisetzung wurde jeweils in Gegenwart von entweder Medium oder 2 % Triton X-100 bestimmt.

### 2.5. Ergebnisse der CTL-Induktion

### a) CTL-zytotoxische Aktivität gegenüber Peptid-gepulsten Zielzellen

In ⁵¹Cr-Freisetzungs-Assays (siehe unter 2.4.) wurde die zytotoxische Aktivität induzierter CTL (siehe unter 2.3.) gegenüber KM22- oder JY-Zellen getestet. Die Zelllinien KM22 und J-Y sind HLA-B*07-positiv.

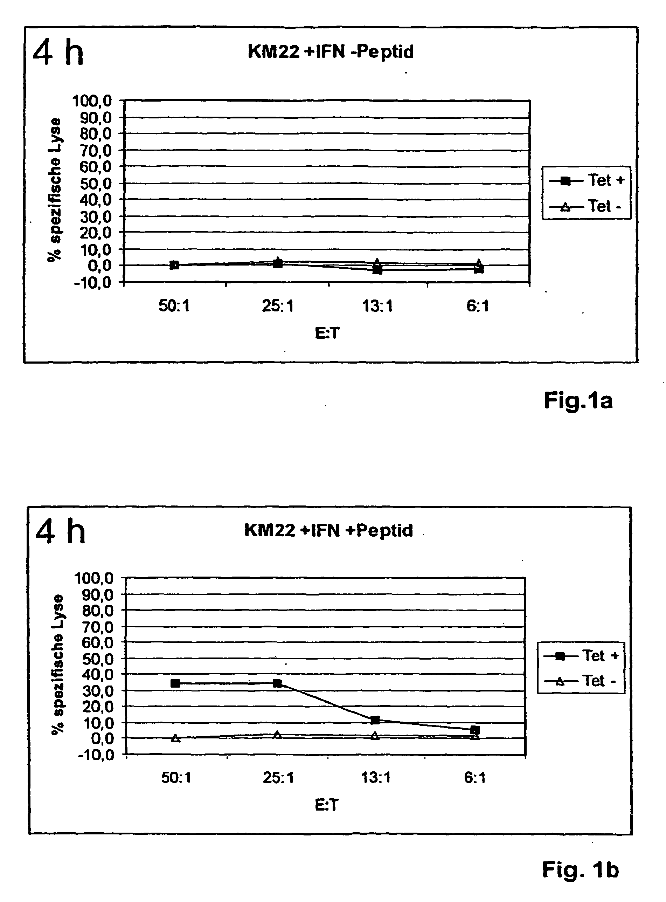

Die Ergebnisse dieser Freisetzungs-Assays sind in den Fig. 1a und 1b dargestellt. In den Fig. 1a und 1b wurde mit "IFN" Interferon-Gamma abgekürzt, mit "E:T" das Effektor-Zielzellen-Verhältnis, mit "Tet+" T-Lymphozyten, die an HLA-B*0702/FPSLREAAL-Tetramere binden und mit "Tet-" T-Lymphozyten, die nicht an HLA-B*0702/FPSLREAAL-Tetramere binden.

Die Ergebnisse zeigen, dass mit CTL-Zelllinien, die nach 2-wöchiger Restimulation gewonnen wurden, eine Antigen-spezifische Abtötung der Zellen erreicht werden konnte: In Fig. 1a ist gezeigt, dass mit Interferon-Gamma behandelte, für das HLA-Allel B*0702 positive KM22-Zielzellen ohne Peptid nicht durch spezifische zytotoxische T-Lymphozyten erkannt wurden. Fig. 1b zeigt, dass mit Interferon-Gamma behandelte, für das HLA-Allel B*0702 positive KM22-Zielzellen, die das Peptid mit der Sequenz FPSLREAAL aus dem Tumorantigen MAGE-1 präsentieren, durch spezifische zytotoxische T-Lymphozyten erkannt und lysiert wurden. Somit wurden also nur diejenigen Zellen durch eine ansteigende Menge an CTL abgetötet, die die jeweils ausgewählten Peptide präsentierten; die mit irrelevanten Peptiden beladenen Kontrollzellen wurden nicht abgetötet. Dadurch konnte die Spezifität der zytolytischen Aktivität gezeigt werden.

### b) Bildung von Interferon-Gamma durch Peptid-stiumlierte T-Lymphozyten

In einem nächsten Versuch wurde gezeigt, dass die zytotoxischen T-Lymphozyten, welche die mit dem Peptid FPSLREAAL (SEQ-ID-Nr. 114) beladenen T2-Zellen lysierten, auch Interferon-Gamma exprimierten, welches als zuverlässiger Marker für die Aktivierung von T-Zellen beschrieben worden ist.

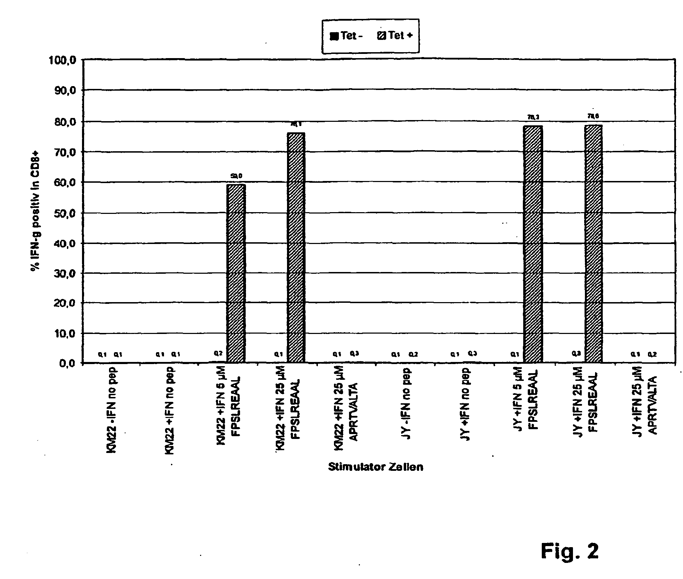

In Fig. 2 sind die Ergebnisse der Messungen gezeigt, wobei hier KM22- und JY-Zellen getestet wurden. Es wurden die gleichen Abkürzungen wie in Fig. 1 verwendet, wobei ferner mit "CD8+" T-Lymphozyten bezeichnet sind, die das Rezeptormolekül CD8 auf der Zelloberfläche exprimieren.

Für den IFN-Gamma -Nachweis wurden 1 x 10⁵ Effektor-Zellen und Stimulator-Zellen, welche Peptid-gepulst wurden, in T-Zell-Medium auf 96-well Platten kultiviert (T-Zell-Medium: RPMI 1640 mit 25 mM HEPES (Gibco/Invitrogen, Karlsruhe, Deutschland; ergänzt mit 10% Hitze-inaktiviertes humanes AB Serum (CC pro, Neustadt/W., Deutschland; 2 mM L-Glutamin, 50 U/ml Penicillin, 50 µg/ml Streptomycin und 20 µg/ml Gentamycin (alle BioWhittaker). Das Peptid-Beladen wurde bei 37°C in X-Vivo 15 Medium mit den jeweiligen Peptiden für ca. 2 Stunden durchgeführt.

Nach 1 bis 2 Stunden wurde GolgiStop (Betcon Dickinson) hinzugefügt und weitere 4 bis 5 Stunden inkubiert. Anschließend wurden die Zellen permeabilisiert und unter Verwendung des Cytofix/Cytoperm Plus-Kits, sowie mit anti-CD4-FITC, anti-IFN-γ-PE und anti-CD8-PerCP nach Empfehlung des Herstellers (Betcon Dickinson) gefärbt. Die zytometrischen Auswertungen erfolgten unter Verwendung des FACSCalibur Zytometers.

Wie in Fig. 2 zu sehen ist, bildeten an HLA-B*0702/FPSLREAAL-Tetramer bindende T-Lymphozyten (=spezifische T-Lymphozyten, "Tet+") Interferon-Gamma, wenn sie durch KM22- oder JY-Zellen, die jeweils mit unterschiedlichen Mengen an Interferon-Gamma vorbehandelt und mit dem Peptid mit der SEQ-ID-Nr. 114 beladen waren, stimuliert wurden (siehe Fig. 2, "KM22+FPSLREAAL" und "JY+ FPSLREAAL", jeweils mit 5 oder 25 µM INF vorbehandelt). Unspezifische T-Lymphozyten hingegen (also nicht an HLA-B*0702/FPSLREAAL-Tetramer bindende T-Lymphozyten) bildeten kein Interferon-Gamma (="Tet-").

In Fig. 2 ist ferner gezeigt, dass weder die spezifischen (="Tet+") noch die unspezifischen ("Tet-") T-Lymphozyten Interferon-Gamma bildeten, wenn sie mit einem unspezifischen Kontrollpeptid (in Fig. 2 beispielhaft: APRTVALTA, SEQ-ID-Nr. 585) stimuliert wurden (siehe Fig. 2 jeweils für "Tet+" und "Tet-": "KM22+APRTVALTA" und "JY+APRTVALTA", jeweils mit 25 µM INF vorbehandelt).

### c) Peptid-spezifische Stimulierung von CD8-positiven T-Zellen

Für einen weiteren Nachweis der Peptid-spezifischen Stimulierung von CD8-positiven T-Zellen wurde das Peptid mit der Sequenz LAALPHSCL (SEQ-ID-Nr. 448) vom Protein RGS-5 und das Kontroll-Peptid mit der Sequenz ELAGIGILTV (SEQ-ID-Nr. 578) vom Melanom-Antigen MELAN-A (Position 26-35, modifiziert durch einen AminosäureAustausch des an Position 27 befindlichen Alanin durch Leucin), wobei das Kontroll-Peptid auch an das HLA-Allel A*02 bindet, unter Verwendung der Fmoc-Chemie standardgemäß synthetisiert. Biotinylierte rekombinante A*02-Moleküle und fluoreszierende MHC-Tetramere wurden wie in Altman et al. ("Phenotypic analysis of antigen-specific T-lymphocytes, Science 274:94, 1996) beschrieben, hergestellt. Zur Herstellung artifizieller Antigen-präsentierender Zellen (APC's) wurden Streptavidin-beschichtete Polystyrol-Partikel (Durchmesser 5,6 µm) mit einer Bindungs-Kapazität von 0,064 µg Biotin-0,064 µg Biotin-FITC/mg Mikrosphären (Bangs Laboratories, Fishers, Illinois/USA) mit 2 x 10⁶ Partikeln/ml in einem Puffer resuspendiert, der die biotinylierten MCH und die Antikörper enthielt, und für 30 min bei Raumtemperatur inkubiert.

Für die Antigen-spezifische in vitro-Stimulation der humanen CD8 T-Zellen wurden PBMC's aus frischen Buffy-Coats durch Gradienten-Trennung standardgemäß isoliert. Unbehandelte CD8 T-Zellen wurden durch negative Depletion mittels MACS angereichert (Miltenyi Biotec, Bergisch Gladbach, Deutschland). Die *in vitro*-Stimulationen wurden auf 24-well-Platten mit 5 x 10⁶ Responder-Zellen und 1 x 10⁶ Beads oder 1 x 10⁶ bestrahlten APC's pro Well in 1,5 ml T-Zell-Medium (s.o.) durchgeführt. 5 ng/ml humanes IL-12 p70 (R%D Systems, USA) wurden mit Mikrosphären hinzugefügt. Nach 3 bis 4 Tagen Koinkubation bei 37°C wurde frisches Medium und 20 U/ml humanes IL-2 (R&D Systems, USA) hinzugefügt, und die Zellen wurden weitere 3 bis 4 Tage inkubiert. Dieser Stimulations-Zyklus wurde zweimal wiederholt.

Für die Zelloberflächen- und intrazelluläre zytometrische Analyse wurden Tetramer-Analysen mit fluoreszierenden MHC-Tetrameren plus anti-CD8-Antikörpern (Hybridom UKT8) auf einem vier-Farben FACSCalibur (Becton Dickinson) durchgeführt.

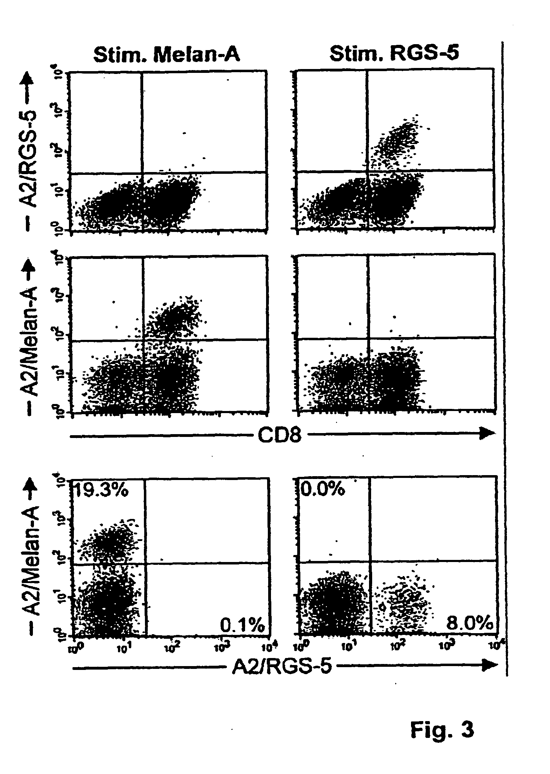

Die Ergebnisse dieser Analysen sind in Fig. 3 gezeigt. Darin ist zu sehen, das das RGS-5-Peptid LAALPHSCL mit der SEQ-ID-Nr. 448 spezifisch humane CD8-positive T-Zellen stimuliert. In Fig. 3 ist in der linken Spalte die Analyse von T-Zellen aus PBMC gezeigt, die wie oben beschrieben zuvor zwei Mal mit einem Kontroll-Peptid mit der Sequenz ELAGIGILTV aus Melan-A (SEQ-ID-Nr. 578) stimuliert worden waren. Die rechte Spalte zeigt die Analysen der T-Zellen, die mit dem RGS-5 Peptid mit der Sequenz LAALPHSCL (SEQ-ID-Nr. 448) stimuliert worden waren.

In Fig. 3 ist oben links gezeigt, dass CD8-positive (X-Achse) T-Zellen, stimuliert mit Melan-A-Peptid/MHC-A*02-Tetramer-Komplexen, nicht an mit dem Peptid mit der Sequenz LAALPHSCL (SEQ-ID-Nr. 448) komplexierte MHC-A*02-Tetramere binden (Y-Achse).

Oben rechts in Fig. 3 ist gezeigt, dass CD8-positive T-Zellen, stimuliert mit RGS-5-Peptid mit der Sequenz LAALPHSCL/MHC-A*02-Tetramer-Komplexen, an mit dem Peptid mit der Sequenz LAALPHSCL (SEQ-ID-Nr. 448) komplexierte MHC-A*02-Tetramere (Y-Achse) binden. Die doppelt angefärbten (doppelt positiven) Zellen sind im oberen rechten Quadranten zu sehen.

Links in der Mitte von Fig. 3 ist zu sehen, dass CD8-positive T-Zellen (X-Achse), die mit Melan-A-Peptid/MHC-A*02-Tetramer-Komplexen stimuliert worden waren, an mit dem Peptid mit der Sequenz ELAGIGILTV (SEQ-ID-Nr. 578) aus Melan-A komplexierte MHC-A*02-Tetramere (Y-Achse) binden. Die doppelt angefärbten (doppelt positiven) Zellen sind im oberen rechten Quadranten zu finden. In der Mitte rechts ist gezeigt, dass CD8-positive T-Zellen, stimuliert mit RGS-5-Peptid mit der Sequenz LAALPHSCL/MHC-A*02-Tetramer-Komplexen, nicht an mit dem Peptid mit der Sequenz ELAGIGILTV (SEQ-ID-Nr. 578) aus Melan-A komplexierte MHC-A*02-Tetramere (Y-Achse) binden.

In der unteren Reihe von Fig. 3 sind links die mengenmäßigen Anteile der Melan-A/MHC-A*02-spezifischen T-Zellen durch Doppelfärbung mit den zwei verwendeten MHC-Tetramer-Peptid-Komplexen (Melan-A/MHC-A*02 und RGS-5/MHC-A*02) gezeigt. Nach vorangegangener Stimulation mit auf artifizielle Antigen-präsentierende Zellen gebundene Melan-A-Peptid/MHC-A*02-Tetrarner-Komplexe binden 19,3 % der stimulierten Zellen spezifisch an den MHC-Tetramer-Komplex aus Melan-A-Peptid und HLA-A*02. Unten rechts sind die mengenmäßigen Anteile der RGS-5/MHC-A*02-spezifischen T-Zellen durch Doppelfärbung mit den zwei verwendeten MHC-Tetramer-Komplexen (Melan-A/MHC-A*02 und RGS-5/MHC-A*02) gezeigt. Nach vorangegangener Stimulation mit auf artifizielle Antigen-präsentierende Zellen gebundene RGS-5-Peptid/MHC-A*02-Tetramer-Komplexe binden 8,0 % der stimulierten Zellen spezifisch an den MHC-Tetramer-Komplex aus RGS-5-Peptid mit der Sequenz LAALPHSCL (SEQ-ID-Nr. 448) und HLA-A*02.

### d) Nachweis von Peptid-spezifischen T-Zellen aus dem Blut von Nierenzellkrebs-Patienten

In weiteren Versuchen konnten Peptid-spezifische T-Zellen aus dem Blut von Nierenzellkrebs-Patienten, die zuvor mit Peptid-beladenen autologen dendritischen Zellen immunisiert worden waren, nachgewiesen werden.

Für diesen Nachweis wurde eine quantitative Echtzeit-Polymerase-Kettenreaktion (RT-PCR) durchgeführt. Diese RT-PCR wurde wie von Kammula et al. beschrieben (Kammula et al., Journal of Immunology 163:6867, 2000) durchgeführt. Hierzu wurden PBMC's in T-Zell-Medium aufgetaut, mit 1 x 10⁶ Zellen in 500 µl Medium ausgesät und über Nacht bei 37°C und 5 % CO₂ inkubiert. Anschließend wurden die synthetischen Peptide mit 5 µg/ml für 3 Stunden hinzugefügt und danach eine RNA-Extraktion mit Trizol (Invitrogen, Karlsruhe, Deutschland) durchgeführt. Die cDNA wurde unter Verwendung von randomisierten Hexamer-Primem (Amersham Biosciences, Freiburg, Deutschland) und M-MLV reverser Transkriptase (Promega GmbH, Mannheim, Deutschland) transkribiert.

Die quantitative RT-PCR wurde mit einem "ABIPrism 7000 Sequence Detection System" (Applied Biosystems, Darmstadt, Deutschland) bezüglich IFN-gamma mRNA und CD8 mRNA doppelt durchgeührt, wobei der Taqman PCR Master Mix (Applied Biosystems), spezifische Primer und fluoreszierende Sonden eingesetzt wurden.

Die Ergebnisse geben die Kopienzahl der IFN-gamma mRNA wieder, wobei jede Probe bezüglich der CD8 mRNA-Kopienzahl (als Referenz-Genprodukt) normalisiert wurde (Stimulationsindex). Die Genexpression in Gegenwart der getesteten Peptide ist relativ zu derjenigen Genexpression dargestellt, die in Gegenwart der Kontrolle (von HIV1 pol abstammendes HLA-A*02-Epitop mit der Sequenz ILKEPVHGV, SEQ-ID-Nr. 579) erhalten wurde (Kopienzahl der Kontrolle wurde auf 1 gesetzt).

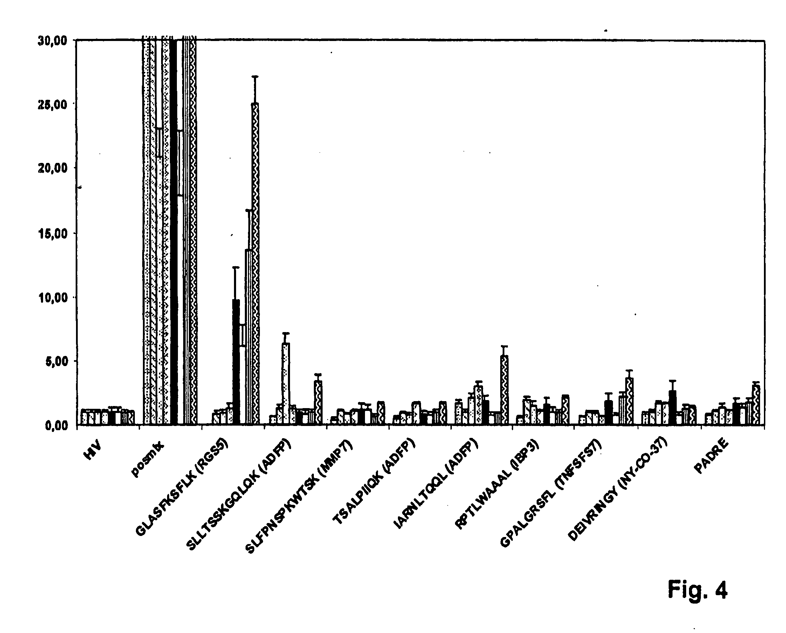

In Fig. 4 sind die Ergebnisse dieser Untersuchungen gezeigt. Die Abbildung der Fig. 4 zeigt die *ex vivo* T-Zell-Aktivierung nach Immunisierung des Patienten RCC98 am Universitätsklinikum Tübingen mit neun HLA-bindenden Peptiden. Die ersten beiden von 11 Säulenblöcken zeigen die Negativ- und die Positiv-Kontrolle des T-Zell-Versuchs. Die Fehlerbalken zeigen die Standardabweichung. Wie oben erwähnt, wurde als Negativ-Kontrolle das HIV-Peptid mit der Sequenz ILKEPVHGV (aus dem viralen Antigen HIV 1 pol, Position 896-904; SEQ-ID-Nr. 579) verwendet. Die Negativ-Kontrolle führte zu keiner T-Zell-Antwort, sofern der Patient HIV-seronegativ war. Als Positiv-Kontrolle wurde eine Mischung aus Peptiden von Influenza-Matrix 58-66 (GILGFVFTL; SEQ-ID-Nr. 580), HCMVA pp65 495-503 (NLVPMVATV; SEQ-ID-Nr. 581), EBNA6-EBV 284-293 (LLDFVRFMGV; SEQ-ID-Nr. 582), IE63-EBV 259-267 (GLCTLVAML; SEQ-ID-Nr. 583) und LMP2-EBV 294-302 (CLGGLLTMV; SEQ-ID-Nr. 584) verwendet, bei der eine sehr starke T-Zellantwort zu erwarten war ("posmix"; Maximum bei Stimulationsindex 43,6). Alle zur Stimulation von PBMC verwendeten Kontroll-Peptide wurden in einer Konzentration von 5 µg/ml eingesetzt. Der Hintergrund wurde als Standardabweichung der Negativkontrolle definiert (Stimulationsindex 1,35, dargestellt als waagrechte gestrichelte Linie).

In Fig. 4 zeigen die nächsten 9 Säulenblöcke der Abbildung die gegen neun immunisierten Peptide gemessene T-Zellaktivierung. Diese neun Peptide wurden insgesamt sieben Mal im Abstand von jeweils 14 Tagen gemeinsam subkutan beladen auf autologen dendritischen Zellen verabreicht. Daher besteht jeder Säulenblock aus 8 Säulen (vor der 1. Immunisierung, nach der 1. Immunisierung, nach der 2. Immunisierung etc.). Jeder Säulenblock zeigt daher den Verlauf der T-Zellantwort im Laufe der Immunisierung.

Bereits ab der 3. Immunisierung waren starke T-Zellantworten für GLASFKSFLK (RGS5 74-83, SEQ-ID-Nr. 153) und SLLTSSKGQLQK (ADFP 369-380, SEQ-ID-Nr. 289) zu sehen, wobei beim letzteren Peptid die T-Zellantwort nach der 4. Immunisierung zunimmt. Solche Fluktuationen in der T-Zellantwort im Verlauf einer Immunisierungstherapie sind auch von anderen Patienten bekannt.

T-Zellantworten waren eindeutig im Hintergrund für LARNLTQQL (ADFP 313-321; SEQ-ID-Nr. 233), GPALGRSFL (TNFSF7 78-86, SEQ-ID-Nr. 577) und schwächer für ein pan-HLA-DR-bindendes (PADRE) bekanntes Peptid zu sehen. Bei diesen drei Peptiden war auch eine Steigerung der T-Zellantwort im Verlauf der Immunisierungstherapie zu beobeobachten (gepunktete Linien). Dies bestätigte, dass diese drei letztgenannten Peptide eine positive T-Zellantwort ausgelöst haben.

Der Patient wurde im Rahmen einer angemeldeten klinischen Studie am Universitätsklinikum Tübingen immunisiert. Die klinische Studie wurde von der Ethikkommission der Universität Tübingen angemeldet und schriftlich genehmigt. Die Studie wurde nach den Regeln und Richtlinien des Arzneimittelgesetzes (AMG) und der "Good Clinical Practice" (GCP) durchgeführt.

Zusammengefasst konnten die Erfinder somit zeigen, dass die identifizierten Peptide vielversprechende Substanzen im Rahmen einer Immuntherapie bei einer Vielzahl von (Tumor-) Erkrankungen darstellen.

### Beispiel 3:

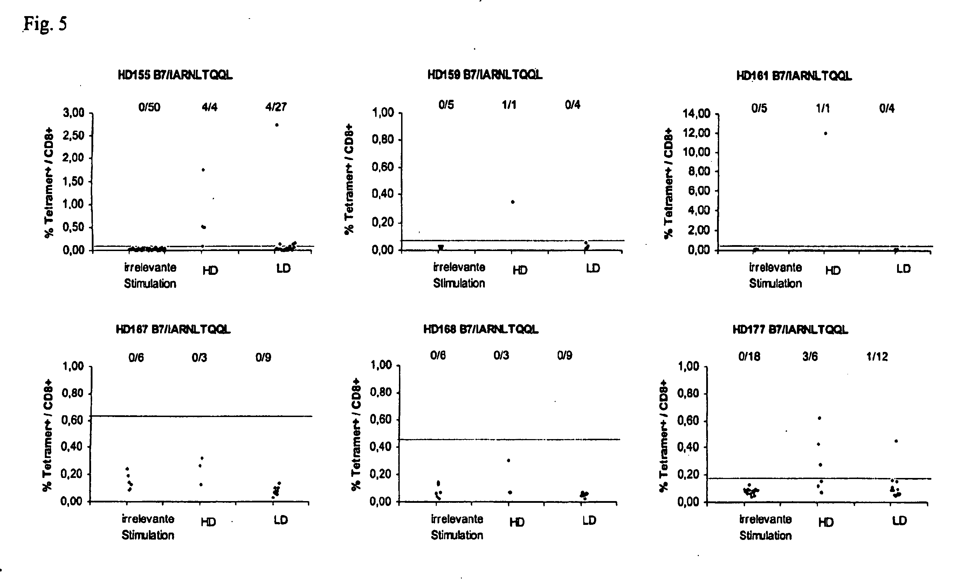

Tetrameranalyse der Mikrosphären-angetriebene Proliferation von B*0702/IARNLTQQL (SEQ ID Nr. 233) spezifischen CD8⁺ Lymphozyten aus peripherem Blut (siehe Fig. 5).

1 x 10⁶ CD8⁺ angereicherte PBMCs pro Well von sechs gesunden HLA-A*0201⁺ Spendern HD155, HD159, HD161, HD167, HD168 und HD177 wurden wöchentlich mit Mikrosphären, gekoppelt an anti-CD28 plus irrelevantem Antigen ("irrelevante Stimulierung"), Tumorantigen B*0702/IARNLTQQL hoher Dichte ("HD") oder Tumorantigen B*0702/IARNLTQQL niedriger Dichte ("LD") wie vorher gezeigt [Walter, S, et al. Cutting Edge: Predetermined avidity of human CD8 T cells expanded on calibrated MHC/anti-CD28-coated microspheres. J. Immunol. 171(10):4974-8, 2003] mit kleinen Modifikationen stimuliert. Nach drei Stimulationen *in vitro* wurden einzelne Wells mit Antikörper CD8 plus Tetramer B*0702/IARMTQQL gefärbt. Jeder Punkt stellt den Prozentsatz von Tetramer⁺ innerhalb CD8⁺ Lymphozyten aus einem Well dar.

Basierend auf der Verteilung von beobachteten Antworten nach irrelevanter Stimulierung in individuellen Spendern wurde ein signifikanter Schwellenwert berechnet und durch eine horizontale rote Linie unter der Verwendung der folgenden Formel:
Schwellenwert = obere Grenze von 95 % Konfidenzintervall für Mittel + 3 x obere Grenze von 95 % Konfidenzintervall für Standardabweichung
dargestellt. Zahlen in den Darstellungen stellen die Zahl von signifikanten positiven Wells unter Gesamtwells für angegebenen Zustand dar.

**Tabelle 1**

| | **Sequenz** | **Position/Gensymbol** | **Acc. Nr.** | **SEQ ID-Nr.** | |
|---|---|---|---|---|---|
| **Sequenzen von NCH359** | | | | | |
| 1. | VPDSSGPERIL | 78-88 HNRPK | NP_002131.2 | SEQ ID-Nr. | 1 |
| 2. | GLAPSIRTK | 1510-1518 TNC | NP_002151.1 | SEQ ID-Nr. | 2 |
| 3. | RLFEHPLYR | 149-157 FAM20C | NP_064608.1 | SEQ ID-Nr. | 3 |
| 4. | TPSEPHPVL | 381-389 HGRG8 | NP_057342.1 | SEQ ID-Nr. | 4 |
| 5. | QIFVKTLTGK | 2-11 RPS27A | AAH01392.1 | SEQ ID-Nr. | 5 |
| 6. | SLMHSFILK | 44-52 DNCL2A | NP_054902.1 | SEQ ID-Nr. | 6 |
| 7. | YPHLHNAEL | 127-135 SOX9 | NP_000337.1 | SEQ ID-Nr. | 7 |
| 8. | RLFVGSIPK | 244-252 SYNCRIP | NP_006363 | SEQ ID-Nr. | 8 |
| 9. | RVFPDKGYSF | 233-242 TIA1 | NP_071320.1 | SEQ ID-Nr. | 9 |
| 10. | SLYKKLEIK | 554-562 SLC9A2 | NP_003039.2 | SEQ ID-Nr. | 10 |
| 11. | HPVSDHEATL | 216-225 HLA-C | NP_002108 | SEQ ID-Nr. | 11 |
| 12. | 1-PTRVDFSL | 46-54 Symbol existiert nicht; Genart: unnamed protein product | BAC87610 | SEQ ID-Nr. | 12 |
| 13. | KSFGSAQEFAW | 386-396 COPB2 | NP_004757 | SEQ ID-Nr. | 13 |
| 14. | SPSTSRTPLL | 1026-1035 EGFR | NP_005219.2 | SEQ ID-Nr. | 14 |
| 15. | STFDSPAHW | 1149-1157 EGFR | NP_005219.2 | SEQ ID-Nr. | 15 |
| 16. | APEEHPVLL | 97-105 ACTB | NP_001092.1 | SEQ ID-Nr. | 16 |
| 17. | RQITQVYGF | 117-125 PPP6C | NP_002712.1 | SEQ ID-Nr. | 17 |
| 18. | KVSDYILQH | 1046-1054 ASTN2 | NP_054729.3 | SEQ ID-Nr. | 18 |
| 19. | KLLPSVVLK | 2-10 DTR | NP_001936.1 | SEQ ID-Nr. | 19 |
| 20. | GVLKKVIRH | 23-31 TNC | NP_002151.1 | SEQ ID-Nr. | 20 |
| 21. | KLFDHAVSKF | 40-49 | NP_004449.1 | SEQ ID-Nr. | 21 |
| | | ACSL4 | | | |
| 22. | ITVLTKPLPV | 112-121 PTPRO | NP_002839.1 | SEQ ID-Nr. | 22 |
| 23. | HPVHPDIKL | 130-138 PIAS1 | NP_057250.1 | SEQ ID-Nr. | 23 |
| 24. | IPRAALLPLL | 3-12 PRSS11 | NP_002766.1 | SEQ ID-Nr. | 24 |
| 25. | ATNRITVTW | 254-262 PIASY | NP_056981.2 | SEQ ID-Nr. | 25 |
| 26. | KLADRFLLY | 29-37 LMO4 | NP_006760.1 | SEQ ID-Nr. | 26 |

| **Sequenzen von NCH361** | | | | | |
|---|---|---|---|---|---|
| 27. | DHDPVDKIVL | 150-159 HNRPA2B1 | NP_002128.1 | SEQ ID-Nr. | 27 |
| 28. | DHHQEVIGF | 165-173 C9ORF10 | NP_055427.2 | SEQ ID-Nr. | 28 |
| 29. | IHDLDNISF | 188-196 PSMB2 | NP_002785.1 | SEQ ID-Nr. | 29 |
| 30. | DHINDIIKI | 834-842 IQGAP1 | NP_003861.1 | SEQ ID-Nr. | 30 |
| 31. | DHMRTISEL | 355-363 CYFIP1 | NP_055423.1 | SEQ ID-Nr. | 31 |
| 32. | THSLPVVVI | 456-464 STAT3 | NP_003141.2 | SEQ ID-Nr. | 32 |
| 33. | MPVGPDAILRY | 929-939 BAT3 | NP_004630.2 | SEQ ID-Nr. | 33 |
| 34. | RLDDAIHVL | 406-414 TCF12 | NP_003196.1 | SEQ ID-Nr. | 34 |
| 35. | QHEGTVNIF | 1953-1961 PTPRZ1 | NP_002842.1 | SEQ ID-Nr. | 35 |
| 36. | ETVNIWTHF | 48-56 PAQR6 | NP_940798 | SEQ ID-Nr. | 36 |
| 37. | VHILDTETF | 195-203 KLHDC2 | NP_055130.1 | SEQ ID-Nr. | 37 |
| 38. | QTPDFTPTKY | 607-616 ZHX3 | NP_055850.1 | SEQ ID-Nr. | 38 |
| 39. | RHVEVFELL | 133-141 MPDZ | NP_003820.1 | SEQ ID-Nr. | 39 |
| 40. | TTIDIGVKY | 136-144 CNN3 | NP_001830.1 | SEQ ID-Nr. | 40 |
| 41. | DLIEHFSQF | 113-121 HNRPA0 | NP_006796.1 | SEQ ID-Nr. | 41 |
| 42. | ETVWRLEEF | 65-73 HLA-DRA | NP_061984 | SEQ ID-Nr. | 42 |
| 43. | DVLESVNLL | 176-184 AP2M1 | NP_004059.2 | SEQ ID-Nr. | 43 |
| 44. | IHDDFVTTF | 466-474 AEBP1 | NP_001120.2 | SEQ ID-Nr. | 44 |
| 45. | IHIPINNII | 57-65 Sec61G | NP_055117.1 | SEQ ID-Nr. | 45 |
| 46. | IHLIDPNTL | 281-289 CGI-07 | NP_057022.2 | SEQ ID-Nr. | 46 |
| 47. | IHVIGGNDV | 1016-1024 KIAA1268 | XP_291055.1 | SEQ ID-Nr. | 47 |
| 48. | KAFQKIVVL | 291-299 BZW1 | NP_055485.2 | SEQ ID-Nr. | 48 |
| 49. | YQDLLNVKL | 349-357 GFAP | NP_002046.1 | SEQ ID-Nr. | 49 |
| 50. | GHYEVAELL | 728-736 TNKS | NP_003738.1 | SEQ ID-Nr. | 50 |
| 51. | LVVYPWTQRF | 33-42 HBB | NP_000509.1 | SEQ ID-Nr. | 51 |
| 52. | MHLRQYELL | 386-393 GNAS | NP_000507.1 | SEQ ID-Nr. | 52 |
| 53. | EAIEQILKY | 149-157 FLJ10539 | NP_060600.1 | SEQ ID-Nr. | 53 |
| 54. | DVAEGDLIEHF | 108-118 HNRPA0 | NP_006796.1 | SEQ ID-Nr. | 54 |
| 55. | DVLQKIKY | 191-198 EPS8L1 | NP_060199.2 | SEQ ID-Nr. | 55 |
| 56. | DSFPMEIRQY | 24-33 STAT1 | NP_009330.1 | SEQ ID-Nr. | 56 |
| 57. | DVISNIETF | 281-289 SOX9 | NP_000337.1 | SEQ ID-Nr. | 57 |
| 58. | DVIRLIMQY | 9-17 SMU-1 | NP_060695.1 | SEQ ID-Nr. | 58 |
| 59. | DVIERVIQY | 792-800 IDN3 | NP_056199.1 | SEQ ID-Nr. | 59 |
| 60. | DVIAQGIGKL | 53-62 RPLP2 | NP_000995.1 | SEQ ID-Nr. | 60 |
| 61. | DVFNEKGWNY | 94-103 PBEF1 | NP_005737.1 | SEQ ID-Nr. | 61 |
| 62. | THLDSVTKI | 254-262 C6.1A | NP_077308.1 | SEQ ID-Nr. | 62 |
| 63. | DVAGIIADY | 294-302 KIAA1238 | XP_048675.4 | SEQ ID-Nr. | 63 |
| 64. | TAAPFPFHL | 536-544 TBX2 | NP_005985.2 | SEQ ID-Nr. | 64 |
| 65. | DTLDKVFTY | 86-94 ACSL3 | NP_004448.2 | SEQ ID-Nr. | 65 |
| 66. | DTISPTLGF | 42-50 ARL2 | NP_001658.1 | SEQ ID-Nr. | 66 |
| 67. | DTGILDSIGRF | 35-45 MBP | NP_002376.1 | SEQ ID-Nr. | 67 |
| 68. | VVYPWTQRF | 34-42 HBB | NP_000509.1 | SEQ ID-Nr. | 68 |
| 69. | EVVAGIKEYF | 128-137 MORF4 | NP_006783.2 | SEQ ID-Nr. | 69 |
| 70. | SSVPGVRLL | 72-80 VIM | NP_003371.1 | SEQ ID-Nr. | 70 |
| 71. | SVVDAIGISRF | 364-374 FLJ45273 | BAC86883.1 | SEQ ID-Nr. | 71 |
| 72. | EVIPPMKEF | 115-123 NDUFB6 | NP_002484.1 | SEQ ID-Nr. | 72 |
| 73. | EVIPPYYSY | 152-160 TTRAP | NP_057698.2 | SEQ ID-Nr. | 73 |
| 74. | EVNGLISMY | 284-292 U5-116KD | NP_004238.2 | SEQ ID-Nr. | 74 |
| 75. | EVIDLMIKEY | 57-66 PHF10 | NP_060758.1 | SEQ ID-Nr. | 75 |
| 76. | EVVAGIKEY | 128-136 MORF4 | NP_006783.2 | SEQ ID-Nr. | 76 |
| 77. | EVFPLAMNY | 76-84 CCND1 | NP_444284.1 | SEQ ID-Nr. | 77 |
| 78. | EVVERVLTF | 28-36 FBXO22 | NP_036302.1 | SEQ ID-Nr. | 78 |
| 79. | SHSPFGLDSF | 1251-1260 JMJD1B | NP_057688.2 | SEQ ID-Nr. | 79 |
| 80. | FGVDRAILY | 457-465 ITGAV | NP_002201.1 | SEQ ID-Nr. | 80 |
| 81. | SHSDYLLTI | 76-84 SOCS2 | NP_003868.1 | SEQ ID-Nr. | 81 |
| 82. | SHLDYDITL | 511-519 KIAA0794 | XP_087353.5 | SEQ ID-Nr. | 82 |
| 83. | SHFVSDVVI | 63-71 GNB2L1 | NP_006089.1 | SEQ ID-Nr. | 83 |
| 84. | EVTELLARY | 155-163 POLR2E | NP_002686.2 | SEQ ID-Nr. | 84 |
| 85. | ETADTLMGLRY | 425-435 GFPT1 | NP_002047.1 | SEQ ID-Nr. | 85 |
| 86. | EHAHLIVVL | 662-670 ABCB9 | NP_062570.1 | SEQ ID-Nr. | 86 |
| 87. | EHSLVIDTL | 53-61 PFDN2 | NP_036526.2 | SEQ ID-Nr. | 87 |
| 88. | EIAEAYLGY | 129-137 HSPAIA | NP_005336.2 | SEQ ID-Nr. | 88 |
| 89. | EIYGGSDSRF | 42-51 SF3B1 | NP_036565.1 | SEQ ID-Nr. | 89 |
| 90. | ELIAKIPNF | 73-81 SET | NP_003002.1 | SEQ ID-Nr. | 90 |
| 91. | EVIKNFIQY | 50-58 EIF3S6IP | NP_057175.1 | SEQ ID-Nr. | 91 |
| 92. | ETADTLLALRY | 426-436 GFPT2 | NP_005101.1 | SEQ ID-Nr. | 92 |
| 93. | EVVSEPFRSF | 581-590 PSMD2 | NP_002799.3 | SEQ ID-Nr. | 93 |
| 94. | ETFDAGLQAF | 2019-2028 SPTAN1 | NP_003118.1 | SEQ ID.Nr. | 94 |
| 95. | SHSQLMQLI | 164-172 ADRM1 | NP_008933.2 | SEQ ID-Nr. | 95 |
| 96. | ETVRELTEF | 255-263 PPARD | NP_006229.1 | SEQ ID-Nr. | 96 |
| 97. | EVAATEIKM | 10-18 HNRPM | NP_005959.2 | SEQ ID-Nr. | 97 |
| 98. | EVAAVLLHF | 214-222 SEC10L1 | NP_006535.1 | SEQ ID-Nr. | 98 |
| 99. | EVFDKTYQF | 132-140 C6orf153 | NP_149103.1 | SEQ ID-Nr. | 99 |
| 100. | ELVKRILNF | 174-182 DEK | NP_003463.1 | SEQ ID-Nr. | 100 |
| 101. | AHDDGRWSL | 95-103 FSCN1 | NP_003079.1 | SEQ ID-Nr. | 101 |
| 102. | SVVSVISRF | 4-12 DAD1 | NP_001335.1 | SEQ ID-Nr. | 102 |
| 103. | SVVELINHY | 132-140 PIK3R3 | NP_003620.2 | SEQ ID-Nr. | 103 |
| 104. | SVVDLINHY | 397-405 PIK3R2 | NP_005018.1 | SEQ ID-Nr. | 104 |
| 105. | AHVDLIEKL | 51-59 POLR2L | NP_066951.1 | SEQ ID-Nr. | 105 |
| 106. | FHNELLTQL | 97-105 BAIAP2 | NP_006331.1 | SEQ ID-Nr. | 106 |
| 107. | SVIEAVAHF | 812-820 C6orf133 | NP_056070.1 | SEQ ID-Nr. | 107 |
| 108. | GHFEKPLFL | 149-157 NTE | NP_006693.2 | SEQ ID-Nr. | 108 |
| 109. | GHDASQITL | 273-281 THIL | NP_057481.2 | SEQ ID-Nr. | 109 |
| 110. | SAVDFIRTL | 293-301 STK17A | NP_004751.1 | SEQ ID-Nr. | 110 |
| 111. | ISTPVIRTF | 989-997 C9orf10 | NP_055427.2 | SEQ ID-Nr. | 111 |
| 112. | GVIEKLLTSY | 28-37 D1S155E | AAH32446 | SEQ ID-Nr. | 112 |
| 113. | SHDLTLVNL | 395-403 KIAA1706 | NP_085139.1 | SEQ ID-Nr. | 113 |

| **Sequenz von JY** | | | | | |
|---|---|---|---|---|---|
| 114. | FPSLREAAL | 294-302 MAGEA1 | NP_004979.2 | SEQ ID-Nr. | 114 |

| **Sequenzen von RCC075** | | | | | |
|---|---|---|---|---|---|
| 115. | SIFKQPVTK | 250-258 MBD2 | NP_003918.1 | SEQ ID-Nr. | 115 |
| 116. | KPNANRIAL | 139-147 LGALS3 | NP_002297.1 | SEQ ID-Nr. | 116 |
| 117. | KLYEMILKR | 174-182 ARL7 | NP_005728.2 | SEQ ID-Nr. | 117 |
| 118. | SLFSRLFGK | 7-15 ARF4 | NP_001651.1 | SEQ ID-Nr. | 118 |
| 119. | KLFDKLLEY | 309-317 API5 | NP_006586.1 | SEQ ID-Nr. | 119 |
| 120. | SLFPNSPKWTSK | 96-107 MMP7 | NP_002414.1 | SEQ ID-Nr. | 120 |
| 121. | LESLDQLEL | 29-37 BAG2 | NP_004273.1 | SEQ ID-Nr. | 121 |
| 122. | VVNKVPLTGK | 101-110 MGC17943 | NP_689474.1 | SEQ ID-Nr. | 122 |
| 123. | SVYDSVLQK | 4470-4478 SYNE1 | NP_149062.1 | SEQ ID-Nr. | 123 |
| 124. | SVYVLVRQK | 39-47 MLSTD2 | NP_115604.1 | SEQ ID-Nr. | 124 |
| 125. | ILENIQRNK | 557-565 ERCC2 | NP_000391.1 | SEQ ID-Nr. | 125 |
| 126. | GSYNKVFLAK | 146-155 PSMD8 | NP_002803.1 | SEQ ID-Nr. | 126 |
| 127. | TESGLNVTL | 6-14 PCBP1 | NP_006187.1 | SEQ ID-Nr. | 127 |
| 128. | TEHGVEVVL | 612-620 SH2D3C | NP_005480.1 | SEQ ID-Nr. | 128 |
| 129. | TEARFGAQL | 327-335 KRT19 | NP_002267.2 | SEQ ID-Nr. | 129 |
| 130. | TLADILLYY | 114-122 EEF1E1 | NP_004271.1 | SEQ ID-Nr. | 130 |
| 131. | LVFPSEIVGK | 133-142 RPS7 | NP_001002.1 | SEQ ID-Nr. | 131 |
| 132. | VLFGKALNPK | 709-718 ABCC3 | NP_003777.2 | SEQ ID-Nr. | 132 |
| 133. | RPELVRPAL | 91-99 AKR1C3 | NP_003730.4 | SEQ ID-Nr. | 133 |
| 134. | VPNQKRLTLL | 576-585 ACSL4 | NP_004449.1 | SEQ ID-Nr. | 134 |
| 135. | QLYWSHPRK | 5-13 RPS29 | NP_001023.1 | SEQ ID-Nr. | 135 |
| 136. | SVYVYKVLK | 39-47 H2BFS | NP_059141.1 | SEQ ID-Nr. | 136 |
| 137. | REKLQEEML | 186-194 VIM | NP_003371.1 | SEQ ID-Nr. | 137 |
| 138. | RVFSGLVSTGLK | 415-426 EEF2 | NP_001952.1 | SEQ ID-Nr. | 138 |
| 139. | KPRDVSSVEL | 1939-1948 SPTBN1 | NP_003119.1 | SEQ ID-Nr. | 139 |
| 140. | NEFPEPIKL | 184-192 RAB7 | NP_004628.4 | SEQ ID-Nr. | 140 |
| 141. | KTYGEIFEK | 106-114 NDUFC2 | NP_004540.1 | SEQ ID-Nr. | 141 |
| 142. | RILFFNTPK | 196-204 PSMD8 | NP_002803.1 | SEQ ID-Nr. | 142 |
| 143. | RVFPWFSVK | 1764-1772 MLL | NP_005924.1 | SEQ ID-Nr. | 143 |
| 144. | SEVQDRVML | 54-62 CGI-127 | NP_057145.1 | SEQ ID-Nr. | 144 |
| 145. | SLWDRLIFH | 410-418 ACSL1 | NP_001986.2 | SEQ ID-Nr. | 145 |
| 146. | KVYNIQIRY | 468-476 LCP2 | NP_005556.1 | SEQ ID-Nr. | 146 |
| 147. | RLLEMILNK | 171-179 AKR1C2 | NP_001345.1 | SEQ ID-Nr. | 147 |
| 148. | SEDKKNIIL | 41-49 CFL1 | NP_005498.1 | SEQ ID-Nr. | 148 |
| 149. | YEELVRMVL | 106-114 MYL6 | NP_524147.1 | SEQ ID-Nr. | 149 |
| 150. | GEITGEVHM | 1758-1766 FLNB | NP_001448.1 | SEQ ID-Nr. | 150 |
| 151. | IVAGSLITK | 183-191 FNBP3 | AAH11788 | SEQ ID-Nr. | 151 |
| 152. | APRITTGPAPVL | 225-236 SKI | NP_006766.1 | SEQ ID-Nr. | 152 |
| 153. | GLASFKSFLK | 74-83 RGS5 | NP_003608.1 | SEQ ID-Nr. | 153 |
| 154. | FPNSPKWTSK | 98-107 MMP7 | NP_002414.1 | SEQ ID-Nr. | 154 |
| 155. | FVIETARQL | 49-57 C14orf4 | NP_078772.1 | SEQ ID-Nr. | 155 |
| 156. | IEVDGKQVEL | 46-55 RHOA | NP_001655.1 | SEQ ID-Nr. | 156 |
| 157. | GELTGEVRM | 1776-1786 FLNC | NP_001449.1 | SEQ ID-Nr. | 157 |
| 158. | GESDDSILRL | 63-72 RPS21 | NP_001015.1 | SEQ ID-Nr. | 158 |
| 159. | GEGDFLAEGGGV | 23-34 FGA | NP_000499.1 | SEQ ID-Nr. | 159 |
| 160. | DNFPQSL | 690-696 CACNA1C | NP_000710.3 | SEQ ID-Nr. | 160 |
| 161. | GLTDVILYH | 269-277 SYNCRIP | NP_006363.3 | SEQ ID-Nr. | 161 |
| 162. | AALVASGVALY | 247-257 P2RY11 | NP_002557.2 | SEQ ID-Nr. | 162 |
| 163. | AEIRHVLVTL | 107-116 MYL6 | NP_066299.2 | SEQ ID-Nr. | 163 |
| 164. | AEPEEVEVL | 10-18 PGR1 | NP_150638.1 | SEQ ID-Nr. | 164 |
| 165. | AIIDHIFASK | 256-265 KIS | NP_787062 | SEQ ID-Nr. | 165 |
| 166. | ALLDGSNVVFK | 48-58 HKE2 | NP_055075.1 | SEQ ID-Nr. | 166 |
| 167. | AMLDTVVFK | 302-310 PSMD14 | NP_005796.1 | SEQ ID-Nr. | 167 |
| 168. | APARLFALL | 2-10 SDC4 | NP_002990.2 | SEQ ID-Nr. | 168 |
| 169. | AVNAHSNILK | 248-257 IMMT | NP_006830 | SEQ ID-Nr. | 169 |
| 170. | APRPGVLLL | 8-16 ELN | NP_000492 | SEQ ID-Nr. | 170 |
| 171. | EAFPLRVID | 749-757 MAN2A2 | NP_006113.1 | SEQ ID-Nr. | 171 |
| 172. | GVADKILKK | 211-219 NMI | NP_004679.1 | SEQ ID-Nr. | 172 |
| 173. | AVFPKPFVEK | 189-198 KIAA0377 | NP_055474.2 | SEQ ID-Nr. | 173 |
| 174. | VVYVGGILTK | 258-267 UGT8 | NP_003351.2 | SEQ ID-Nr. | 174 |
| 175. | HLEDIVRQK | 1751-1759 TRIP12 | XP_376178.1 | SEQ ID-Nr. | 175 |
| 176. | VTLTLVILSY | 207-216 LOC390323 | XP_372460.1 | SEQ ID-Nr. | 176 |
| 177. | SLLSLVTGLK | Leserahmen +3 Symbol existiert nicht; Genart: expressed sequence tag | CD105815 | SEQ ID-Nr. | 177 |
| 178. | QTYVGITEK | 687-695 U5-200KD | NP_054733.2 | SEQ ID-Nr. | 178 |
| 179. | HEDKIRVVL | 210-218 EHD2 | NP_055416.2 | SEQ ID-Nr. | 179 |
| 180. | QISIPFLLK | 208-216 C9orf88 | AAH01979 | SEQ ID-Nr. | 180 |
| 181. | GLMGFIVYK | 29-37 C14orf2 | NP_004885.1 | SEQ ID-Nr. | 181 |
| 182. | FADQEVRSL | 950-958 PIK3C2A | NP_002636.1 | SEQ ID-Nr. | 182 |
| 183. | IVALILSTK | 147-155 ATP6V0C | NP_001685.1 | SEQ ID-Nr. | 183 |
| 184. | GTYAPAEVPK | 22-31 | NP_001344.2 | SEQ ID-Nr. | 184 |
| | | AKR1C1 | | | |
| 185. | GTMTGMLYK | 161-169 TIMM23 | NP_006318.1 | SEQ ID-Nr. | 185 |
| 186. | SLAEILLKK | 439-447 IPO8 | NP_006381.1 | SEQ ID-Nr. | 186 |
| 187. | KLTYIYIQK | Leserahmen +1 Symbol existiert nicht; Genart: expressed sequence tag | AA295205 | SEQ ID-Nr. | 187 |
| 188. | KLLNYAPLEK | 58-67 POLR2L | NP_055427 | SEQ ID-Nr. | 188 |
| 189. | GTLPHPLQR | 182-190 SCNN1A | NP_001029.1 | SEQ ID-Nr. | 189 |
| 190. | GLYEFFRAK | 680-688 CHERP | NP_006378.2 | SEQ ID-Nr. | 190 |
| 191. | KEPEINTTL | 226-234 FLJ34588 | NP_689939.1 | SEQ ID-Nr. | 191 |
| 192. | HASDRIIAL | 330-338 TKT | NP_001055.1 | SEQ ID-Nr. | 192 |

| **Sequenzen von RCC098** | | | | | |
|---|---|---|---|---|---|
| 193. | RPTLWAAAL | 5-13 IGFBP3 | NP_000589.1 | SEQ ID-Nr. | 193 |
| 194. | APSPRPLSL | 11-19 C19orf28 | NP_778148.1 | SEQ ID-Nr. | 194 |
| 195. | ASDFITKMDY | 362-371 GSN | NP_000168.1 | SEQ ID-Nr. | 195 |
| 196. | EERVINEEY | 13-21 RBBP4 | NP_005601.1 | SEQ ID-Nr. | 196 |
| 197. | ATGSWDSFLK | 328-337 GNB1 | NP_002065.1 | SEQ ID-Nr. | 197 |
| 198. | RMFDMGFEY | 411-419 DDX42 | NP_031398.2 | SEQ ID-Nr. | 198 |
| 199. | APLLRWVL | 265-272 HMOX1 | NP_002124.1 | SEQ ID-Nr. | 199 |
| 200. | ALRPSTSRSLY | 43-53 VIM | NP_003371.1 | SEQ ID-Nr. | 200 |
| 201. | RQIPYTMMK | 225-233 SLC25A3 | NP_002626.1 | SEQ ID-Nr. | 201 |
| 202. | AETHIVLLF | 267-275 DKFZp564K142 | NP_115497.3 | SEQ ID-Nr. | 202 |
| 203. | RVHAYIISY | 305-313 EHD2 | NP_055416.2 | SEQ ID-Nr. | 203 |
| 204. | AVIVLVENFYK | 11-21 S100A16 | NP_525127.1 | SEQ ID-Nr. | 204 |
| 205. | SEELLREHY | 61-69 NME3 | NP_002504.2 | SEQ ID-Nr. | 205 |
| 206. | RADGNFLLY | 368-376 KIAA0930 | XP_047214.6 | SEQ ID-Nr. | 206 |
| 207. | SEFTGVWKY | 83-91 PDCD6 | NP_037364.1 | SEQ ID-Nr. | 207 |
| 208. | SIDRTVMYY | 389-397 SLC3A1 | NP_000332.1 | SEQ ID-Nr. | 208 |
| 209. | ETDLLDIRSEY | 463-473 ANXA11 | NP_001148.1 | SEQ ID-Nr. | 209 |
| 210. | ESYEALPQH | 397-405 DNMT1 | NP_001370.1 | SEQ ID-Nr. | 210 |
| 211. | SEEEIREAF | 82-90 CALM2 | NP_001734.1 | SEQ ID-Nr. | 211 |
| 212. | KVMQQNLVY | 329-337 CRTAP | NP_006362.1 | SEQ ID-Nr. | 212 |
| 213. | DEKSITTY | 262-269 SPTBN1 | NP_003119.1 | SEQ ID-Nr. | 213 |
| 214. | EEIEGFRY | 421-428 DDX56 | NP_061955.1 | SEQ ID-Nr. | 214 |
| 215. | MENLFINRF | 186-194 ALOX5 | NP_000689.1 | SEQ ID-Nr. | 215 |
| 216. | MEKIWHHTF | 82-90 ACTB | NP_001092.1 | SEQ ID-Nr. | 216 |
| 217. | MEHAMETMMF | 5-14 S100A10 | NP_002957.1 | SEQ ID-Nr. | 217 |
| 218. | EEIFNLKF | 353-542 GTF2I | NP_001509.2 | SEQ ID-Nr. | 218 |
| 219. | LVLMVLYLI | 153-161 PIGM | NP_660150.1 | SEQ ID-Nr. | 219 |
| 220. | EELQQKVSY | 285-293 STAT3 | NP_003141.2 | SEQ ID-Nr. | 220 |
| 221. | LRVAPEEHPVL | 94-104 ACTB | NP_001092.1 | SEQ ID-Nr. | 221 |
| 222. | DGHLFQVEY | 13-21 PSMA7 | NP_002783.1 | SEQ ID-Nr. | 222 |
| 223. | LAELAHREY | 14-22 OGT | NP_003596.2 | SEQ ID-Nr. | 223 |
| 224. | NEADVHGIYF | 651-660 CP | NP_000087.1 | SEQ ID-Nr. | 224 |
| 225. | KVFQEPLFY | 114-122 CTSL | NP_001903.1 | SEQ ID-Nr. | 225 |
| 226. | GVLAWVKEK | 171-179 NK4 | NP_004212.3 | SEQ ID-Nr. | 226 |
| 227. | HEALLYYVL | 738-746 KIAA0746 | NP_056002.1 | SEQ ID-Nr. | 227 |
| 228. | HEMIILKL | 3489-3496 KIAA1554 | XP_290768.3 | SEQ ID-Nr. | 228 |
| 229. | IVPANFPSL | 443-451 C9orf3 | NP_116212.3 | SEQ ID-Nr. | 229 |
| 230. | HLDLGILYY | 162-170 DPAGT1 | NP_001373.2 | SEQ ID-Nr. | 230 |
| 231. | ITDSAGHILY | 76-85 TMP21 | NP_006818.2 | SEQ ID-Nr. | 231 |
| 232. | HTDDPLTWDY | 267-276 HCA66 | NP_060898.1 | SEQ ID-Nr. | 232 |
| 233. | IARNLTQQL | 313-321 ADFP | NP_001113.2 | SEQ ID-Nr. | 233 |
| 234. | IDQTALAVY | 1087-1095 TPP2 | NP_003282.1 | SEQ ID-Nr. | 234 |
| 235. | LEDVVIERY | 41-49 FKBP10 | NP_068758.2 | SEQ ID-Nr. | 235 |
| 236. | QIASFILLR | 316-324 HIMAP4 | NP_060796.1 | SEQ ID-Nr. | 236 |
| 237. | DEHYILTF | 550-557 OSBPL9 | NP_078862.2 | SEQ ID-Nr. | 237 |
| 238. | DEIGLPKIFY | 124-133 IQGAP1 | NP_003861.1 | SEQ ID-Nr. | 238 |
| 239. | DEIVRINGY | 132-140 USHlC | NP_005700.1 | SEQ ID-Nr. | 239 |
| 240. | DEKLLYDTF | 112-120 SF3B4 | NP_005841.1 | SEQ ID-Nr. | 240 |
| 241. | RIIEETLALK | 9-18 ARPC2 | NP_005722.1 | SEQ ID-Nr. | 241 |
| 242. | GTDELRLLY | 107-115 FLJ12525 | NP_112483.1 | SEQ ID-Nr. | 242 |
| 243. | DELEIIEGMKF | 209-219 HSPD1 | NP_002147.2 | SEQ ID-Nr. | 243 |
| 244. | QVDPLSALKY | 649-658 MKLN1 | NP_037387.2 | SEQ ID-Nr. | 244 |
| 245. | DELHYLEVY | 72-80 VPS35 | NP_060676.2 | SEQ ID-Nr. | 245 |
| 246. | EEFELLGKAY | 81-90 EIF3S8 | NP_003743.1 | SEQ ID-Nr. | 246 |
| 247. | QLEDGRTLSDY | 49-59 UBB | NP_061828.1 | SEQ ID-Nr. | 247 |
| 248. | DEFLWREQF | 42-50 FBXW5 | NP_061871.1 | SEQ ID-Nr. | 248 |
| 249. | DEMLSRGF | 185-192 EIF4A1 | NP_001407. | SEQ ID-Nr. | 249 |
| 250. | DEPLLKHWEF | 196-205 HLA-DRA | NP_061984.1 | SEQ ID-Nr. | 250 |
| 251. | PSRDSLPLPV | 418-427 GPSM1 | NP_056412.2 | SEQ ID-Nr. | 251 |
| 252. | NLRETNLDSLP | 422-432 VIM | NP_003371.1 | SEQ ID-Nr. | 252 |
| 253. | DEVKFLTVL | 191-199 ANXA4 | NP-001144.1 | SEQ ID-Nr. | 253 |
| 254. | NEVEKTMEY | 440-448 RSHL2 | NP_114130.3 | SEQ ID-Nr. | 254 |
| 255. | DEVQVVRGHY | 53-62 RPL26 | NP_000978.1 | SEQ ID-Nr. | 255 |
| 256. | DEWLKPELF | 296-304 CGI-26 | NP_057038.1 | SEQ ID-Nr. | 256 |
| 257. | DEYSLVREL | 125-133 TLN1 | NP_006280.2 | SEQ ID-Nr. | 257 |
| 258. | NEFEATQKL | 343-351 NFIL3 | NP_005375.1 | SEQ ID-Nr. | 258 |
| 259. | DELQQPLEL | 704-712 STAT2 | NP_005410.1 | SEQ ID-Nr. | 259 |
| 260. | DVVMTQSPLSL | 20-30 IGKV@ | S40322 | SEQ ID-Nr. | 260 |
| 261. | SEREAIEVF | 358-366 GBP2 | NP_004111 | SEQ ID-Nr. | 261 |
| 262. | RYFYHQEEY | 21-29 HLA-DRB1 | CAA09468 | SEQ ID-Nr. | 262 |
| 263. | TSALPIIQK | 63-71 ADFP | NP_001113.2 | SEQ ID-Nr. | 263 |
| 264. | RVQEAVESMVK | 8-18 FLJ14668 | AAH 14975 | SEQ ID-Nr. | 264 |
| 265. | TVMELVKIIYK | 237-247 LACTB2 | NP_057111.1 | SEQ ID-Nr. | 265 |
| 266. | RLLQKVLAY | 103-111 FLJ10211 | BAA91493 | SEQ ID-Nr. | 266 |
| 267. | RIHFPLATY | 264-272 K-ALPHA-1 | NP_006073 | SEQ ID-Nr. | 267 |
| 268. | VGGLKNTLVHRL | 279-290 FLJ31579 | NP_695000.1 | SEQ ID-Nr. | 268 |
| 269. | QAQADSLTVY | 679-688 PCDHB5 | AAP97251.1 | SEQ ID-Nr. | 269 |
| 270. | VLDPYLLKY | 34-42 MRPS17 | NP_057053.1 | SEQ ID-Nr. | 270 |
| 271. | IFSPPFPLFY | 83-92 FKSG63 | AAK08108.1 | SEQ ID-Nr. | 271 |
| 272. | TELLLKEGF | 260-268 SND1 | NP_055205.1 | SEQ ID-Nr. | 272 |
| 273. | GLFEVGAGWIGK | 235-246 HSD17B4 | NP_000405.1 | SEQ ID-Nr. | 273 |
| 274. | YEYKFGFEL | 97-105 TXNIP | NP_006463.2 | SEQ ID-Nr. | 274 |
| 275. | WPLWRLVSL | 2-10 BGN | NP_001702.1 | SEQ ID-Nr. | 275 |
| 276. | YIDEQFERY | 121-129 NEDD5 | NP_004395.1 | SEQ ID-Nr. | 276 |
| 277. | YLDEKLALLNA | 897-907 BAIAP3 | NP_003924.2 | SEQ ID-Nr. | 277 |
| 278. | DEHLITFF | 1248-1255 U5-200KD | NP_054733.2 | SEQ ID-Nr. | 278 |
| 279. | DDFHIYVY | 234-241 SPIN | NP_006708 | SEQ ID-Nr. | 279 |
| 280. | APRTVLLLL | 5-13 HLA-A,-B or -C | AAL30417.1 | SEQ ID-Nr. | 280 |
| 281. | APRTVALTALL | 9-19 HLA-DPB1 | NP_002112 | SEQ ID-Nr. | 281 |
| 282. | FTDVNSILRY | 58-67 EPRS | AAH58921 | SEQ ID-Nr. | 282 |
| 283. | YSEEECRQY | 61-69 GNAI2 | NP_002061.1 | SEQ ID-Nr. | 283 |
| 284. | YSEKIVDMY | 134-142 MYH11 | NP_002465.1 | SEQ ID-Nr. | 284 |
| 285. | YTDLLRLFEY | 68-77 PPP1CB | NP_002700.1 | SEQ ID-Nr. | 285 |
| 286. | YVDPQFLTY | 341-349 PJA1 | NP_071763.2 | SEQ ID-Nr. | 286 |
| 287. | HERTFLLEY | 96-104 SNX6 | NP_067072.2 | SEQ ID-Nr. | 287 |
| 288. | SSVPGVRLLQDSVD FSL | 72-88 VIM | NP_003371.1 | SEQ ID-Nr. | 288 |
| 289. | SLLTSSKGQLQK | 369-380 ADFP | NP_0011113.2 | SEQ ID-Nr. | 289 |
| 290. | SPRENILVSL | 281-290 SCD | NP_005054.2 | SEQ ID-Nr. | 290 |
| 291. | DEVDIKSRAAY | 18-28 FTO | XP_051200.4 | SEQ ID-Nr. | 291 |
| 292. | TSPSQSLFY | 154-162 SLC11A1 | AAH41787.1 | SEQ ID-Nr. | 292 |
| 293. | YTETEPYHNY | 392-401 LOC 124245 | NP_653205.2 | SEQ ID-Nr. | 293 |
| 294. | SSVPGVRLLQDSVD F | 72-86 VIM | NP_003371.1 | SEQ ID-Nr. | 294 |
| 295. | VALISPKDI | Leserahmen -1 Symbol existiert nicht; Genart: expressed sequence tag | AC079587.4 | SEQ ID-Nr. | 295 |
| 296. | STDKAEYTFY | 332-341 RBPSUH | NP_005340.2 | SEQ ID-Nr. | 296 |
| 297. | VTEIFRQAF | 250-258 GARNL1 | BAA74907.1 | SEQ ID-Nr. | 297 |
| 298. | SVLSPLLNK | 380-388 EPS8 | NP_004438.2 | SEQ ID-Nr. | 298 |

| **Sequenzen von RCC100** | | | | | |
|---|---|---|---|---|---|
| 299. | RAFSSLGLLK | 615-624 UMOD | NP_003352.1 | SEQ ID-Nr. | 299 |
| 300. | FSKLRPLISK | 243-252 PGBD3 | NP_736609.1 | SEQ ID-Nr. | 300 |
| 301. | RTFTWLVGK | 3S3-361 MYO1C | NP_203693.2 | SEQ ID-Nr. | 301 |
| 302. | KVANIILSY | 1273-1281 FLJ21439 | NP_079413.2 | SEQ ID-Nr. | 302 |
| 303. | TMLARLASA | 21-29 CSPG4 | NP_001888.1 | SEQ ID-Nr. | 303 |
| 304. | HELPLPHSV | 39-47 EPAS1 | NP_001421.2 | SEQ ID-Nr. | 304 |

| **Sequenzen von RCC103** | | | | | |
|---|---|---|---|---|---|
| 305. | AVQRTLLEK | 177-185 CD99 | NP_002405.1 | SEQ ID-Nr. | 305 |
| 306. | ETRPAGDGTFQKW | 256-268 HLA-A | NP_002107 | SEQ ID-Nr. | 306 |
| 307. | AVLSILPAIFQK | 392-403 KIAA0033 | XP_084530.5 | SEQ ID-Nr. | 307 |
| 308. | EIAGHIMEF | 848-856 PUM1 | NP_055491.1 | SEQ ID-Nr. | 308 |
| 309. | ELIRTIMGW | 131-139 BAX | NP_004315.1 | SEQ ID-Nr. | 309 |
| 310. | EVFPLKVFGY | 45-54 ZNF258 | AAH29439 | SEQ ID-Nr. | 310 |
| 311. | ATPTSPIRVK | 856-865 FLNA | NP_001447.1 | SEQ ID-Nr. | 311 |
| 312. | AVLYQPLFDK | 107-116 NAPIL1 | NP_004528.1 | SEQ ID-Nr. | 312 |
| 313. | EVVDFIQSKI | 451-460 PPM1G | NP_817092 | SEQ ID-Nr. | 313 |
| 314. | AVQEFGLARFK | 142-152 PX19 | NP_037369.1 | SEQ ID-Nr. | 314 |
| 315. | EAIQDLWQW | 282-290 NPM1 | NP_002511.1 | SEQ D-Nr. | 315 |
| 316. | GVIRSLMAF | 60-68 SF3B3 | NP_036558.2 | SEQ ID-Nr. | 316 |
| 317. | HIISGTCASW | 241-250 TXNIP | NP_006463.2 | SEQ ID-Nr. | 317 |
| 318. | GVIDVITKTW | 261-270 MFTC | NP_110407.2 | SEQ ID-Nr. | 318 |
| 319. | GVIDLIFEK | 600-608 EIF4G1 | NP_004944.2 | SEQ ID-Nr. | 319 |
| 320. | GVCHIFASF | 29-37 RPS 14 | NP_005608.1 | SEQ ID-Nr. | 320 |
| 321. | GTYVSSVPR | 242-250 HLA-DOA | NP_002110.1 | SEQ ID-Nr. | 321 |
| 322. | GTAGLLEQWLK | 329-339 DC12 | NP_064572.1 | SEQ ID-Nr. | 322 |
| 323. | HVITGLLEHY | 133-142 SCRN2 | NP_612364.1 | SEQ ID-Nr. | 323 |
| 324. | GTADELVLHSW | 176-186 LYPLAL1 | NP_620149.1 | SEQ ID-Nr. | 324 |
| 325. | EIKEVILEF | 873-881 VPS 13C | NP_060154 | SEQ ID-Nr. | 325 |
| 326. | EEASLLHQF | 741-749 SPTBN1 | NP_003119.1 | SEQ ID-Nr. | 326 |
| 327. | KLFIGGLSF | 15-23 HNRPA1 | NP_002127.1 | SEQ ID-Nr. | 327 |
| 328. | DVVPAVRKW | 134-142 MASA | NP_067027.1 | SEQ ID-Nr. | 328 |
| 329. | DVTGVVRQW | 200-208 TGFB1 | NP_000651.1 | SEQ ID-Nr. | 329 |
| 330. | DVKDYIQEY | 2500-2508 KIAA1554 | XP_290768.3 | SEQ ID-Nr. | 330 |
| 331. | DVIDNDSWRLW | 207-217 PAICS | NP_006443.1 | SEQ ID-Nr. | 331 |
| 332. | DVFSSKGMTRW | 90-100 RASSF6 | NP_803876.1 | SEQ ID-Nr. | 332 |
| 333. | DTVKKIESF | 3197-3205 RANBP2 | NP_006258.2 | SEQ ID-Nr. | 333 |
| 334. | DLPSNHVIDRW | 211-221 SKB1 | NP_006100.2 | SEQ ID-Nr. | 334 |
| 335. | DLIGHIVEF | 726-734 PUM2 | NP_056132.1 | SEQ ID-Nr. | 335 |
| 336. | DKESQLEAY | 106-114 LOC284680 | NP_872387.1 | SEQ ID-Nr. | 336 |
| 337. | EVIKLKGYTSW | 240-250 LDHA | NP_005557.1 | SEQ ID-Nr. | 337 |
| 338. | GSSDVIIHR | 519-527 KIAA1542 | XP_290536.2 | SEQ ID-Nr. | 338 |
| 339. | GTLDYILQR | 158-166 FTO | XP_051200.4 | SEQ ID-Nr. | 339 |
| 340. | EVDKRVHMTW | 326-335 PSMD13 | NP_002808.2 | SEQ ID-Nr. | 340 |
| 341. | SVPYFLFQHW | 197-206 SOAT1 | NP_003092.3 | SEQ ID-Nr. | 341 |
| 342. | SVEEISTLVQK | 93-103 MRPL43 | NP_115488.2 | SEQ ID-Nr. | 342 |
| 343. | STFQQMWISK | 352-361 ACTA2 | NP_001604.1 | SEQ ID-Nr. | 343 |
| 344. | TTIPHALLTW | 1533-1542 BIG1 | NP_006412.1 | SEQ ID-Nr. | 344 |
| 345. | SAFLLLGLFK | 419-428 TAPBP | NP_003181.3 | SEQ ID-Nr. | 345 |
| 346. | NIGDEALIGRW | 637-647 MAGED4 | NP_110428.2 | SEQ ID-Nr. | 346 |
| 347. | TVAFVPISGW | 187-196 EEF1A1 | NP_001393.1 | SEQ ID-Nr. | 347 |
| 348. | ETVNLRSLGF | 1930-1939 AIM1 | XP_166300.3 | SEQ ID-Nr. | 348 |
| 349. | MPKFSMPGF | 72-80 AHNAK | BAC87652.1 | SEQ ID-Nr. | 349 |
| 350. | EVMEIMSRF | 98-106 POLH | NP_006493.1 | SEQ ID-Nr. | 350 |
| 351. | EVMDVFLRF | 695-703 CSGlcA-T | XP_376724.1 | SEQ ID-Nr. | 351 |
| 352. | RLQEALNLF | 265-273 GNAS | NP_000507.1 | SEQ ID-Nr. | 352 |
| 353. | ETIDWKVFESW | 174-184 CD74 | NP_004346.1 | SEQ ID-Nr. | 353 |
| 354. | ELMEHGVVSW | 39-48 ELMO3 | NP_078988.1 | SEQ ID-Nr. | 354 |
| 355. | ASVAWAVLK | 2-10 CASPR3 | NP_387504.1 | SEQ ID-Nr. | 355 |
| 356. | SVSPVVHVR | 73-81 LOC92906 | NP_612403.2 | SEQ ID-Nr. | 356 |
| 357. | HVVDRDTEAW | 27-36 FLJ35220 | NP_775898.2 | SEQ ID-Nr. | 357 |
| 358. | ETITGLRVW | 6493-6501 NEB | NP_004534.1 | SEQ ID-Nr. | 358 |
| 359. | RQLEDILSTY | 77-86 DKFZp451J0118 | NP_787048.1 | SEQ ID-Nr. | 359 |
| 360. | AIAQAESLRYK | 98-108 RPS3 | NP_000996.2 | SEQ ID-Nr. | 360 |
| 361. | GVLQLGNIVFK | 345-355 MYH9 | NP_002464.1 | SEQ ID-Nr. | 361 |
| 362. | EVINALKQTW | 489-498 LIM | NP_006448.1 | SEQ ID-Nr. | 362 |
| 363. | STAAFFLLR | 407-415 SLC37A4 | NP_001458.1 | SEQ ID-Nr. | 363 |
| 364. | DIYNFPIHAF | 177-186 LOC84549 | NP_115898.2 | SEQ ID-Nr. | 364 |
| 365. | TVVERMLSNW | 1398-1407 PLXNB2 | BAA21571.1 | SEQ ID-Nr. | 365 |
| 366. | TKPWFASQIPF | 210-220 LOC345778 | XP_293971.3 | SEQ ID-Nr. | 366 |

| **Sequenzen von RCC112** | | | | | |
|---|---|---|---|---|---|
| 367. | GRVDFAYKF | 111-119 PHC2 | NP_004418.2 | SEQ ID-Nr. | 367 |
| 368. | GRDLTDYLM | 182-190 ACTG1 | NP_001605.1 | SEQ ID-Nr. | 368 |
| 369. | GRISITGVGF | 101-110 MGC21644 | NP_612501.3 | SEQ ID-Nr. | 369 |
| 370. | GRIVTLISF | 262-270 MCL1 | NP_068779.1 | SEQ ID-Nr. | 370 |
| 371. | GRLDLQYAKL | 622-631 PLEC1 | NP_000436.1 | SEQ ID-Nr. | 371 |
| 372. | GRTNLIVNY | 18-26 ELAVL1 | NP_001410.2 | SEQ ID-Nr. | 372 |
| 373. | RYFDTAVSR | 5-13 HLA-A,-B or -C | AAC17722 | SEQ ID-Nr. | 373 |
| 374. | GRMVQVHEL | 170-178 SEC23A | NP_006355.2 | SEQ ID-Nr. | 374 |
| 375. | FLDASGAKLDY | 53-63 BZW1 | NP_055485.2 | SEQ ID-Nr. | 375 |
| 376. | ATDYHVRVY | 348-356 FAD104 | NP_073600.2 | SEQ ID-Nr. | 376 |
| 377. | ARLPWAGQL | 624-632 PBXIP1 | NP_065385.2 | SEQ ID-Nr. | 377 |
| 378. | YGMPRQIL | 192-199 TAGLN2 | NP_003555.1 | SEQ ID-Nr. | 378 |
| 379. | GRLLVATTF | 385-393 IARS | NP_002152.1 | SEQ ID-Nr. | 379 |
| 380. | AGGDWFTSR | 136-144 PPP2R1A | NP_055040.2 | SEQ ID-Nr. | 380 |
| 381. | GRAPISNPGM | 179-188 RPA2 | NP_002937 | SEQ ID-Nr. | 381 |
| 382. | GRMENLASYR | 308-317 PPP1R3C | NP_005389 | SEQ ID-Nr. | 382 |
| 383. | VLPKSRVEL | 89-97 HLA-DOA | BAA81787 | SEQ ID-Nr. | 383 |
| 384. | DAKIRIFDL | 28-36 RPL10 | NP_006004.1 | SEQ ID-Nr. | 384 |
| 385. | GRAMVARLGL | 2-11 CD24 | NP_037362.1 | SEQ ID-Nr. | 385 |
| 386. | FIDASRLVY | 612-620 CTNNA1 | NP_001894.1 | SEQ ID-Nr. | 386 |
| 387. | DPMKARVVL | 21-29 SRP9 | NP_003124.1 | SEQ ID-Nr. | 387 |
| 388. | FRFDPQFAL | 77-85 HLA-DQA1 | XP_371812 | SEQ ID-Nr. | 388 |
| 389. | DTDHYFLRY | 165-173 PIGT | NP_057021.2 | SEQ ID-Nr. | 389 |
| 390. | ELLIRKLPF | 60-68 HIST3H3 | NP_003484.1 | SEQ ID-Nr. | 390 |
| 391. | EAFVRHIL | 142-149 MYL6 | NP_066299.2 | SEQ ID-Nr. | 391 |
| 392. | RYFDTAMSR | 5-13 HLA-A,-B or -C | AAB48498.1 | SEQ ID-Nr. | 392 |
| 393. | GRVFIISKY | 416-424 FLJ31657 | NP_689971 | SEQ ID-Nr. | 393 |
| 394. | TFRPAAMLVER | 154-164 LAMB2 | NP_002283.2 | SEQ ID-Nr. | 394 |
| 395. | YLLEKSRAI | 257-265 MYH9 | NP_002464.1 | SEQ ID-Nr. | 395 |
| 396. | LSDLGKLSY | 353-361 MYST1 | NP_115564.1 | SEQ ID-Nr. | 396 |
| 397. | VTDSIRDEY | 258-266 DNM1L | NP_005681.1 | SEQ ID-Nr. | 397 |
| 398. | LTDRELEEY | 567-575 ADD1 | NP_001110.2 | SEQ ID-Nr. | 398 |
| 399. | LTDRGVMSY | 252-260 IRF3 | NP_001562.1 | SEQ ID-Nr. | 399 |
| 400. | KGLSVFLNR | 527-535 GPNMB | NP_002501.1 | SEQ ID-Nr. | 400 |
| 401. | VTDNRAFGY | 128-136 DAB2 | NP_001334.1 | SEQ ID-Nr. | 401 |
| 402. | STDVSDLLHQY | 257-267 PSMB8 | NP_004150.1 | SEQ ID-Nr. | 402 |
| 403. | RSLPFFSAR | 135-143 TRAPPC1 | NP_067033.1 | SEQ ID-Nr. | 403 |
| 404. | YRFMGTEAY | 378-386 SLC3A1 | NP_000332.1 | SEQ ID-Nr. | 404 |
| 405. | MPLLRQEEL | 394-402 EHD2 | NP_055416.2 | SEQ ID-Nr. | 405 |
| 406. | VTEIDQDKY | 2380-2388 FLNA | NP_001447.1 | SEQ ID-Nr. | 406 |
| 407. | MRHLGAFLF | 1-9 TCN2 | NP_000346.2 | SEQ ID-Nr. | 407 |
| 408. | TTEESLRNYY | 20-29 HNRPA2B1 | NP_002128.1 | SEQ ID-Nr. | 408 |
| 409. | MRTSYLLLF | 1-9 DEFB1 | NP_005209.1 | SEQ ID-Nr. | 409 |
| 410. | TVDQVKDLY | 882-890 CP | NP_000087.1 | SEQ ID-Nr. | 410 |
| 411. | MRYVASYLL | 1-9 RPLP2 | NP_000995.1 | SEQ ID-Nr. | 411 |
| 412. | VGLIRNLAL | 511-519 CTNNB1 | NP_001895.1 | SEQ ID-Nr. | 412 |
| 413. | GRLDAVLQR | 317-325 PML | NP_002666.1 | SEQ ID-Nr. | 413 |
| 414. | LLDQGQLNKY | 421-430 CLTC | NP_004850.1 | SEQ ID-Nr. | 414 |
| 415. | NRFAGFGIGL | 98-107 LOC91137 | NP_620128.1 | SEQ ID-Nr. | 415 |
| 416. | KRLGTLVVTY | 305-314 GBP4 | NP_443173.2 | SEQ ID-Nr. | 416 |
| 417. | KRGDVIYIL | 319-327 SCAP2 | NP_003921.2 | SEQ ID-Nr. | 417 |
| 418. | SRFDIPLGL | 1103-1111 PCF11 | NP_056969.2 | SEQ ID-Nr. | 418 |
| 419. | STDPSVLGKY | 101-110 HES1 | NP_005515.1 | SEQ ID-Nr. | 419 |
| 420. | SRFLKSDLF | 130-138 RGS10 | NP_002916.1 | SEQ ID-Nr. | 420 |
| 421. | VQKPSYYVR | 211-219 ADFP | NP_001113.2 | SEQ ID-Nr. | 421 |
| 422. | SRISLPLPNF | 409-418 VIM | NP_003371.1 | SEQ ID-Nr. | 422 |
| 423. | LRSGLPLLL | 231-239 MADHIP | NP_004790.1 | SEQ ID-Nr. | 423 |
| 424. | SFKDYIQER | 330-338 ETS2 | NP_005230.1 | SEQ ID-Nr. | 424 |
| 425. | HTQGPVDGSLY | 104-114 TENS1 | NP_073585.6 | SEQ ID-Nr. | 425 |
| 426. | STDKFKTDFY | 271-280 COPS6 | NP_006824.2 | SEQ ID-Nr. | 426 |

| **Sequenzen von RCC115** | | | | | |
|---|---|---|---|---|---|
| 427. | GSHSMRYFF | 25-33 HLA-A | NP_002107 | SEQ ID-Nr. | 427 |
| 428. | GSHSMRYFFT | 25-34 HLA-A | NP_002107 | SEQ ID-Nr. | 428 |
| 429. | GSHSMRYFH | 25-33 HLA-B | I37515 | SEQ ID-Nr. | 429 |
| 430. | AAILGMHNL | 135-143 TMOD3 | NP_055362.1 | SEQ ID-Nr. | 430 |
| 431. | KLDPTKTTL | 275-283 NDRG1 | NP_006087.2 | SEQ ID-Nr. | 431 |
| 432. | FVHDLVLYL | 783-791 CLTCL1 | NP_001826.1 | SEQ ID-Nr. | 432 |
| 433. | FVHDLVL | 783-789 CLTCL1 | NP_001826.1 | SEQ ID-Nr. | 433 |
| 434. | VLIPKLPQL | 134-142 ORMDL3 | NP_644809.1 | SEQ ID-Nr. | 434 |
| 435. | NEITIPVTF | 177-185 HSPB1 | NP_001531.1 | SEQ ID-Nr. | 435 |
| 436. | YLADFLLTK | 255-263 SLC17A3 | NP_006623.1 | SEQ ID-Nr. | 436 |
| 437. | YLIPLLERL | 139-147 DDX6 | NP_004388.1 | SEQ ID-Nr. | 437 |
| 438. | NEVVTREY | 18-2S RPL31 | NP_000984.1 | SEQ ID-Nr. | 438 |
| 439. | DEFKIGELF | 145-153 PRKDC | NP_008835 | SEQ ID-Nr. | 439 |
| 440. | IQRTPKIQVYS | 21-31 B2M | NP_004039.1 | SEQ ID-Nr. | 440 |
| 441. | LTGPVMPVR | 150-158 RPL13 | NP_000968.2 | SEQ ID-Nr. | 441 |
| 442. | AVAIKAMAK | 146-154 EIF5A | NP_001961.1 | SEQ ID-Nr. | 442 |
| 443. | FVQMMTAK | 142-149 CALM1 | NP_008819.1 | SEQ ID-Nr. | 443 |
| 444. | ATDPNILGR | 4111-4119 PRKDC | NP_008835.5 | SEQ ID-Nr. | 444 |
| 445. | LLLLSIVIL | 212-220 EDG1 | NP_001391.2 | SEQ ID-Nr. | 445 |
| 446. | KLPNFGFVVF | 376-385 G3BP | NP_005745.1 | SEQ ID-Nr. | 446 |
| 447. | KLSEIDVAL | 174-182 EFHD1 | NP_079478.1 | SEQ ID-Nr. | 447 |
| 448. | LAALPHSCL | 5-13 RGS5 | NP_003608.1 | SEQ ID-Nr. | 448 |
| 449. | YSIITPNILRL | 26-36 C3 | NP_000055.1 | SEQ ID-Nr. | 449 |
| 450. | ALPSRILLWK | 2-11 MGC3047 | NP_115724.1 | SEQ ID-Nr. | 450 |
| 451. | VKGFYPSDIAVE | 247-258 IGHG2 | AAB59393.1 | SEQ ID-Nr. | 451 |
| 452. | FLLDLSRSV | 92-100 GPR31 | NP_005290.1 | SEQ ID-Nr. | 452 |
| 453. | IIYKGGTSR | 545-553 GSN | NP_000168.1 | SEQ ID-Nr. | 453 |
| 454. | IVADHVASY | 3-11 MHC class II | AAC41957 | SEQ ID-Nr. | 454 |
| 455. | EVGGEALGRLL | 23-33 HBB | NP_000509.1 | SEQ ID-Nr. | 455 |
| 456. | RTGPPMGSRF | 175-184 WBSCR1 | NP_071496.1 | SEQ ID-Nr. | 456 |
| 457. | RQIQESVTF | 1305-1313 ANK2 | NP_001139.2 | SEQ ID-Nr. | 457 |
| 458. | RVAPEEHPV | 95-103 ACTB | NP_001092.1 | SEQ ID-Nr. | 458 |
| 459. | TLADLLALR | 1433-1441 DNAH11 | NP_003768.1 | SEQ ID-Nr. | 459 |
| 460. | RVAPEEHPVLLT | 95-106 ACTB | NP_001092.1 | SEQ ID-Nr. | 460 |
| 461. | TLADIIARL | 1487-1495 KIAA1305 | XP_370756.2 | SEQ ID-Nr. | 461 |
| 462. | RWEDGSPLNF | 142-151 KLRG1 | NP_005801.2 | SEQ ID-Nr. | 462 |
| 463. | YEVSQLKD | 468-475 CNDP2 | NP_060705.1 | SEQ ID-Nr. | 463 |
| 464. | YRDIPELQGF | 663-672 AACS | NP_076417 | SEQ ID-Nr. | 464 |
| 465. | YVDGTQFVRF | 51-60 HLA-A,-B or -C | BAA04965 | SEQ ID-Nr. | 465 |
| 466. | SLLDEFYKL | 184-192 M11S1 | NP_005889.3 | SEQ ID-Nr. | 466 |
| 467. | HGIDPTGTY | 28-36 TUBB5 | NP_006078.2 | SEQ ID-Nr. | 467 |
| 468. | SLDKFLASVSTVL | 125-137 HBA1 | NP_000549.1 | SEQ ID-Nr. | 468 |
| 469. | SIGERDLIFH | 289-298 TIMELESS | NP_003911.1 | SEQ ID-Nr. | 469 |
| 470. | STTSVFITK | 1788-1796 TRRAP | NP_003487.1 | SEQ ID-Nr. | 470 |
| 471. | FGEEILLESDLF | 28-38 CRYAB | NP_001876.1 | SEQ D-Nr. | 471 |
| 472. | FLDPIKAYL | 76-84 GPR116 | NP_056049.3 | SEQ ID-Nr. | 472 |
| 473. | FLADPSAFVAA | 268-278 RPLP0 | NP_000993.1 | SEQ ID-Nr. | 473 |
| 474. | ITAPPSRVL | 20-28 SCD | NP_005054.2 | SEQ ID-Nr. | 474 |
| 475. | VLDELKNMKC | 170-179 CYFIP2 | NP_055191.1 | SEQ ID-Nr. | 475 |
| 476. | LLGPRLVLA | 23-31 TMP21 | NP_006818.2 | SEQ ID-Nr. | 476 |
| 477. | IIMPHNIYL | 251-259 SLC11A1 | NP_000569.2 | SEQ ID-Nr. | 477 |
| 478. | LVRMVLNG | 144-151 MYL6 | NP_034990 | SEQ ID-Nr. | 478 |
| 479. | RLYGPSSVSF | 133-142 SERPINH1 | NP_001226.2 | SEQ ID-Nr. | 479 |
| 480. | FEAPIKLVF | 236-244 HM13 | NP_10416.1 | SEQ ID-Nr. | 480 |
| 481. | IQPGAVKVY | 1472-1480 C3 | NP_000055.1 | SEQ ID-Nr. | 481 |
| 482. | VLAEVPTQL | 501-509 CPNE1 | NP_003906.1 | SEQ ID-Nr. | 482 |
| 483. | IMRAGMSSL | 521-529 CCT6A | NP_001753.1 | SEQ ID-Nr. | 483 |
| 484. | VEFSSGLKGMSL | 96-107 ATP5A1 | NP_004037.1 | SEQ ID-Nr. | 484 |
| 485. | ILNPDNSFEIL | 241-251 CANX | NP_001737.1 | SEQ ID-Nr. | 485 |
| 486. | VALEFALHL | 344-352 CABLES1 | NP_612384.1 | SEQ ID-Nr. | 486 |
| 487. | TVAVPLVGK | 22-30 MGC3067 | NP_077271.1 | SEQ ID-Nr. | 487 |
| 488. | TLSDLRVYL | 121-129 C200rf139 | NP_542763.1 | SEQ ID-Nr. | 488 |
| 489. | TLIDIMTRF | 35-43 HK1 | NP_000179.1 | SEQ ID-Nr. | 489 |

| **Sequenzen von RCC116** | | | | | |
|---|---|---|---|---|---|
| 490. | HDFPRALIF | 64-72 CG018 | AAH22188 | SEQ ID-Nr. | 490 |
| 491. | GSHSMRYF | 25-32 HLA-A,-B or -C | BAA04965 | SEQ ID-Nr. | 491 |
| 492. | SLMDHTIPEV | 289-298 SDCBP | NP_005616.1 | SEQ ID-Nr. | 492 |
| 493. | SGVHTFPAVLQ | 155-165 Ig heavy chain | AA022172 | SEQ ID-Nr. | 493 |
| 494. | FLVTVIHTL | 1065-1073 PLXNC1 | NP_005752.1 | SEQ ID-Nr. | 494 |
| 495. | TDGKVFQF | 24-31 RPL24 | NP_000977.1 | SEQ ID-Nr. | 495 |
| 496. | YDLLRNTNF | 246-254 DYRK1A | NP_001387.2 | SEQ ID-Nr. | 496 |
| 497. | ILYPKTLFL | 138-146 PPP3CA | NP_000935.1 | SEQ ID-Nr. | 497 |
| 498. | MRYVASYL | 1-8 RPLP2 | NP_000995.1 | SEQ ID-Nr. | 498 |
| 499. | FIWENIHTL | 3725-3733 BPAG1 | NP_056363.2 | SEQ ID-Nr. | 499 |
| 500. | RELPAWVSF | 125-133 MBC2 | NP_056107.1 | SEQ ID-Nr. | 500 |
| 501 | QDLNRIFPL | 81-89 PRG1 | NP_002718.2 | SEQ ID-Nr. | 501 |
| 502. | RDSIVAEL | 97-104 COPE | NP_009194.2 | SEQ ID-Nr. | 502 |
| 503. | ADVLKVEVF | 130-138 ITGB4BP | NP_002203.1 | SEQ ID-Nr. | 503 |
| 504. | YDSIIYRM | 335-342 ATP6AP2 | NP_005756.2 | SEQ ID-Nr. | 504 |
| 505. | AMNPVEHPF | 203-211 RPL8 | NP_000964.1 | SEQ ID-Nr. | 505 |
| 506. | SELIRNVTL | 126-134 U5-116KD | NP_004238.2 | SEQ ID-Nr. | 506 |
| 507. | QDVARVLGF | 117-125 PNMA1 | NP_006020.3 | SEQ ID-Nr. | 507 |
| 508. | SDHIHIIAL | 215-223 OTUB1 | NP_060140.1 | SEQ ID-Nr. | 508 |
| 509. | ADSLRLQQL | 781-789 SPTAN1 | NP_003118.1 | SEQ ID-Nr. | 509 |
| 510. | LLDIRSEY | 466-473 ANXA11 | NP_001148.1 | SEQ ID-Nr. | 510 |
| 511. | VLFGLLREV | 663-671 DHX38 | NP_054722.2 | SEQ ID-Nr. | 511 |
| 512. | VAVGRALYY | 510-518 DDB1 | NP_001914.2 | SEQ ID-Nr. | 512 |
| 513. | MRFLAATFL | 1-9 NPC2 | NP_006423.1 | SEQ ID-Nr. | 513 |
| 514. | YTDPEVFKY | 398-406 PTGIS | NP_000952.1 | SEQ ID-Nr. | 514 |
| 515. | HDFLKYDFF | 232-240 SURF4 | NP_149351.1 | SEQ ID-Nr. | 515 |
| 516. | AIDQLHLEY | 525-533 ACTN4 | NP_004915.2 | SEQ ID-Nr. | 516 |
| 517. | SDLERVTSL | 316-324 FLJ21616 | NP_078843.2 | SEQ ID-Nr. | 517 |
| 518. | TLLPLRVFL | 128-136 FLJ90013 | NP_699196.1 | SEQ ID-Nr. | 518 |
| 519. | YSIITPNILR | 26-35 C3 | NP_000055.1 | SEQ ID-Nr. | 519 |
| 520. | FELQRNFQL | 19-27 ING4 | NP_057246.2 | SEQ ID-Nr. | 520 |
| 521. | LDLQRNYIF | 186-194 UNQ3030 | NP_940967.1 | SEQ ID-Nr. | 521 |
| 522. | RRLDPIPQL | 56-64 MGC8721 | MP_057211.4 | SEQ ID-Nr. | 522 |
| 523. | SLPIKESEIIDF | 85-96 RPS2 | NP_002943.2 | SEQ ID-Nr. | 523 |
| 524. | TELLRYYML | 292-300 SNX5 | NP_055241.1 | SEQ ID-Nr. | 524 |
| 525. | FIYHGEVPQA | 254-263 MHC2TA | NP_000237.1 | SEQ ID-Nr. | 525 |
| 526. | AEMLRSISF | 217-225 CSTF1 | NP_001315.1 | SEQ ID-Nr. | 526 |
| 527. | RLQEDPPVGV | 15-24 UBE2B | NP_003328.1 | SEQ ID-Nr. | 527 |
| 528. | AELERAAAL | 465-473 FLJ35453 | NP_689813.1 | SEQ ID-Nr. | 528 |
| 529. | YTDKIDRY | 107-114 TM4SF7 | NP_003262.1 | SEQ ID-Nr. | 529 |
| 530. | FLLPDVIRI | 329-337 TBC1D13 | NP_060671.2 | SEQ ID-Nr. | 530 |
| 531. | VELPHINLL | 169-177 FLJ10349 | NP_060536.2 | SEQ ID-Nr. | 531 |
| 532. | VMLDVPIRL | 725-733 RASAL2 | NP_004832.1 | SEQ ID-Nr. | 532 |
| 533. | SLLENLEKI | 209-216 HNRPC | NP_112604.1 | SEQ ID-Nr. | 533 |
| 534. | YADPVNAHY | 226-234 ROD1 | NP_005147.3 | SEQ ID-Nr. | 534 |
| 535. | AELLRGLSL | 165-173 FBXL5 | NP_036293.1 | SEQ ID-Nr. | 535 |
| 536. | TTEVHPELY | 51-59 SDBCAG84 | NP_057050.1 | SEQ ID-Nr. | 536 |
| 537. | RETNLDSLP | 424-432 VIM | NP_003371.1 | SEQ ID-Nr. | 537 |
| 538. | ELEDSTLRY | 543-551 PLEC1 | NP_000436.1 | SEQ ID-Nr. | 538 |

| **Sequenzen von RCC130** | | | | | |
|---|---|---|---|---|---|
| 539. | FLDIYIFL | 84-91 LOC390875 | XP_372703.1 | SEQ ID-Nr. | 539 |
| 540. | TYTDRVFFL | 1282-1290 PLXNB2 | BAA21571.1 | SEQ ID-Nr. | 540 |
| 541. | SPHLANYFYF | 147-156 Symbol existiert nicht; Genart: unnamed protein product | BAC87422 | SEQ ID-Nr. | 541 |
| 542. | SPRLPVGGF | 1921-1929 TRIP12 | XP_376178.1 | SEQ ID-Nr. | 542 |
| 543. | KLLDKVQAYS | 9-18 GJA1 | NP_000156.1 | SEQ ID-Nr. | 543 |
| 544. | AYQHLFYLL | 955-963 IQGAP3 | NP_839943.2 | SEQ ID-Nr. | 544 |
| 545. | KYILLMDIIA | 148-157 TBX3 | NP_005987.2 | SEQ ID-Nr. | 545 |
| 546. | RYSSMAASF | 82-90 MAP17 | NP_005753.1 | SEQ ID-Nr. | 546 |
| 547. | SPRAAEPVQL | 397-406 CA9 | NP_001207.1 | SEQ ID-Nr. | 547 |
| 548. | IYTSSVNRL | 535-543 COPB2 | NP_004757.1 | SEQ ID-Nr. | 548 |
| 549. | LYPQFMFHL | 576-584 SEC23A | NP_006355.2 | SEQ ID-Nr. | 549 |
| 550. | RYIPTAAAF | 415-423 SEC61A1 | NP_037468.1 | SEQ ID-Nr. | 550 |
| 551. | EYIVKKIPV | 237-245 EIF2S3 | NP_001406.1 | SEQ ID-Nr. | 551 |
| 552. | SRVEAVYVL | 13-21 PADI2 | NP_031391.1 | SEQ ID-Nr. | 552 |
| 553. | MPRGVVVTL | 851-859 HECTD1 | NP_056197.1 | SEQ ID-Nr. | 553 |
| 554. | LPKPPGRGV | 341-349 FBXL6 | NP_036294.1 | SEQ ID-Nr. | 554 |
| 555. | RLWGEPVNL | 1665-1673 USP9X | NP_004643.2 | SEQ ID-Nr. | 555 |
| 556. | RLLDVLAPL | 14-22 COL18A1 | NP_569712.1 | SEQ ID-Nr. | 556 |
| 557. | LYILSSHDI | 474-482 FBXO24 | NP_277041.1 | SEQ ID-Nr. | 557 |
| 558. | TPMGPGRTV | 235-243 LGALS8 | NP_006490.3 | SEQ ID-Nr. | 558 |
| 559. | GPPGTGKTDVAVQI | 823-836 AQR | NP_055506.1 | SEQ ID-Nr. | 559 |
| 560. | NEIEDTFRQF | 46-55 ATP6V1F | NP_004222.2 | SEQ ID-Nr. | 560 |
| 561. | EEIDLRSVGW | 315-324 UNC93B1 | NP_112192.2 | SEQ ID-Nr. | 561 |
| 562. | KYQKGFSLW | 245-253 TRAM1 | NP_055109.1 | SEQ ID-Nr. | 562 |
| 563. | VYPDGIRHI | 519-527 SF3B3 | NP_036558.2 | SEQ ID-Nr. | 563 |
| 564. | KFIDTTSKF | 366-374 RPL3L | NP_005052.1 | SEQ ID-Nr. | 564 |
| 565. | FLDILNTLI | 1729-1737 DNAH8 | NP_001362.1 | SEQ ID-Nr. | 565 |
| 566. | KYTTQGQLLQF | 200-210 ELOVL5 | NP_068586.1 | SEQ ID-Nr. | 566 |
| 567. | KYLSVQGQLF | 344-353 MTCH1 | NP_05S156.1 | SEQ ID-Nr. | 567 |
| 568. | RYFDEPVEL | 355-363 ARFGAP3 | NP_055385.2 | SEQ ID-Nr. | 568 |
| 569. | KYDEIFYNL | 452-460 EHD2 | NP_055416.2 | SEQ ID-Nr. | 569 |
| 570. | SYIEHIFEI | 61-69 PEA15 | NP_003759.1 | SEQ ID-Nr. | 570 |
| 571. | KFIDPIYQVW | 572-581 RRN3 | NP_060897.2 | SEQ ID-Nr. | 571 |
| 572. | LGYTEGALLAL | 1370-1380 PCDH15 | NP_149045.2 | SEQ ID-Nr. | 572 |
| 573. | KYPSPFFVF | 2-10 DHX9 | NP_085077.1 | SEQ ID-Nr. | 573 |
| 574. | EYPDRIMNTF | 158-167 TUBB4 | NP_006077.1 | SEQ ID-Nr. | 574 |
| 575. | VYISEHEHF | 107-115 CLPTM1 | NP_001285.1 | SEQ ID-Nr. | 575 |
| 576. | KYFLKPEVL | 167-175 KIAA1363 | NP_065843.2 | SEQ ID-Nr. | 576 |

| **Sequenz von JY** | | | | | |
|---|---|---|---|---|---|
| 577. | GPALGRSFL | 78-86 TNFSF7 | NP_001243.1 | SEQ ID-Nr. | 577 |

| **Sequenzen der Kontrollpeptide** | | | | | |
|---|---|---|---|---|---|
| 578. | ELAGIGILTV | 26-35 MLANA(modi fied A27->L) | NP_005502.1 | SEQ ID-Nr. | 578 |
| 579. | ILKEPVHGV | 896-904 pol | NP_0578494 | SEQ ID-Nr. | 579 |
| 580. | GILGFVFTL | 58-66 Symbol existiert nicht; Genart: matrix protein M 1 | S 14616 | SEQ ID-Nr. | 580 |
| 581. | NLVPMVATV | 495-503 Symbol existiert nicht; Genart: pp65 | P06725 | SEQ ID-Nr. | 581 |
| 582. | LLDFVRFMGV | 284-293 Symbol existiert nicht; Genart: EBNA-6 nuclear protein | P03204 | SEQ ID-Nr. | 582 |
| 583. | GLCTLVAML | 259-267 Symbol existiert nicht; Genart: Immediate-early transactivator | NP_039857.1 | SEQ ID-Nr. | 583 |
| 584. | CLGGLLTMV | 294-302 Symbol existiert nicht; Genart: latent membrane protein 2 | AAB59844.1 | SEQ ID-Nr. | 584 |
| 585. | APRTVALTA | 9-17 HLA-DPB1 | NP_002112 | SEQ ID-Nr. | 585 |

## Patentansprüche

1. Tumor-assoziiertes Peptid, wobei das Peptid die Fähigkeit aufweist, an ein Molekül des menschlichen Haupt-Histokompatibilitäts-Komplexes (MHC) Klasse-I zu binden, ausgewählt aus,
a) einer Aminosäuresequenz bestehend aus SEQ ID Nr. 303 (TMLARLASA),
b) einem Peptid gemäß a), wobei eine Aminosäure durch eine andere Aminosäure mit ähnlichen chemischen Eigenschaften ersetzt ist, und
c) einem Peptid gemäß a) oder b), wobei N- oder/und C-terminal eine weitere Aminosäure vorhanden ist.

2. Verwendung eines oder mehrerer Peptide nach Anspruch 1 zur Herstellung eines Arzneimittels zur Behandlung von Tumorerkrankungen und/oder adenomatöser Erkrankungen.

3. Verwendung des Peptids nach Anspruch 1 zur Herstellung eines Medikaments zur Behandlung von Tumorerkrankungen und/oder adenomatöser Erkrankungen.

4. Verwendung nach Anspruch 2 oder 3, **dadurch gekennzeichnet, dass** die Erkrankung Nieren-, Brust-, Pankreas-, Magen-, Gehirn-, Blasen-, Hodenkrebs und/oder eine Tumorerkrankung des Nervensystems ist.

5. Verwendung nach Anspruch 3 oder 4, **dadurch gekennzeichnet, dass** das Peptid zusammen mit einem Adjuvans eingesetzt wird.

6. Verwendung nach Anspruch 3 oder 4, **dadurch gekennzeichnet, dass** das Peptid gebunden auf einer Antigen-präsentierenden Zelle eingesetzt wird.

7. Verwendung des Peptids nach Anspruch 1 zur Herstellung eines Antikörpers.

8. Pharmazeutische Zusammensetzung enthaltend eines oder mehrere Peptide nach Anspruch 1.

9. Nukleinsäuremolekül, das für das Peptid mit der Sequenz ID-Nr. 303
kodiert

10. Zelle, die mit Hilfe des Nukleinsäuremoleküls nach Anspruch 9 genetisch derart verändert wurde, dass sie ein Peptid nach Anspruch 1 produziert.

## Claims

1. Tumour-associated peptide, the peptide being capable of binding to a molecule of the human major histocompatibility complex (MHC) class I, selected from
a) an amino acid sequence consisting of SEQ ID No. 303 (TMLARLASA)
b) a peptide according to a) wherein one amino acid is replaced by another amino acid with similar chemical properties, and
c) a peptide according to a) or b) wherein one additional amino acid is present at the N- or/and C-terminus.

2. Use of one or more peptides according to claim 1 for the preparation of a drug for the treatment of tumour diseases and/or adenomatous diseases.

3. Use of the peptide according to claim 1 for the preparation of a drug for the treatment of tumour diseases and/or adenomatous diseases.

4. Use according to claim 2 or 3, **characterized by** the disease being kidney, mammary, pancreas, gastric, brain, bladder, testicular cancer and/or a tumour disease of the nerve system.

5. Use according to claim 3 or 4, **characterized by** the peptide being used together with an adjuvant.

6. Use according to claim 3 or 4, **characterized by** the peptide being used bound to an antigen presenting cell.

7. Use of the peptide according to claim 1 for preparing an antibody.

8. Pharmaceutical composition comprising one or more peptides according to claim 1.

9. Nucleic acid molecule that encode for the peptide having the sequence ID-No. 303.

10. Cell genetically modified by a nucleic acid molecule according to claim 9, which is producing a peptide according to claim 1.

## Revendications

1. Peptide tumoral, le peptide étant capable de se lier à une molécule de classe 1 du complexe majeur d'histocompatibilité (CMH) humain, choisi parmi
a) une séquence d'acide aminé consistant en SEQ ID N° 303 (TMLARLASA),
b) un peptide selon a), un acide aminé étant remplacé par un autre acide aminé ayant les mêmes propriétés chimiques, et
c) un peptide selon a) ou b), un acide aminé supplémentaire étant présent en position N-terminale ou/et C-terminale.

2. Utilisation d'un ou de plusieurs peptides selon la revendication 1 pour préparer une formulation pharmaceutique destinée au traitement d'affections tumorales et/ou d'affections adénomateuses.

3. Utilisation du peptide selon la revendication 1 pour préparer un médicament destiné au traitement d'affections tumorales et/ou d'affections adénomateuses.

4. Utilisation selon la revendication 2 ou 3, **caractérisée par le fait que** l'affection est un cancer du rein, du sein, du pancréas, de l'estomac, du cerveau, de la vésicule biliaire, du testicule et/ou une affection tumorale du système nerveux.

5. Utilisation selon la revendication 3 ou 4, **caractérisée par le fait que** le peptide est utilisé avec un adjuvant.

6. Utilisation selon la revendication 3 ou 4, **caractérisée par le fait que** le peptide est utilisé lié à une cellule de présentation de l'antigène.

7. Utilisation du peptide selon la revendication 1 pour la préparation d'un anticorps.

8. Composition pharmaceutique contenant un ou plusieurs peptides selon la revendication 1.

9. Molécule d'acide nucléique codant le peptide de séquence ID N° 303.

10. Cellule ayant été génétiquement modifiée à l'aide de la molécule d'acide nucléique selon la revendication 9 de telle sorte qu'elle produise un peptide selon la revendication 1.


### SEQUENZPROTOKOLL

<110> Immatics Biotechnologies GmbH
<120> An MHC-Moleküle bindende Tumor-assoziierte Peptide
<130> I30270PCT
<160> 585
<170> PatentIn version 3.1
<210> 1
   <211> 11
   <212> PRT
   <213> Homo sapiens
<400> 1
   Val Pro Asp Ser Ser Gly Pro Glu Arg Ile Leu
   1               5                   10
<210> 2
   <211> 9
   <212> PRT
   <213> Homo sapiens
<400> 2
   Gly Leu Ala Pro Ser Ile Arg Thr Lys
   1 5
<210> 3
   <211> 9
   <212> PRT
   <213> Homo sapiens
<400> 3
   Arg Leu Phe Glu His Pro Leu Tyr Arg
   1 5
<210> 4
   <211> 9
   <212> PRT
   <213> Homo sapiens
<400> 4
   Thr Pro Ser Glu Pro His Pro Val Leu
   1 5
<210> 5
   <211> 10
   <212> PRT
   <213> Homo sapiens
<400> 5
   Gln Ile Phe Val Lys Thr Leu Thr Gly Lys
   1 5 10
<210> 6
   <211> 9
   <212> PRT
   <213> Homo sapiens
<400> 6
   Ser Leu Met His Ser Phe Ile Leu Lys
   1 5
<210> 7
   <211> 9
   <212> PRT
   <213> Homo sapiens
<400> 7
   Tyr Pro His Leu His Asn Ala Glu Leu
   1 5
<210> 8
   <211> 9
   <212> PRT
   <213> Homo sapiens
<400> 8
   Arg Leu Phe Val Gly Ser Ile Pro Lys
   1 5
<210> 9
   <211> 10
   <212> PRT
   <213> Homo sapiens
<400> 9
   Arg Val Phe Pro Asp Lys Gly Tyr Ser Phe
   1 5 10
<210> 10
   <211> 9
   <212> PRT
   <213> Homo sapiens
<400> 10
   Ser Leu Tyr Lys Lys Leu Glu Ile Lys
   1 5
<210> 11
   <211> 10
   <212> PRT
   <213> Homo sapiens
<400> 11
   His Pro Val Ser Asp His Glu Ala Thr Leu
   1 5 10
<210> 12
   <211> 9
   <212> PRT
   <213> Homo sapiens
<400> 12
   Leu Pro Thr Arg Val Asp Phe Ser Leu
   1 5
<210> 13
   <211> 11
   <212> PRT
   <213> Homo sapiens
<400> 13
   Lys Ser Phe Gly Ser Ala Gln Glu Phe Ala Trp
   1 5 10
<210> 14
   <211> 10
   <212> PRT
   <213> Homo sapiens
<400> 14
   Ser Pro Ser Thr Ser Arg Thr Pro Leu Leu
   1 5 10
<210> 15
   <211> 9
   <212> PRT
   <213> Homo sapiens
<400> 15
   Ser Thr Phe Asp Ser Pro Ala His Trp
   1 5
<210> 16
   <211> 9
   <212> PRT
   <213> Homo sapiens
<400> 16
   Ala Pro Glu Glu His Pro Val Leu Leu
   1 5
<210> 17
   <211> 9
   <212> PRT
   <213> Homo sapiens
<400> 17
   Arg Gln Ile Thr Gln Val Tyr Gly Phe
   1 5
<210> 18
   <211> 9
   <212> PRT
   <213> Homo sapiens
<400> 18
   Lys Val Ser Asp Tyr Ile Leu Gln His
   1 5
<210> 19
   <211> 9
   <212> PRT
   <213> Homo sapiens
<400> 19
   Lys Leu Leu Pro Ser Val Val Leu Lys
   1 5
<210> 20
   <211> 9
   <212> PRT
   <213> Homo sapiens
<400> 20
   Gly Val Leu Lys Lys Val Ile Arg His
   1 5
<210> 21
   <211> 10
   <212> PRT
   <213> Homo sapiens
<400> 21
   Lys Leu Phe Asp His Ala Val Ser Lys Phe
   1 5 10
<210> 22
   <211> 10
   <212> PRT
   <213> Homo sapiens
<400> 22
   Ile Thr Val Leu Thr Lys Pro Leu Pro Val
   1 5 10
<210> 23
   <211> 9
   <212> PRT
   <213> Homo sapiens
<400> 23
   His Pro Val His Pro Asp Ile Lys Leu
   1 5

<210> 24
   <211> 10
   <212> PRT
   <213> Homo sapiens
<400> 24
   Ile Pro Arg Ala Ala Leu Leu Pro Leu Leu
   1 5 10
<210> 25
   <211> 9
   <212> PRT
   <213> Homo sapiens
<400> 25
   Ala Thr Asn Arg Ile Thr Val Thr Trp
   1 5
<210> 26
   <211> 9
   <212> PRT
   <213> Homo sapiens
<400> 26
   Lys Ile Ala Asp Arg Phe Leu Leu Tyr
   1 5
<210> 27
   <211> 10
   <212> PRT
   <213> Homo sapiens
<400> 27
   Asp His Asp Pro Val Asp Lys Ile Val Leu
   1 5 10
<210> 28
   <211> 9
   <212> PRT
   <213> Homo sapiens
<400> 28
   Asp His His Gln Glu Val Ile Gly Phe
   1 5
<210> 29
   <211> 9
   <212> PRT
   <213> Homo sapiens
<400> 29
   Ile His Asp Leu Asp Asn Ile Ser Phe
   1 5
<210> 30
   <211> 9
   <212> PRT
   <213> Homo sapiens
<400> 30
   Asp His Ile Asn Asp Ile Ile Lys Ile
   1 5
<210> 31
   <211> 9
   <212> PRT
   <213> Homo sapiens
<400> 31
   Asp His Met Arg Phe Ile Ser Glu Leu
   1 5
<210> 32
   <211> 9
   <212> PRT
   <213> Homo sapiens
<400> 32
   Thr His Ser Leu Pro Val Val Val Ile
   1 5
<210> 33
   <211> 11
   <212> PRT
   <213> Homo sapiens
<400> 33
   Met Pro Val Gly Pro Asp Ala Ile Leu Arg Tyr
   1 5 10
<210> 34
   <211> 9
   <212> PRT
   <213> Homo sapiens
<400> 34
   Arg Leu Asp Asp Ala Ile His Val Leu
   1 5
<210> 35
   <211> 9
   <212> PRT
   <213> Homo sapiens
<400> 35
   Gln His Glu Gly Thr Val Asn Ile Phe
   1 5
<210> 36
   <211> 9
   <212> PRT
   <213> Homo sapiens
<400> 36
   Glu Thr Val Asn Ile Trp Thr His Phe
   1 5
<210> 37
   <211> 9
   <212> PRT
   <213> Homo sapiens
<400> 37
   Val His Ile Leu Asp Thr Glu Thr Phe
   1 5
<210> 38
   <211> 10
   <212> PRT
   <213> Homo sapiens
<400> 38
   Gln Thr Pro Asp Phe Thr Pro Thr Lys Tyr
   1 5 10
<210> 39
   <211> 9
   <212> PRT
   <213> Homo sapiens
<400> 39
   Arg His Val Glu Val Phe Glu Leu Leu
   1 5
<210> 40
   <211> 9
   <212> PRT
   <213> Homo sapiens
<400> 40
   Thr Thr Ile Asp Ile Gly Val Lys Tyr
   1 5
<210> 41
   <211> 9
   <212> PRT
   <213> Homo sapiens
<400> 41
   Asp Leu Ile Glu His Phe Ser Gln Phe
   1 5
<210> 42
   <211> 9
   <212> PRT
   <213> Homo sapiens
<400> 42
   Glu Thr Val Trp Arg Leu Glu Glu Phe
   1 5
<210> 43
   <211> 9
   <212> PRT
   <213> Homo sapiens
<400> 43
   Asp Val Leu Glu Ser Val Asn Leu Leu
   1 5
<210> 44
   <211> 9
   <212> PRT
   <213> Homo sapiens
<400> 44
   Ile His Asp Asp Phe Val Thr Thr Phe
   1 5
<210> 45
   <211> 9
   <212> PRT
   <213> Homo sapiens
<400> 45
   Ile His Ile Pro Ile Asn Asn Ile Ile
   1 5
<210> 46
   <211> 9
   <212> PRT
   <213> Homo sapiens
<400> 46
   Ile His Leu Ile Asp Pro Asn Thr Leu
   1 5
<210> 47
   <211> 9
   <212> PRT
   <213> Homo sapiens
<400> 47
   Ile His Val Ile Gly Gly Asn Asp Val
   1 5
<210> 48
   <211> 9
   <212> PRT
   <213> Homo sapiens
<400> 48
   Lys Ala Phe Gln Lys Ile Val Val Leu
   1 5
<210> 49
   <211> 9
   <212> PRT
   <213> Homo sapiens
<400> 49
   Tyr Gln Asp Leu Leu Asn Val Lys Leu
   1 5

<210> 50
   <211> 9
   <212> PRT
   <213> Homo sapiens
<400> 50
   Gly His Tyr Glu Val Ala Glu Leu Leu
   1 5
<210> 51
   <211> 10
   <212> PRT
   <213> Homo sapiens
<400> 51
   Leu Val Val Tyr Pro Trp Thr Gln Arg Phe
   1 5 10
<210> 52
   <211> 9
   <212> PRT
   <213> Homo sapiens
<400> 52
   Met His Leu Arg Gln Tyr Glu Leu Leu
   1 5
<210> 53
   <211> 9
   <212> PRT
   <213> Homo sapiens
<400> 53
   Glu Ala Ile Glu Gln Ile Leu Lys Tyr
   1 5
<210> 54
   <211> 11
   <212> PRT
   <213> Homo sapiens
<400> 54
   Asp Val Ala Glu Gly Asp Leu Ile Glu His Phe
   1 5 10
<210> 55
   <211> 8
   <212> PRT
   <213> Homo sapiens
<400> 55
   Asp Val Leu Gln Lys Ile Lys Tyr
   1 5
<210> 56
   <211> 10
   <212> PRT
   <213> Homo sapiens
<400> 56
   Asp Ser Phe Pro Met Glu Ile Arg Gln Tyr
   1 5 10
<210> 57
   <211> 9
   <212> PRT
   <213> Homo sapiens
<400> 57
   Asp Val Ile Ser Asn Ile Glu Thr Phe
   1 5
<210> 58
   <211> 9
   <212> PRT
   <213> Homo sapiens
<400> 58
   Asp Val Ile Arg Leu Ile Met Gln Tyr
   1 5
<210> 59
   <211> 9
   <212> PRT
   <213> Homo sapiens
<400> 59
   Asp Val Ile Glu Arg Val Ile Gln Tyr
   1 5
<210> 60
   <211> 10
   <212> PRT
   <213> Homo sapiens
<400> 60
   Asp Val Ile Ala Gln Gly Ile Gly Lys Leu
   1 5 10
<210> 61
   <211> 10
   <212> PRT
   <213> Homo sapiens
<400> 61
   Asp Val Phe Asn Glu Lys Gly Trp Asn Tyr
   1 5 10
<210> 62
   <211> 9
   <212> PRT
   <213> Homo sapiens
<400> 62
   Thr His Leu Asp Ser Val Thr Lys Ile
   1 5
<210> 63
   <211> 9
   <212> PRT
   <213> Homo sapiens
<400> 63
   Asp Val Ala Gly Ile Ile Ala Asp Tyr
   1 5
<210> 64
   <211> 9
   <212> PRT
   <213> Homo sapiens
<400> 64
   Thr Ala Ala Pro Phe Pro Phe His Leu
   1 5
<210> 65
   <211> 9
   <212> PRT
   <213> Homo sapiens
<400> 65
   Asp Thr Leu Asp Lys Val Phe Thr Tyr
   1 5
<210> 66
   <211> 9
   <212> PRT
   <213> Homo sapiens
<400> 66
   Asp Thr Ile Ser Pro Thr Leu Gly Phe
   1 5
<210> 67
   <211> 11
   <212> PRT
   <213> Homo sapiens
<400> 67
   Asp Thr Gly Ile Leu Asp Ser Ile Gly Arg Phe
   1 5 10
<210> 68
   <211> 9
   <212> PRT
   <213> Homo sapiens
<400> 68
   Val Val Tyr Pro Trp Thr Gln Arg Phe
   1 5
<210> 69
   <211> 10
   <212> PRT
   <213> Homo sapiens
<400> 69
   Glu Val Val Ala Gly Ile Lys Glu Tyr Phe
   1 5 10
<210> 70
   <211> 9
   <212> PRT
   <213> Homo sapiens
<400> 70
   Ser Ser Val Pro Gly Val Arg Leu Leu
   1 5
<210> 71
   <211> 11
   <212> PRT
   <213> Homo sapiens
<400> 71
   Ser Val Val Asp Ala Ile Gly Ile Ser Arg Phe
   1 5 10
<210> 72
   <211> 9
   <212> PRT
   <213> Homo sapiens
<400> 72
   Glu Val Ile Pro Pro Met Lys Glu Phe
   1 5
<210> 73
   <211> 9
   <212> PRT
   <213> Homo sapiens
<400> 73
   Glu Val Ile Pro Pro Tyr Tyr Ser Tyr
   1 5
<210> 74
   <211> 9
   <212> PRT
   <213> Homo sapiens
<400> 74
   Glu Val Asn Gly Leu Ile Ser Met Tyr
   1 5
<210> 75
   <211> 10
   <212> PRT
   <213> Homo sapiens
<400> 75
   Glu Val Ile Asp Leu Met Ile Lys Glu Tyr
   1 5 10
<210> 76
   <211> 9
   <212> PRT
   <213> Homo sapiens
<400> 76
   Glu Val Val Ala Gly Ile Lys Glu Tyr
   1 5
<210> 77
   <211> 9
   <212> PRT
   <213> Homo sapiens
<400> 77
   Glu Val Phe Pro Leu Ala Met Asn Tyr
   1 5
<210> 78
   <211> 9
   <212> PRT
   <213> Homo sapiens
<400> 78
   Glu Val Val Glu Arg Val Leu Thr Phe
   1 5
<210> 79
   <211> 10
   <212> PRT
   <213> Homo sapiens
<400> 79
   Ser His Ser Pro Phe Gly Leu Asp Ser Phe
   1 5 10
<210> 80
   <211> 9
   <212> PRT
   <213> Homo sapiens
<400> 80
   Phe Gly Val Asp Arg Ala Ile Leu Tyr
   1 5
<210> 81
   <211> 9
   <212> PRT
   <213> Homo sapiens
<400> 81
   Ser His Ser Asp Tyr Leu Leu Thr Ile
   1 5
<210> 82
   <211> 9
   <212> PRT
   <213> Homo sapiens
<400> 82
   Ser His Leu Asp Tyr Asp Ile Thr Leu
   1 5
<210> 83
   <211> 9
   <212> PRT
   <213> Homo sapiens
<400> 83
   Ser His Phe Val Ser Asp Val Val Ile
   1 5
<210> 84
   <211> 9
   <212> PRT
   <213> Homo sapiens
<400> 84
   Glu Val Thr Glu Leu Leu Ala Arg Tyr
   1 5
<210> 85
   <211> 11
   <212> PRT
   <213> Homo sapiens
<400> 85
   Glu Thr Ala Asp Thr Leu Met Gly Leu Arg Tyr
   1 5 10
<210> 86
   <211> 9
   <212> PRT
   <213> Homo sapiens
<400> 86
   Glu His Ala His Leu Ile Val Val Leu
   1 5
<210> 87
   <211> 9
   <212> PRT
   <213> Homo sapiens
<400> 87
   Glu His Ser Leu Val Ile Asp Thr Leu
   1 5
<210> 88
   <211> 9
   <212> PRT
   <213> Homo sapiens
<400> 88
   Glu Ile Ala Glu Ala Tyr Leu Gly Tyr
   1 5
<210> 89
   <211> 10
   <212> PRT
   <213> Homo sapiens
<400> 89
   Glu Ile Tyr Gly Gly Ser Asp Ser Arg Phe
   1 5 10
<210> 90
   <211> 9
   <212> PRT
   <213> Homo sapiens
<400> 90
   Glu Leu Ile Ala Lys Ile Pro Asn Phe
   1 5
<210> 91
   <211> 9
   <212> PRT
   <213> Homo sapiens
<400> 91
   Glu Val Ile Lys Asn Phe Ile Gln Tyr
   1 5
<210> 92
   <211> 11
   <212> PRT
   <213> Homo sapiens
<400> 92
   Glu Thr Ala Asp Thr Leu Leu Ala Leu Arg Tyr
   1 5 10
<210> 93
   <211> 10
   <212> PRT
   <213> Homo sapiens
<400> 93
   Glu Val Val Ser Glu Pro Phe Arg Ser Phe
   1 5 10
<210> 94
   <211> 10
   <212> PRT
   <213> Homo sapiens
<400> 94
   Glu Thr Phe Asp Ala Gly Leu Gln Ala Phe
   1 5 10
<210> 95
   <211> 9
   <212> PRT
   <213> Homo sapiens
<400> 95
   Ser His Ser Gln Leu Met Gln Leu Ile
   1 5
<210> 96
   <211> 9
   <212> PRT
   <213> Homo sapiens
<400> 96
   Glu Thr Val Arg Glu Leu Thr Glu Phe
   1 5
<210> 97
   <211> 9
   <212> PRT
   <213> Homo sapiens
<400> 97
   Glu Val Ala Ala Thr Glu Ile Lys Met
   1 5
<210> 98
   <211> 9
   <212> PRT
   <213> Homo sapiens
<400> 98
   Glu Val Ala Ala Val Leu Leu His Phe
   1 5
<210> 99
   <211> 9
   <212> PRT
   <213> Homo sapiens
<400> 99
   Glu Val Phe Asp Lys Thr Tyr Gln Phe
   1 5
<210> 100
   <211> 9
   <212> PRT
   <213> Homo sapiens
<400> 100
   Glu Leu Val Lys Arg Ile Leu Asn Phe
   1 5
<210> 101
   <211> 9
   <212> PRT
   <213> Homo sapiens
<400> 101
   Ala His Asp Asp Gly Arg Trp Ser Leu
   1 5
<210> 102
   <211> 9
   <212> PRT
   <213> Homo sapiens
<400> 102
   Ser Val Val Ser Val Ile Ser Arg Phe
   1 5
<210> 103
   <211> 9
   <212> PRT
   <213> Homo sapiens
<400> 103
   Ser Val Val Glu Leu Ile Asn His Tyr
   1 5
<210> 104
   <211> 9
   <212> PRT
   <213> Homo sapiens
<400> 104
   Ser Val Val Asp Leu Ile Asn His Tyr
   1 5
<210> 105
   <211> 9
   <212> PRT
   <213> Homo sapiens
<400> 105
   Ala His Val Asp Leu Ile Glu Lys Leu
   1 5
<210> 106
   <211> 9
   <212> PRT
   <213> Homo sapiens
<400> 106
   Phe His Asn Glu Leu Leu Thr Gln Leu
   1 5
<210> 107
   <211> 9
   <212> PRT
   <213> Homo sapiens
<400> 107
   Ser Val Ile Glu Ala Val Ala His Phe
   1 5
<210> 108
   <211> 9
   <212> PRT
   <213> Homo sapiens
<400> 108
   Gly His Phe Glu Lys Pro Leu Phe Leu
   1 5
<210> 109
   <211> 9
   <212> PRT
   <213> Homo sapiens
<400> 109
   Gly His Asp Ala Ser Gln Ile Thr Leu
   1 5
<210> 110
   <211> 9
   <212> PRT
   <213> Homo sapiens
<400> 110
   Ser Ala Val Asp Phe Ile Arg Thr Leu
   1 5
<210> 111
   <211> 9
   <212> PRT
   <213> Homo sapiens
<400> 111
   Ile Ser Thr Pro Val Ile Arg Thr Phe
   1 5
<210> 112
   <211> 10
   <212> PRT
   <213> Homo sapiens

<400> 112
   Gly Val Ile Glu Lys Leu Leu Thr Ser Tyr
   1 5 10
<210> 113
   <211> 9
   <212> PRT
   <213> Homo sapiens
<400> 113
   Ser His Asp Leu Thr Leu Val Asn Leu
   1 5
<210> 114
   <211> 9
   <212> PRT
   <213> Homo sapiens
<400> 114
   Phe Pro Ser Leu Arg Glu Ala Ala Leu
   1 5
<210> 115 ,
   <211> 9
   <212> PRT
   <213> Homo sapiens
<400> 115
   Ser Ile Phe Lys Gln Pro Val Thr Lys
   1 5
<210> 116
   <211> 9
   <212> PRT
   <213> Homo sapiens
<400> 116
   Lys Pro Asn Ala Asn Arg Ile Ala Leu
   1 5
<210> 117
   <211> 9
   <212> PRT
   <213> Homo sapiens
<400> 117
   Lys Leu Tyr Glu Met Ile Leu Lys Arg
   1 5
<210> 118
   <211> 9
   <212> PRT
   <213> Homo sapiens
<400> 118
   Ser Leu Phe Ser Arg Leu Phe Gly Lys
   1 5
<210> 119
   <211> 9
   <212> PRT
   <213> Homo sapiens
<400> 119
   Lys Leu Phe Asp Lys Leu Leu Glu Tyr
   1 5
<210> 120
   <211> 12
   <212> PRT
   <213> Homo sapiens
<400> 120
   Ser Leu Phe Pro Asn Ser Pro Lys Trp Thr Ser Lys
   1 5 10
<210> 121
   <211> 9
   <212> PRT
   <213> Homo sapiens
<400> 121
   Leu Glu Ser Leu Asp Gln Leu Glu Leu
   1 5
<210> 122
   <211> 10
   <212> PRT
   <213> Homo sapiens
<400> 122
   Val Val Asn Lys Val Pro Leu Thr Gly Lys
   1 5 10
<210> 123
   <211> 9
   <212> PRT
   <213> Homo sapiens
<400> 123
   Ser Val Tyr Asp Ser Val Leu Gln Lys
   1 5
<210> 124
   <211> 9
   <212> PRT
   <213> Homo sapiens
<400> 124
   Ser Val Tyr Val Leu Val Arg Gln Lys
   1 5
<210> 125
   <211> 9
   <212> PRT
   <213> Homo sapiens
<400> 125
   Ile Leu Glu Asn Ile Gln Arg Asn Lys
   1 5
<210> 126
   <211> 10
   <212> PRT
   <213> Homo sapiens
<400> 126
   Gly Ser Tyr Asn Lys Val Phe Leu Ala Lys
   1 5 10
<210> 127
   <211> 9
   <212> PRT
   <213> Homo sapiens
<400> 127
   Thr Glu Ser Gly Leu Asn Val Thr Leu
   1 5
<210> 128
   <211> 9
   <212> PRT
   <213> Homo sapiens
<400> 128
   Thr Glu His Gly Val Glu Val Val Leu
   1 5
<210> 129
   <211> 9
   <212> PRT
   <213> Homo sapiens
<400> 129
   Thr Glu Ala Arg Phe Gly Ala Gln Leu
   1 5
<210> 130
   <211> 9
   <212> PRT
   <213> Homo sapiens
<400> 130
   Thr Leu Ala Asp Ile Leu Leu Tyr Tyr
   1 5
<210> 131
   <211> 10
   <212> PRT
   <213> Homo sapiens
<400> 131
   Leu Val Phe Pro Ser Glu Ile Val Gly Lys
   1 5 10
<210> 132
   <211> 10
   <212> PRT
   <213> Homo sapiens
<400> 132
   Val Leu Phe Gly Lys Ala Leu Asn Pro Lys
   1 5 10
<210> 133
   <211> 9
   <212> PRT
   <213> Homo sapiens
<400> 133
   Arg Pro Glu Leu Val Arg Pro Ala Leu
   1 5
<210> 134
   <211> 10
   <212> PRT
   <213> Homo sapiens
<400> 134
   Val Pro Asn Gln Lys Arg Leu Thr Leu Leu
   1 5 10
<210> 135
   <211> 9
   <212> PRT
   <213> Homo sapiens
<400> 135
   Gln Leu Tyr Trp Ser His Pro Arg Lys
   1 5
<210> 136
   <211> 9
   <212> PRT
   <213> Homo sapiens
<400> 136
   Ser Val Tyr Val Tyr Lys Val Leu Lys
   1 5
<210> 137
   <211> 9
   <212> PRT
   <213> Homo sapiens
<400> 137
   Arg Glu Lys Leu Gln Glu Glu Met Leu
   1 5
<210> 138
   <211> 12
   <212> PRT
   <213> Homo sapiens
<400> 138
   Arg Val Phe Ser Gly Leu Val Ser Thr Gly Leu Lys
   1 5 10
<210> 139
   <211> 10
   <212> PRT
   <213> Homo sapiens
<400> 139
   Lys Pro Arg Asp Val Ser Ser Val Glu Leu
   1 5 10
<210> 140
   <211> 9
   <212> PRT
   <213> Homo sapiens
<400> 140
   Asn Glu Phe Pro Glu Pro Ile Lys Leu
   1 5
<210> 141
   <211> 9
   <212> PRT
   <213> Homo sapiens
<400> 141
   Lys Thr Tyr Gly Glu Ile Phe Glu Lys
   1 5
<210> 142
   <211> 9
   <212> PRT
   <213> Homo sapiens
<400> 142
   Arg Ile Leu Phe Phe Asn Thr Pro Lys
   1 5
<210> 143
   <211> 9
   <212> PRT
   <213> Homo sapiens
<400> 143
   Arg Val Phe Pro Trp Phe Ser Val Lys
   1 5
<210> 144
   <211> 9
   <212> PRT
   <213> Homo sapiens
<400> 144
   Ser Glu Val Gln Asp Arg Val Met Leu
   1 5
<210> 145
   <211> 9
   <212> PRT
   <213> Homo sapiens
<400> 145
   Ser Leu Trp Asp Arg Leu Ile Phe His
   1 5
<210> 146
   <211> 9
   <212> PRT
   <213> Homo sapiens
<400> 146
   Lys Val Tyr Asn Ile Gln Ile Arg Tyr
   1 5
<210> 147
   <211> 9
   <212> PRT
   <213> Homo sapiens
<400> 147
   Arg Leu Leu Glu Met Ile Leu Asn Lys
   1 5
<210> 148
   <211> 9
   <212> PRT
   <213> Homo sapiens
<400> 148
   Ser Glu Asp Lys Lys Asn Ile Ile Leu
   1 5
<210> 149
   <211> 9
   <212> PRT
   <213> Homo sapiens
<400> 149
   Tyr Glu Glu Leu Val Arg Met Val Leu
   1 5
<210> 150
   <211> 9
   <212> PRT
   <213> Homo sapiens
<400> 150
   Gly Glu Ile Thr Gly Glu Val His Met
   1 5
<210> 151
   <211> 9
   <212> PRT
   <213> Homo sapiens
<400> 151
   Ile Val Ala Gly Ser Leu Ile Thr Lys
   1 5
<210> 152
   <211> 12
   <212> PRT
   <213> Homo sapiens
<400> 152
   Ala Pro Arg Ile Ile Thr Gly Pro Ala Pro Val Leu
   1               5                   10
<210> 153
   <211> 10
   <212> PRT
   <213> Homo sapiens
<400> 153
   Gly Leu Ala Ser Phe Lys Ser Phe Leu Lys
   1               5                   10
<210> 154
   <211> 10
   <212> PRT
   <213> Homo sapiens
<400> 154
   Phe Pro Asn Ser Pro Lys Trp Thr Ser Lys
   1               5                   10
<210> 155
   <211> 9
   <212> PRT
   <213> Homo sapiens
<400> 155
   Phe Val Ile Glu Thr Ala Arg Gln Leu
   1               5
<210> 156
   <211> 10
   <212> PRT
   <213> Homo sapiens
<400> 156
   Ile Glu Val Asp Gly Lys Gln Val Glu Leu
   1               5                   10
<210> 157
   <211> 9
   <212> PRT
   <213> Homo sapiens
<400> 157
   Gly Glu Leu Thr Gly Glu Val Arg Met
   1 5
<210> 158
   <211> 10
   <212> PRT
   <213> Homo sapiens
<400> 158
   Gly Glu Ser Asp Asp Ser Ile Leu Arg Leu
   1 5 10
<210> 159
   <211> 12
   <212> PRT
   <213> Homo sapiens
<400> 159
   Gly Glu Gly Asp Phe Leu Ala Glu Gly Gly Gly Val
   1 5 10
<210> 160
   <211> 7
   <212> PRT
   <213> Homo sapiens
<400> 160
   Asp Asn Phe Pro Gln Ser Leu
   1 5
<210> 161
   <211> 9
   <212> PRT
   <213> Homo sapiens
<400> 161
   Gly Leu Thr Asp Val Ile Leu Tyr His
   1 5
<210> 162
   <211> 11
   <212> PRT
   <213> Homo sapiens
<400> 162
   Ala Ala Leu Val Ala Ser Gly Val Ala Leu Tyr
   1 5 10
<210> 163
   <211> 10
   <212> PRT
   <213> Homo sapiens
<400> 163
   Ala Glu Ile Arg His Val Leu Val Thr Leu
   1 5 10
<210> 164
   <211> 9
   <212> PRT
   <213> Homo sapiens
<400> 164
   Ala Glu Pro Glu Glu Val Glu Val Leu
   1 5
<210> 165
   <211> 10
   <212> PRT
   <213> Homo sapiens
<400> 165
   Ala Ile Ile Asp His Ile Phe Ala Ser Lys
   1 5 10

<210> 166
   <211> 11
   <212> PRT
   <213> Homo sapiens
<400> 166
   Ala Leu Leu Asp Gly Ser Asn Val Val Phe Lys
   1               5                   10
<210> 167
   <211> 9
   <212> PRT
   <213> Homo sapiens
<400> 167
   Ala Met Leu Asp Thr Val Val Phe Lys
   1               5
<210> 168
   <211> 9
   <212> PRT
   <213> Homo sapiens
<400> 168
   Ala Pro Ala Arg Leu Phe Ala Leu Leu
   1               5
<210> 169
   <211> 10
   <212> PRT
   <213> Homo sapiens
<400> 169
   Ala Val Asn Ala His Ser Asn Ile Leu Lys
   1               5                   10
<210> 170
   <211> 9
   <212> PRT
   <213> Homo sapiens
<400> 170
   Ala Pro Arg Pro Gly Val Leu Leu Leu
   1               5
<210> 171
   <211> 9
   <212> PRT
   <213> Homo sapiens
<400> 171
   Glu Ala Phe Pro Leu Arg Val Ile Asp
   1 5
<210> 172
   <211> 9
   <212> PRT
   <213> Homo sapiens
<400> 172
   Gly Val Ala Asp Lys Ile Leu Lys Lys
   1 5
<210> 173
   <211> 10
   <212> PRT
   <213> Homo sapiens
<400> 173
   Ala Val Phe Pro Lys Pro Phe Val Glu Lys
   1 5 10
<210> 174
   <211> 10
   <212> PRT
   <213> Homo sapiens
<400> 174
   Val Val Tyr Val Gly Gly Ile Leu Thr Lys
   1 5 10
<210> 175
   <211> 9
   <212> PRT
   <213> Homo sapiens
<400> 175
   His Leu Glu Asp Ile Val Arg Gln Lys
   1 5
<210> 176
   <211> 10
   <212> PRT
   <213> Homo sapiens
<400> 176
   Val Thr Leu Thr Leu Val Ile Leu Ser Tyr
   1 5 10
<210> 177
   <211> 10
   <212> PRT
   <213> Homo sapiens
<400> 177
   Ser Leu Leu Ser Leu Val Thr Gly Leu Lys
   1 5 10
<210> 178
   <211> 9
   <212> PRT
   <213> Homo sapiens
<400> 178
   Gln Thr Tyr Val Gly Ile Thr Glu Lys
   1 5
<210> 179
   <211> 9
   <212> PRT
   <213> Homo sapiens
<400> 179
   His Glu Asp Lys Ile Arg Val Val Leu
   1 5
<210> 180
   <211> 9
   <212> PRT
   <213> Homo sapiens
<400> 180
   Gln Ile Ser Ile Pro Phe Leu Leu Lys
   1 5
<210> 181
   <211> 9
   <212> PRT
   <213> Homo sapiens
<400> 181
   Gly Leu Met Gly Phe Ile Val Tyr Lys
   1 5
<210> 182
   <211> 9
   <212> PRT
   <213> Homo sapiens
<400> 182
   Phe Ala Asp Gln Glu Val Arg Ser Leu
   1 5
<210> 183
   <211> 9
   <212> PRT
   <213> Homo sapiens
<400> 183
   Ile Val Ala Leu Ile Leu Ser Thr Lys
   1 5
<210> 184
   <211> 10
   <212> PRT
   <213> Homo sapiens
<400> 184
   Gly Thr Tyr Ala Pro Ala Glu Val Pro Lys
   1 5 10
<210> 185
   <211> 9
   <212> PRT
   <213> Homo sapiens
<400> 185
   Gly Thr Met Thr Gly Met Leu Tyr Lys
   1 5
<210> 186
   <211> 9
   <212> PRT
   <213> Homo sapiens
<400> 186
   Ser Leu Ala Glu Ile Leu Leu Lys Lys
   1 5
<210> 187
   <211> 9
   <212> PRT '
   <213> Homo sapiens
<400> 187
   Lys Leu Thr Tyr Ile Tyr Ile Gln Lys
   1 5
<210> 188
   <211> 10
   <212> PRT
   <213> Homo sapiens
<400> 188
   Lys Leu Leu Asn Tyr Ala Pro Leu Glu Lys
   1 5 10
<210> 189
   <211> 9
   <212> PRT
   <213> Homo sapiens
<400> 189
   Gly Thr Leu Pro His Pro Leu Gln Arg
   1 5
<210> 190
   <211> 9
   <212> PRT
   <213> Homo sapiens
<400> 190
   Gly Leu Tyr Glu Phe Phe Arg Ala Lys
   1 5
<210> 191
   <211> 9
   <212> PRT
   <213> Homo sapiens
<400> 191
   Lys Glu Pro Glu Ile Asn Thr Thr Leu
   1 5
<210> 192
   <211> 9
   <212> PRT
   <213> Homo sapiens
<400> 192
   His Ala Ser Asp Arg Ile Ile Ala Leu
   1 5
<210> 193
   <211> 9
   <212> PRT
   <213> Homo sapiens
<400> 193
   Arg Pro Thr Leu Trp Ala Ala Ala Leu
   1 5
<210> 194
   <211> 9
   <212> PRT
   <213> Homo sapiens
<400> 194
   Ala Pro Ser Pro Arg Pro Leu Ser Leu
   1 5
<210> 195
   <211> 10
   <212> PRT
   <213> Homo sapiens
<400> 195
   Ala Ser Asp Phe Ile Thr Lys Met Asp Tyr
   1 5 10
<210> 196
   <211> 9
   <212> PRT
   <213> Homo sapiens
<400> 196
   Glu Glu Arg Val Ile Asn Glu Glu Tyr
   1 5
<210> 197
   <211> 10
   <212> PRT
   <213> Homo sapiens
<400> 197
   Ala Thr Gly Ser Trp Asp Ser Phe Leu Lys
   1 5 10
<210> 198
   <211> 9
   <212> PRT
   <213> Homo sapiens
<400> 198
   Arg Met Phe Asp Met Gly Phe Glu Tyr
   1 5
<210> 199
   <211> 8
   <212> PRT
   <213> Homo sapiens
<400> 199
   Ala Pro Leu Leu Arg Trp Val Leu
   1 5
<210> 200
   <211> 11
   <212> PRT
   <213> Homo sapiens
<400> 200
   Ala Leu Arg Pro Ser Thr Ser Arg Ser Leu Tyr
   1 5 10
<210> 201
   <211> 9
   <212> PRT
   <213> Homo sapiens
<400> 201
   Arg Gln Ile Pro Tyr Thr Met Met Lys
   1 5
<210> 202
   <211> 9
   <212> PRT
   <213> Homo sapiens
<400> 202
   Ala Glu Thr His Ile Val Leu Leu Phe
   1 5
<210> 203
   <211> 9
   <212> PRT
   <213> Homo sapiens
<400> 203
   Arg Val His Ala Tyr Ile Ile Ser Tyr
   1 5
<210> 204
   <211> 11
   <212> PRT
   <213> Homo sapiens
<400> 204
   Ala Val Ile Val Leu Val Glu Asn Phe Tyr Lys
   1 5 10
<210> 205
   <211> 9
   <212> PRT
   <213> Homo sapiens
<400> 205
   Ser Glu Glu Leu Leu Arg Glu His Tyr
   1 5
<210> 206
   <211> 9
   <212> PRT
   <213> Homo sapiens
<400> 206
   Arg Ala Asp Gly Asn Phe Leu Leu Tyr
   1 5
<210> 207
   <211> 9
   <212> PRT
   <213> Homo sapiens
<400> 207
   Ser Glu Phe Thr Gly Val Trp Lys Tyr
   1 5
<210> 208
   <211> 9
   <212> PRT
   <213> Homo sapiens
<400> 208
   Ser Ile Asp Arg Thr Val Met Tyr Tyr
   1 5
<210> 209
   <211> 11
   <212> PRT
   <213> Homo sapiens
<400> 209
   Glu Thr Asp Leu Leu Asp Ile Arg Ser Glu Tyr
   1 5 10
<210> 210
   <211> 9
   <212> PRT
   <213> Homo sapiens
<400> 210
   Glu Ser Tyr Glu Ala Leu Pro Gln His
   1 5
<210> 211
   <211> 9
   <212> PRT
   <213> Homo sapiens
<400> 211
   Ser Glu Glu Glu Ile Arg Glu Ala Phe
   1 5
<210> 212
   <211> 9
   <212> PRT
   <213> Homo sapiens
<400> 212
   Lys Val Met Gln Gln Asn Leu Val Tyr
   1 5
<210> 213
   <211> 8
   <212> PRT
   <213> Homo sapiens
<400> 213
   Asp Glu Lys Ser Ile Ile Thr Tyr
   1 5
<210> 214
   <211> 8
   <212> PRT
   <213> Homo sapiens
<400> 214
   Glu Glu Ile Glu Gly Phe Arg Tyr
   1 5
<210> 215
   <211> 9
   <212> PRT
   <213> Homo sapiens
<400> 215
   Met Glu Asn Leu Phe Ile Asn Arg Phe
   1 5
<210> 216
   <211> 9
   <212> PRT
   <213> Homo sapiens
<400> 216
   Met Glu Lys Ile Trp His His Thr Phe
   1 5
<210> 217
   <211> 10
   <212> PRT
   <213> Homo sapiens
<400> 217
   Met Glu His Ala Met Glu Thr Met Met Phe
   1 5 10
<210> 218
   <211> 8
   <212> PRT
   <213> Homo sapiens
<400> 218
   Glu Glu Ile Phe Asn Leu Lys Phe
   1 5
<210> 219
   <211> 9
   <212> PRT
   <213> Homo sapiens
<400> 219
   Leu Val Leu Met Val Leu Tyr Leu Ile
   1 5
<210> 220
   <211> 9
   <212> PRT
   <213> Homo sapiens
<400> 220
   Glu Glu Leu Gln Gln Lys Val Ser Tyr
   1 5
<210> 221
   <211> 11
   <212> PRT
   <213> Homo sapiens
<400> 221
   Leu Arg Val Ala Pro Glu Glu His Pro Val Leu
   1 5 10
<210> 222
   <211> 9
   <212> PRT
   <213> Homo sapiens
<400> 222
   Asp Gly His Leu Phe Gln Val Glu Tyr
   1 5

<210> 223
   <211> 9
   <212> PRT
   <213> Homo sapiens
<400> 223
   Leu Ala Glu Leu Ala His Arg Glu Tyr
   1 5
<210> 224
   <211> 10
   <212> PRT
   <213> Homo sapiens
<400> 224
   Asn Glu Ala Asp Val His Gly Ile Tyr Phe
   1 5 10
<210> 225
   <211> 9
   <212> PRT
   <213> Homo sapiens
<400> 225
   Lys Val Phe Gln Glu Pro Leu Phe Tyr
   1 5
<210> 226
   <211> 9
   <212> PRT
   <213> Homo sapiens
<400> 226
   Gly Val Leu Ala Trp Val Lys Glu Lys
   1 5
<210> 227
   <211> 9
   <212> PRT
   <213> Homo sapiens
<400> 227
   His Glu Ala Leu Leu Tyr Tyr Val Leu
   1 5
<210> 228
   <211> 8
   <212> PRT
   <213> Homo sapiens
<400> 228
   His Glu Met Ile Ile Leu Lys Leu
   1 5
<210> 229
   <211> 9
   <212> PRT
   <213> Homo sapiens
<400> 229
   Ile Val Pro Ala Asn Phe Pro Ser Leu
   1 5
<210> 230
   <211> 9
   <212> PRT
   <213> Homo sapiens
<400> 230
   His Leu Asp Leu Gly Ile Leu Tyr Tyr
   1 5
<210> 231
   <211> 10
   <212> PRT
   <213> Homo sapiens
<400> 231
   Ile Thr Asp Ser Ala Gly His Ile Leu Tyr
   1 5 10
<210> 232
   <211> 10
   <212> PRT
   <213> Homo sapiens
<400> 232
   His Thr Asp Asp Pro Leu Thr Trp Asp Tyr
   1 5 10
<210> 233
   <211> 9
   <212> PRT
   <213> Homo sapiens
<400> 233
   Ile Ala Arg Asn Leu Thr Gln Gln Leu
   1 5
<210> 234
   <211> 9
   <212> PRT
   <213> Homo sapiens
<400> 234
   Ile Asp Gln Thr Ala Leu Ala Val Tyr
   1 5
<210> 235
   <211> 9
   <212> PRT
   <213> Homo sapiens
<400> 235
   Leu Glu Asp Val Val Ile Glu Arg Tyr
   1 5
<210> 236
   <211> 9
   <212> PRT
   <213> Homo sapiens
<400> 236
   Gln Ile Ala Ser Phe Ile Leu Leu Arg
   1 5
<210> 237
   <211> 8
   <212> PRT
   <213> Homo sapiens
<400> 237
   Asp Glu His Tyr Ile Leu Thr Phe
   1 5
<210> 238
   <211> 10
   <212> PRT
   <213> Homo sapiens
<400> 238
   Asp Glu Ile Gly Leu Pro Lys Ile Phe Tyr
   1 5 10
<210> 239
   <211> 9
   <212> PRT
   <213> Homo sapiens
<400> 239
   Asp Glu Ile Val Arg Ile Asn Gly Tyr
   1 5
<210> 240
   <211> 9
   <212> PRT
   <213> Homo sapiens
<400> 240
   Asp Glu Lys Leu Leu Tyr Asp Thr Phe
   1 5
<210> 241
   <211> 10
   <212> PRT
   <213> Homo sapiens
<400> 241
   Arg Ile Ile Glu Glu Thr Leu Ala Leu Lys
   1 5 10
<210> 242
   <211> 9
   <212> PRT
   <213> Homo sapiens
<400> 242
   Gly Thr Asp Glu Leu Arg Leu Leu Tyr
   1 5
<210> 243
   <211> 11
   <212> PRT
   <213> Homo sapiens
<400> 243
   Asp Glu Leu Glu Ile Ile Glu Gly Met Lys Phe
   1 5 10
<210> 244
   <211> 10
   <212> PRT
   <213> Homo sapiens
<400> 244
   Gln Val Asp Pro Leu Ser Ala Leu Lys Tyr
   1 5 10
<210> 245
   <211> 9
   <212> PRT
   <213> Homo sapiens
<400> 245
   Asp Glu Leu His Tyr Leu Glu Val Tyr
   1 5
<210> 246
   <211> 10
   <212> PRT
   <213> Homo sapiens
<400> 246
   Glu Glu Phe Glu Leu Leu Gly Lys Ala Tyr
   1 5 10
<210> 247
   <211> 11
   <212> PRT
   <213> Homo sapiens
<400> 247
   Gln Leu Glu Asp Gly Arg Thr Leu Ser Asp Tyr
   1 5 10
<210> 248
   <211> 9
   <212> PRT
   <213> Homo sapiens
<400> 248
   Asp Glu Phe Leu Trp Arg Glu Gln Phe
   1 5
<210> 249
   <211> 8
   <212> PRT
   <213> Homo sapiens
<400> 249
   Asp Glu Met Leu Ser Arg Gly Phe
   1 5
<210> 250
   <211> 10
   <212> PRT
   <213> Homo sapiens
<400> 250
   Asp Glu Pro Leu Leu Lys His Trp Glu Phe
   1 5 10
<210> 251
   <211> 10
   <212> PRT
   <213> Homo sapiens
<400> 251
   Pro Ser Arg Asp Ser Leu Pro Leu Pro Val
   1 5 10
<210> 252
   <211> 11
   <212> PRT
   <213> Homo sapiens
<400> 252
   Asn Leu Arg Glu Thr Asn Leu Asp Ser Leu Pro
   1 5 10
<210> 253
   <211> 9
   <212> PRT
   <213> Homo sapiens
<400> 253
   Asp Glu Val Lys Phe Leu Thr Val Leu
   1 5
<210> 254
   <211> 9
   <212> PRT
   <213> Homo sapiens
<400> 254
   Asn Glu Val Glu Lys Thr Met Glu Tyr
   1 5
<210> 255
   <211> 10
   <212> PRT
   <213> Homo sapiens
<400> 255
   Asp Glu Val Gln Val Val Arg Gly His Tyr
   1 5 10
<210> 256
   <211> 9
   <212> PRT
   <213> Homo sapiens
<400> 256
   Asp Glu Trp Leu Lys Pro Glu Leu Phe
   1 5
<210> 257
   <211> 9
   <212> PRT
   <213> Homo sapiens
<400> 257
   Asp Glu Tyr Ser Leu Val Arg Glu Leu
   1 5
<210> 258
   <211> 9
   <212> PRT
   <213> Homo sapiens
<400> 258
   Asn Glu Phe Glu Ala Thr Gln Lys Leu
   1 5

<210> 259
   <211> 9
   <212> PRT
   <213> Homo sapiens
<400> 259
   Asp Glu Leu Gln Gln Pro Leu Glu Leu
   1 5
<210> 260
   <211> 11
   <212> PRT
   <213> Homo sapiens
<400> 260
   Asp Val Val Met Thr Gln Ser Pro Leu Ser Leu
   1 5 10
<210> 261
   <211> 9
   <212> PRT
   <213> Homo sapiens
<400> 261
   Ser Glu Arg Glu Ala Ile Glu Val Phe
   1 5
<210> 262
   <211> 9
   <212> PRT
   <213> Homo sapiens
<400> 262
   Arg Tyr Phe Tyr His Gln Glu Glu Tyr
   1 5
<210> 263
   <211> 9
   <212> PRT
   <213> Homo sapiens
<400> 263
   Thr Ser Ala Leu Pro Ile Ile Gln Lys
   1 5
<210> 264
   <211> 11
   <212> PRT
   <213> Homo sapiens
<400> 264
   Arg Val Gln Glu Ala Val Glu Ser Met Val Lys
   1 5 10
<210> 265
   <211> 11
   <212> PRT
   <213> Homo sapiens
<400> 265
   Thr Val Met Glu Leu Val Lys Ile Ile Tyr Lys
   1 5 10
<210> 266
   <211> 9
   <212> PRT
   <213> Homo sapiens
<400> 266
   Arg Leu Leu Gln Lys Val Leu Ala Tyr
   1 5
<210> 267
   <211> 9
   <212> PRT
   <213> Homo sapiens
<400> 267
   Arg Ile His Phe Pro Leu Ala Thr Tyr
   1 5
<210> 268
   <211> 12
   <212> PRT
   <213> Homo sapiens
<400> 268
   Val Gly Gly Leu Lys Asn Thr Leu Val His Arg Leu
   1 5 10
<210> 269
   <211> 10
   <212> PRT
   <213> Homo sapiens
<400> 269
   Gln Ala Gln Ala Asp Ser Leu Thr Val Tyr
   1 5 10
<210> 270
   <211> 9
   <212> PRT
   <213> Homo sapiens
<400> 270
   Val Leu Asp Pro Tyr Leu Leu Lys Tyr
   1 5
<210> 271
   <211> 10
   <212> PRT
   <213> Homo sapiens
<400> 271
   Ile Phe Ser Pro Pro Phe Pro Leu Phe Tyr
   1 5 10
<210> 272
   <211> 9
   <212> PRT
   <213> Homo sapiens
<400> 272
   Thr Glu Leu Leu Leu Lys Glu Gly Phe
   1 5
<210> 273
   <211> 12
   <212> PRT
   <213> Homo sapiens
<400> 273
   Gly Leu Phe Glu Val Gly Ala Gly Trp Ile Gly Lys
   1 5 10
<210> 274
   <211> 9
   <212> PRT
   <213> Homo sapiens
<400> 274
   Tyr Glu Tyr Lys Phe Gly Phe Glu Leu
   1 5
<210> 275
   <211> 9
   <212> PRT
   <213> Homo sapiens
<400> 275
   Trp Pro Leu Trp Arg Leu Val Ser Leu
   1 5
<210> 276
   <211> 9
   <212> PRT
   <213> Homo sapiens
<400> 276
   Tyr Ile Asp Glu Gln Phe Glu Arg Tyr
   1 5
<210> 277
   <211> 11
   <212> PRT
   <213> Homo sapiens
<400> 277
   Tyr Leu Asp Glu Lys Leu Ala Leu Leu Asn Ala
   1 5 10
<210> 278
   <211> 8
   <212> PRT
   <213> Homo sapiens
<400> 278
   Asp Glu His Leu Ile Thr Phe Phe
   1 5
<210> 279
   <211> 8
   <212> PRT
   <213> Homo sapiens
<400> 279
   Asp Asp Phe His Ile Tyr Val Tyr
   1 5
<210> 280
   <211> 9
   <212> PRT
   <213> Homo sapiens
<400> 280
   Ala Pro Arg Thr Val Leu Leu Leu Leu
   1 5
<210> 281
   <211> 11
   <212> PRT
   <213> Homo sapiens
<400> 281
   Ala Pro Arg Thr Val Ala Leu Thr Ala Leu Leu
   1 5 10
<210> 282
   <211> 10
   <212> PRT
   <213> Homo sapiens
<400> 282
   Phe Thr Asp Val Asn Ser Ile Leu Arg Tyr
   1 5 10
<210> 283
   <211> 9
   <212> PRT
   <213> Homo sapiens
<400> 283
   Tyr Ser Glu Glu Glu Cys Arg Gln Tyr
   1 5
<210> 284
   <211> , 9
   <212> PRT
   <213> Homo sapiens
<400> 284
<210> 285
   <211> 10
   <212> PRT
   <213> Homo sapiens
<400> 285
   Tyr Thr Asp Leu Leu Arg Leu Phe Glu Tyr
   1 5 10
<210> 286
   <211> 9
   <212> PRT
   <213> Homo sapiens
<400> 286
   Tyr Val Asp Pro Gln Phe Leu Thr Tyr
   1 5
<210> 287
   <211> 9
   <212> PRT
   <213> Homo sapiens
<400> 287
   His Glu Arg Thr Phe Leu Leu Glu Tyr
   1 5
<210> 288
   <211> 17
   <212> PRT
   <213> Homo sapiens
<400> 288
   Ser Ser Val Pro Gly Val Arg Leu Leu Gln Asp Ser Val Asp Phe Ser
   1 5 10 15
   Leu
<210> 289
   <211> 12
   <212> PRT
   <213> Homo sapiens
<400> 289
   Ser Leu Leu Thr Ser Ser Lys Gly Gln Leu Gln Lys
   1 5 10
<210> 290
   <211> 10
   <212> PRT
   <213> Homo sapiens
<400> 290
   Ser Pro Arg Glu Asn Ile Leu Val Ser Leu
   1 5 10
<210> 291
   <211> 11
   <212> PRT
   <213> Homo sapiens
<400> 291
   Asp Glu Val Asp Ile Lys Ser Arg Ala Ala Tyr
   1 5 10
<210> 292
   <211> 9
   <212> PRT
   <213> Homo sapiens
<400> 292
   Thr Ser Pro Ser Gln Ser Leu Phe Tyr
   1 5
<210> 293
   <211> 10
   <212> PRT
   <213> Homo sapiens
<400> 293
   Tyr Thr Glu Thr Glu Pro Tyr His Asn Tyr
   1 5 10
<210> 294
   <211> 15
   <212> PRT
   <213> Homo sapiens
<400> 294
   Ser Ser Val Pro Gly Val Arg Leu Leu Gln Asp Ser Val Asp Phe
   1 5 10 15
<210> 295
   <211> 9
   <212> PRT
   <213> Homo sapiens
<400> 295
   Val Ala Leu Ile Ser Pro Lys Asp Ile
   1 5
<210> 296
   <211> 10
   <212> PRT
   <213> Homo sapiens
<400> 296
   Ser Thr Asp Lys Ala Glu Tyr Thr Phe Tyr
   1 5 10
<210> 297
   <211> 9
   <212> PRT
   <213> Homo sapiens
<400> 297
   Val Thr Glu Ile Phe Arg Gln Ala Phe
   1 5
<210> 298
   <211> 9
   <212> PRT
   <213> Homo sapiens
<400> 298
   Ser Val Leu Ser Pro Leu Leu Asn Lys
   1 5
<210> 299
   <211> 10
   <212> PRT
   <213> Homo sapiens
<400> 299
   Arg Ala Phe Ser Ser Leu Gly Leu Leu Lys
   1 5 10
<210> 300
   <211> 10
   <212> PRT
   <213> Homo sapiens
<400> 300
   Phe Ser Lys Leu Arg Pro Leu Ile Ser Lys
   1 5 10
<210> 301
   <211> 9
   <212> PRT
   <213> Homo sapiens
<400> 301
   Arg Thr Phe Thr Trp Leu Val Gly Lys
   1 5
<210> 302
   <211> 9
   <212> PRT
   <213> Homo sapiens
<400> 302
   Lys Val Ala Asn Ile Ile Leu Ser Tyr
   1 5
<210> 303
   <211> 9
   <212> PRT
   <213> Homo sapiens
<400> 303
   Thr Met Leu Ala Arg Leu Ala Ser Ala
   1 5
<210> 304
   <211> 9
   <212> PRT
   <213> Homo sapiens
<400> 304
   His Glu Leu Pro Leu Pro His Ser Val
   1 5
<210> 305
   <211> 9
   <212> PRT
   <213> Homo sapiens
<400> 305
   Ala Val Gln Arg Thr Leu Leu Glu Lys
   1 5
<210> 306
   <211> 13
   <212> PRT
   <213> Homo sapiens
<400> 306
   Glu Thr Arg Pro Ala Gly Asp Gly Thr Phe Gln Lys Trp
   1 5 10
<210> 307
   <211> 12
   <212> PRT
   <213> Homo sapiens
<400> 307
   Ala Val Leu Ser Ile Leu Pro Ala Ile Phe Gln Lys
   1 5 10
<210> 308
   <211> 9
   <212> PRT
   <213> Homo sapiens
<400> 308
   Glu Ile Ala Gly His Ile Met Glu Phe
   1 5
<210> 309
   <211> 9
   <212> PRT
   <213> Homo sapiens
<400> 309
   Glu Leu Ile Arg Thr Ile Met Gly Trp
   1 5
<210> 310
   <211> 10
   <212> PRT
   <213> Homo sapiens
<400> 310
   Glu Val Phe Pro Leu Lys Val Phe Gly Tyr
   1 5 10
<210> 311
   <211> 10
   <212> PRT
   <213> Homo sapiens
<400> 311
   Ala Thr Pro Thr Ser Pro Ile Arg Val Lys
   1 5 10
<210> 312
   <211> 10
   <212> PRT
   <213> Homo sapiens
<400> 312
   Ala Val Leu Tyr Gln Pro Leu Phe Asp Lys
   1 5 10
<210> 313
   <211> 10
   <212> PRT
   <213> Homo sapiens
<400> 313
   Glu Val Val Asp Phe Ile Gln Ser Lys Ile
   1 5 10
<210> 314
   <211> 11
   <212> PRT
   <213> Homo sapiens
<400> 314
   Ala Val Gln Glu Phe Gly Leu Ala Arg Phe Lys
   1 5 10
<210> 315
   <211> 9
   <212> PRT
   <213> Homo sapiens
<400> 315
   Glu Ala Ile Gln Asp Leu Trp Gln Trp
   1 5
<210> 316
   <211> 9
   <212> PRT
   <213> Homo sapiens
<400> 316
   Gly Val Ile Arg Ser Leu Met Ala Phe
   1 5
<210> 317
   <211> 10
   <212> PRT
   <213> Homo sapiens
<400> 317
   His Ile Ile Ser Gly Thr Cys Ala Ser Trp
   1 5 10
<210> 318
   <211> 10
   <212> PRT
   <213> Homo sapiens
<400> 318
   Gly Val Ile Asp Val Ile Thr Lys Thr Trp
   1 5 10
<210> 319
   <211> 9
   <212> PRT
   <213> Homo sapiens
<400> 319
   Gly Val Ile Asp Leu Ile Phe Glu Lys
   1 5
<210> 320
   <211> 9
   <212> PRT
   <213> Homo sapiens
<400> 320
   Gly Val Cys His Ile Phe Ala Ser Phe
   1 5
<210> 321
   <211> 9
   <212> PRT
   <213> Homo sapiens
<400> 321
   Gly Thr Tyr Val Ser Ser Val Pro Arg
   1 5
<210> 322
   <211> 11
   <212> PRT
   <213> Homo sapiens
<400> 322
   Gly Thr Ala Gly Leu Leu Glu Gln Trp Leu Lys
   1 5 10
<210> 323
   <211> 10
   <212> PRT
   <213> Homo sapiens
<400> 323
   His Val Ile Thr Gly Leu Leu Glu His Tyr
   1 5 10
<210> 324
   <211> 11
   <212> PRT
   <213> Homo sapiens
<400> 324
   Gly Thr Ala Asp Glu Leu Val Leu His Ser Trp
   1 5 10
<210> 325
   <211> 9
   <212> PRT
   <213> Homo sapiens
<400> 325
   Glu Ile Lys Glu Val Ile Leu Glu Phe
   1 5
<210> 326
   <211> 9
   <212> PRT
   <213> Homo sapiens
<400> 326
   Glu Glu Ala Ser Leu Leu His Gln Phe
   1 5
<210> 327
   <211> 9
   <212> PRT
   <213> Homo sapiens
<400> 327
   Lys Leu Phe Ile Gly Gly Leu Ser Phe
   1 5
<210> 328
   <211> 9
   <212> PRT
   <213> Homo sapiens
<400> 328
   Asp Val Val Pro Ala Val Arg Lys Trp
   1 5
<210> 329
   <211> 9
   <212> PRT
   <213> Homo sapiens
<400> 329
   Asp Val Thr Gly Val Val Arg Gln Trp
   1 5
<210> 330
   <211> 9
   <212> PRT
   <213> Homo sapiens
<400> 330
   Asp Val Lys Asp Tyr Ile Gln Glu Tyr
   1 5
<210> 331
   <211> 11
   <212> PRT
   <213> Homo sapiens
<400> 331
   Asp Val Ile Asp Asn Asp Ser Trp Arg Leu Trp
   1 5 10
<210> 332
   <211> 11
   <212> PRT
   <213> Homo sapiens
<400> 332
   Asp Val Phe Ser Ser Lys Gly Met Thr Arg Trp
   1 5 10
<210> 333
   <211> 9
   <212> PRT
   <213> Homo sapiens
<400> 333
   Asp Thr Val Lys Lys Ile Glu Ser Phe
   1 5
<210> 334
   <211> 11
   <212> PRT
   <213> Homo sapiens
<400> 334
   Asp Leu Pro Ser Asn His Val Ile Asp Arg Trp
   1 5 10
<210> 335
   <211> 9
   <212> PRT
   <213> Homo sapiens
<400> 335
   Asp Leu Ile Gly His Ile Val Glu Phe
   1 5
<210> 336
   <211> 9
   <212> PRT
   <213> Homo sapiens
<400> 336
   Asp Lys Glu Ser Gln Leu Glu Ala Tyr
   1 5
<210> 337
   <211> 11
   <212> PRT
   <213> Homo sapiens
<400> 337
   Glu Val Ile Lys Leu Lys Gly Tyr Thr Ser Trp
   1 5 10
<210> 338
   <211> 9
   <212> PRT
   <213> Homo sapiens
<400> 338
   Gly Ser Ser Asp Val Ile Ile His Arg
   1               5
<210> 339
   <211> 9
   <212> PRT
   <213> Homo sapiens
<400> 339
   Gly Thr Leu Asp Tyr Ile Leu Gln Arg
   1               5
<210> 340
   <211> 10
   <212> PRT
   <213> Homo sapiens
<400> 340
   Glu Val Asp Lys Arg Val His Met Thr Trp
   1               5                   10
<210> 341
   <211> 10
   <212> PRT
   <213> Homo sapiens
<400> 341
   Ser Val Pro Tyr Phe Leu Phe Gln His Trp
   1               5                   10
<210> 342
   <211> 11
   <212> PRT
   <213> Homo sapiens
<400> 342
   Ser Val Glu Glu Ile Ser Thr Leu Val Gln Lys
   1               5                   10

<210> 343
   <211> 10
   <212> PRT
   <213> Homo sapiens
<400> 343
   Ser Thr Phe Gln Gln Met Trp Ile Ser Lys
   1 5 10
<210> 344
   <211> 10
   <212> PRT
   <213> Homo sapiens
<400> 344
   Thr Thr Ile Pro His Ala Leu Leu Thr Trp
   1 5 10
<210> 345
   <211> 10
   <212> PRT
   <213> Homo sapiens
<400> 345
   Ser Ala Phe Leu Leu Leu Gly Leu Phe Lys
   1 5 10
<210> 346
   <211> 11
   <212> PRT
   <213> Homo sapiens
<400> 346
   Asn Ile Gly Asp Glu Ala Leu Ile Gly Arg Trp
   1 5 10
<210> 347
   <211> 10
   <212> PRT
   <213> Homo sapiens
<400> 347
   Thr Val Ala Phe Val Pro Ile Ser Gly Trp
   1 5 10
<210> 348
   <211> 10
   <212> PRT
   <213> Homo sapiens
<400> 348
   Glu Thr Val Asn Leu Arg Ser Leu Gly Phe
   1 5 10
<210> 349
   <211> 9
   <212> PRT
   <213> Homo sapiens
<400> 349
   Met Pro Lys Phe Ser Met Pro Gly Phe
   1 5
<210> 350
   <211> 9
   <212> PRT
   <213> Homo sapiens
<400> 350
   Glu Val Met Glu Ile Met Ser Arg Phe
   1 5
<210> 351
   <211> 9
   <212> PRT
   <213> Homo sapiens
<400> 351
   Glu Val Met Asp Val Phe Leu Arg Phe
   1 5
<210> 352
   <211> 9
   <212> PRT
   <213> Homo sapiens
<400> 352
   Arg Leu Gln Glu Ala Leu Asn Leu Phe
   1 5
<210> 353
   <211> 11
   <212> PRT
   <213> Homo sapiens
<400> 353
   Glu Thr Ile Asp Trp Lys Val Phe Glu Ser Trp
   1 5 10
<210> 354
   <211> 10
   <212> PRT
   <213> Homo sapiens
<400> 354
   Glu Leu Met Glu His Gly Val Val Ser Trp
   1 5 10
<210> 355
   <211> 9
   <212> PRT
   <213> Homo sapiens
<400> 355
   Ala Ser Val Ala Trp Ala Val Leu Lys
   1 5
<210> 356
   <211> 9
   <212> PRT
   <213> Homo sapiens
<400> 356
   Ser Val Ser Pro Val Val His Val Arg
   1 5
<210> 357
   <211> 10
   <212> PRT
   <213> Homo sapiens
<400> 357
   His Val Val Asp Arg Asp Thr Glu Ala Trp
   1 5 10
<210> 358
   <211> 9
   <212> PRT
   <213> Homo sapiens
<400> 358
   Glu Thr Ile Thr Gly Leu Arg Val Trp
   1 5
<210> 359
   <211> 10
   <212> PRT
   <213> Homo sapiens
<400> 359
   Arg Gln Leu Glu Asp Ile Leu Ser Thr Tyr
   1 5 10
<210> 360
   <211> 11
   <212> PRT
   <213> Homo sapiens
<400> 360
   Ala Ile Ala Gln Ala Glu Ser Leu Arg Tyr Lys
   1 5 10
<210> 361
   <211> 11
   <212> PRT
   <213> Homo sapiens
<400> 361
   Gly Val Leu Gln Leu Gly Asn Ile Val Phe Lys
   1 5 10
<210> 362
   <211> 10
   <212> PRT
   <213> Homo sapiens
<400> 362
   Glu Val Ile Asn Ala Leu Lys Gln Thr Trp
   1 5 10
<210> 363
   <211> 9
   <212> PRT
   <213> Homo sapiens
<400> 363
   Ser Thr Ala Ala Phe Phe Leu Leu Arg
   1 5
<210> 364
   <211> 10
   <212> PRT
   <213> Homo sapiens
<400> 364
   Asp Ile Tyr Asn Phe Pro Ile His Ala Phe
   1 5 10
<210> 365
   <211> 10
   <212> PRT
   <213> Homo sapiens
<400> 365
   Thr Val Val Glu Arg Met Leu Ser Asn Trp
   1 5 10
<210> 366
   <211> 11
   <212> PRT
   <213> Homo sapiens
<400> 366
   Thr Lys Pro Trp Phe Ala Ser Gln Ile Pro Phe
   1 5 10
<210> 367
   <211> 9
   <212> PRT
   <213> Homo sapiens
<400> 367
   Gly Arg Val Asp Phe Ala Tyr Lys Phe
   1 5
<210> 368
   <211> 9
   <212> PRT
   <213> Homo sapiens
<400> 368
   Gly Arg Asp Leu Thr Asp Tyr Leu Met
   1 5
<210> 369
   <211> 10
   <212> PRT
   <213> Homo sapiens
<400> 369
   Gly Arg Ile Ser Ile Thr Gly Val Gly Phe
   1 5 10
<210> 370
   <211> 9
   <212> PRT
   <213> Homo sapiens
<400> 370
   Gly Arg Ile Val Thr Leu Ile Ser Phe
   1 5
<210> 371
   <211> 10
   <212> PRT
   <213> Homo sapiens
<400> 371
   Gly Arg Leu Asp Leu Gln Tyr Ala Lys Leu
   1 5 10
<210> 372
   <211> 9
   <212> PRT
   <213> Homo sapiens
<400> 372
   Gly Arg Thr Asn Leu Ile Val Asn Tyr
   1 5
<210> 373
   <211> 9
   <212> PRT
   <213> Homo sapiens
<400> 373
   Arg Tyr Phe Asp Thr Ala Val Ser Arg
   1 5
<210> 374
   <211> 9
   <212> PRT
   <213> Homo sapiens
<400> 374
   Gly Arg Met Val Gln Val His Glu Leu
   1 5
<210> 375
   <211> 11
   <212> PRT
   <213> Homo sapiens
<400> 375
   Phe Leu Asp Ala Ser Gly Ala Lys Leu Asp Tyr
   1 5 10
<210> 376
   <211> 9
   <212> PRT
   <213> Homo sapiens
<400> 376
   Ala Thr Asp Tyr His Val Arg Val Tyr
   1               5
<210> 377
   <211> 9
   <212> PRT
   <213> Homo sapiens
<400> 377
   Ala Arg Leu Pro Trp Ala Gly Gln Leu
   1               5
<210> 378
   <211> 8
   <212> PRT
   <213> Homo sapiens
<400> 378
   Tyr Gly Met Pro Arg Gln Ile Leu
   1               5
<210> 379
   <211> 9
   <212> PRT
   <213> Homo sapiens
<400> 379
   Gly Arg Leu Leu Val Ala Thr Thr Phe
   1               5
<210> 380
   <211> 9
   <212> PRT
   <213> Homo sapiens
<400> 380
   Ala Gly Gly Asp Trp Phe Thr Ser Arg
   1               5
<210> 381
   <211> 10
   <212> PRT
   <213> Homo sapiens
<400> 381
   Gly Arg Ala Pro Ile Ser Asn Pro Gly Met
   1 5 10
<210> 382
   <211> 10
   <212> PRT
   <213> Homo sapiens
<400> 382
   Gly Arg Met Glu Asn Leu Ala Ser Tyr Arg
   1 5 10
<210> 383
   <211> 9
   <212> PRT
   <213> Homo sapiens
<400> 383
   Val Leu Pro Lys Ser Arg Val Glu Leu
   1 5
<210> 384
   <211> 9
   <212> PRT
   <213> Homo sapiens
<400> 384
   Asp Ala Lys Ile Arg Ile Phe Asp Leu
   1 5
<210> 385
   <211> 10
   <212> PRT
   <213> Homo sapiens
<400> 385
   Gly Arg Ala Met Val Ala Arg Leu Gly Leu
   1 5 10
<210> 386
   <211> 9
   <212> PRT
   <213> Homo sapiens
<400> 386
   Phe Ile Asp Ala Ser Arg Leu Val Tyr
   1 5
<210> 387
   <211> 9
   <212> PRT
   <213> Homo sapiens
<400> 387
   Asp Pro Met Lys Ala Arg Val Val Leu
   1 5
<210> 388
   <211> 9
   <212> PRT
   <213> Homo sapiens
<400> 388
   Phe Arg Phe Asp Pro Gln Phe Ala Leu
   1 5
<210> 389
   <211> 9
   <212> PRT
   <213> Homo sapiens
<400> 389
   Asp Thr Asp His Tyr Phe Leu Arg Tyr
   1 5
<210> 390
   <211> 9
   <212> PRT
   <213> Homo sapiens
<400> 390
   Glu Leu Leu Ile Arg Lys Leu Pro Phe
   1 5
<210> 391
   <211> 8
   <212> PRT
   <213> Homo sapiens
<400> 391
   Glu Ala Phe Val Arg His Ile Leu
   1 5
<210> 392
   <211> 9
   <212> PRT
   <213> Homo sapiens
<400> 392
   Arg Tyr Phe Asp Thr Ala Met Ser Arg
   1 5
<210> 393
   <211> 9
   <212> PRT
   <213> Homo sapiens
<400> 393
   Gly Arg Val Phe Ile Ile Ser Lys Tyr
   1 5
<210> 394
   <211> 11
   <212> PRT
   <213> Homo sapiens
<400> 394
   Thr Phe Arg Pro Ala Ala Met Leu Val Glu Arg
   1 5 10
<210> 395
   <211> 9
   <212> PRT
   <213> Homo sapiens
<400> 395
   Tyr Leu Leu Glu Lys Ser Arg Ala Ile
   1 5
<210> 396
   <211> 9
   <212> PRT
   <213> Homo sapiens
<400> 396
   Leu Ser Asp Leu Gly Lys Leu Ser Tyr
   1 5
<210> 397
   <211> 9
   <212> PRT
   <213> Homo sapiens
<400> 397
   Val Thr Asp Ser Ile Arg Asp Glu Tyr
   1 5
<210> 398
   <211> 9
   <212> PRT
   <213> Homo sapiens
<400> 398
   Leu Thr Asp Arg Glu Leu Glu Glu Tyr
   1 5
<210> 399
   <211> 9
   <212> PRT
   <213> Homo sapiens
<400> 399
   Leu Thr Asp Arg Gly Val Met Ser Tyr
   1 5
<210> 400
   <211> 9
   <212> PRT
   <213> Homo sapiens
<400> 400
   Lys Gly Leu Ser Val Phe Leu Asn Arg
   1 5
<210> 401
   <211> 9
   <212> PRT
   <213> Homo sapiens
<400> 401
   Val Thr Asp Asn Arg Ala Phe Gly Tyr
   1 5
<210> 402
   <211> 11
   <212> PRT
   <213> Homo sapiens
<400> 402
   Ser Thr Asp Val Ser Asp Leu Leu His Gln Tyr
   1 5 10
<210> 403
   <211> 9
   <212> PRT
   <213> Homo sapiens
<400> 403
   Arg Ser Leu Pro Phe Phe Ser Ala Arg
   1 5
<210> 404
   <211> 9
   <212> PRT
   <213> Homo sapiens
<400> 404
   Tyr Arg Phe Met Gly Thr Glu Ala Tyr
   1 5
<210> 405
   <211> 9
   <212> PRT
   <213> Homo sapiens
<400> 405
   Met Pro Leu Leu Arg Gln Glu Glu Leu
   1 5
<210> 406
   <211> 9
   <212> PRT
   <213> Homo sapiens
<400> 406
   Val Thr Glu Ile Asp Gln Asp Lys Tyr
   1 5
<210> 407
   <211> 9
   <212> PRT
   <213> Homo sapiens
<400> 407
   Met Arg His Leu Gly Ala Phe Leu Phe
   1 5
<210> 408
   <211> 10
   <212> PRT
   <213> Homo sapiens
<400> 408
   Thr Thr Glu Glu Ser Leu Arg Asn Tyr Tyr
   1 5 10
<210> 409
   <211> 9
   <212> PRT
   <213> Homo sapiens
<400> 409
   Met Arg Thr Ser Tyr Leu Leu Leu Phe
   1 5
<210> 410
   <211> 9
   <212> PRT
   <213> Homo sapiens
<400> 410
   Thr Val Asp Gln Val Lys Asp Leu Tyr
   1 5
<210> 411
   <211> 9
   <212> PRT
   <213> Homo sapiens
<400> 411
   Met Arg Tyr Val Ala Ser Tyr Leu Leu
   1 5
<210> 412
   <211> 9
   <212> PRT
   <213> Homo sapiens
<400> 412
   Val Gly Leu Ile Arg Asn Leu Ala Leu
   1 5
<210> 413
   <211> 9
   <212> PRT
   <213> Homo sapiens
<400> 413
   Gly Arg Leu Asp Ala Val Leu Gln Arg
   1 5
<210> 414
   <211> 10
   <212> PRT
   <213> Homo sapiens
<400> 414
   Leu Leu Asp Gln Gly Gln Leu Asn Lys Tyr
   1 5 10
<210> 415
   <211> 10
   <212> PRT
   <213> Homo sapiens
<400> 415
   Asn Arg Phe Ala Gly Phe Gly Ile Gly Leu
   1 5 10
<210> 416
   <211> 10
   <212> PRT
   <213> Homo sapiens
<400> 416
   Lys Arg Leu Gly Thr Leu Val Val Thr Tyr
   1 5 10
<210> 417
   <211> 9
   <212> PRT
   <213> Homo sapiens
<400> 417
   Lys Arg Gly Asp Val Ile Tyr Ile Leu
   1 5
<210> 418
   <211> 9
   <212> PRT
   <213> Homo sapiens
<400> 418
   Ser Arg Phe Asp Ile Pro Leu Gly Leu
   1 5
<210> 419
   <211> 10
   <212> PRT
   <213> Homo sapiens
<400> 419
   Ser Thr Asp Pro Ser Val Leu Gly Lys Tyr
   1 5 10
<210> 420
   <211> 9
   <212> PRT
   <213> Homo sapiens
<400> 420
   Ser Arg Phe Leu Lys Ser Asp Leu Phe
   1 5
<210> 421
   <211> 9
   <212> PRT
   <213> Homo sapiens
<400> 421
   Val Gln Lys Pro Ser Tyr Tyr Val Arg
   1 5
<210> 422
   <211> 10
   <212> PRT
   <213> Homo sapiens
<400> 422
   Ser Arg Ile Ser Leu Pro Leu Pro Asn Phe
   1 5 10
<210> 423
   <211> 9
   <212> PRT
   <213> Homo sapiens
<400> 423
   Leu Arg Ser Gly Leu Pro Leu Leu Leu
   1 5
<210> 424
   <211> 9
   <212> PRT
   <213> Homo sapiens
<400> 424
   Ser Phe Lys Asp Tyr Ile Gln Glu Arg
   1 5
<210> 425
   <211> 11
   <212> PRT
   <213> Homo sapiens
<400> 425
   His Thr Gln Gly Pro Val Asp Gly Ser Leu Tyr
   1 5 10
<210> 426
   <211> 10
   <212> PRT
   <213> Homo sapiens
<400> 426
   Ser Thr Asp Lys Phe Lys Thr Asp Phe Tyr
   1 5 10
<210> 427
   <211> 9
   <212> PRT
   <213> Homo sapiens
<400> 427
   Gly Ser His Ser Met Arg Tyr Phe Phe
   1 5
<210> 428
   <211> 10
   <212> PRT
   <213> Homo sapiens
<400> 428
   Gly Ser His Ser Met Arg Tyr Phe Phe Thr
   1 5 10
<210> 429
   <211> 9
   <212> PRT
   <213> Homo sapiens
<400> 429
   Gly Ser His Ser Met Arg Tyr Phe His
   1 5
<210> 430
   <211> 9
   <212> PRT
   <213> Homo sapiens
<400> 430
   Ala Ala Ile Leu Gly Met His Asn Leu
   1 5
<210> 431
   <211> 9
   <212> PRT
   <213> Homo sapiens
<400> 431
   Lys Leu Asp Pro Thr Lys Thr Thr Leu
   1 5

<210> 432
   <211> 9
   <212> PRT
   <213> Homo sapiens
<400> 432
   Phe Val His Asp Leu Val Leu Tyr Leu
   1 5
<210> 433
   <211> 7
   <212> PRT
   <213> Homo sapiens
<400> 433
   Phe Val His Asp Leu Val Leu
   1 5
<210> 434
   <211> 9
   <212> PRT
   <213> Homo sapiens
<400> 434
   Val Leu Ile Pro Lys Leu Pro Gln Leu
   1 5
<210> 435
   <211> 9
   <212> PRT
   <213> Homo sapiens
<400> 435
   Asn Glu Ile Thr Ile Pro Val Thr Phe
   1 5
<210> 436
   <211> 9
   <212> PRT
   <213> Homo sapiens
<400> 436
   Tyr Leu Ala Asp Phe Leu Leu Thr Lys
   1 5
<210> 437
   <211> 9
   <212> PRT
   <213> Homo sapiens
<400> 437
   Tyr Leu Ile Pro Leu Leu Glu Arg Leu
   1 5
<210> 438
   <211> 8
   <212> PRT
   <213> Homo sapiens
<400> 438
   Asn Glu Val Val Thr Arg Glu Tyr
   1 5
<210> 439
   <211> 9
   <212> PRT
   <213> Homo sapiens
<400> 439
   Asp Glu Phe Lys Ile Gly Glu Leu Phe
   1 5
<210> 440
   <211> 11
   <212> PRT
   <213> Homo sapiens
<400> 440
   Ile Gln Arg Thr Pro Lys Ile Gln Val Tyr Ser
   1 5 10
<210> 441
   <211> 9
   <212> PRT
   <213> Homo sapiens
<400> 441
   Leu Thr Gly Pro Val Met Pro Val Arg
   1 5
<210> 442
   <211> 9
   <212> PRT
   <213> Homo sapiens
<400> 442
   Ala Val Ala Ile Lys Ala Met Ala Lys
   1 5
<210> 443
   <211> 8
   <212> PRT
   <213> Homo sapiens
<400> 443
   Phe Val Gln Met Met Thr Ala Lys
   1 5
<210> 444
   <211> 9
   <212> PRT
   <213> Homo sapiens
<400> 444
   Ala Thr Asp Pro Asn Ile Leu Gly Arg
   1 5
<210> 445
   <211> 9
   <212> PRT
   <213> Homo sapiens
<400> 445
   Leu Leu Leu Leu Ser Ile Val Ile Leu
   1 5
<210> 446
   <211> 10
   <212> PRT
   <213> Homo sapiens
<400> 446
   Lys Leu Pro Asn Phe Gly Phe Val Val Phe
   1 5 10
<210> 447
   <211> 9
   <212> PRT
   <213> Homo sapiens
<400> 447
   Lys Leu Ser Glu Ile Asp Val Ala Leu
   1 5
<210> 448
   <211> 9
   <212> PRT
   <213> Homo sapiens
<400> 448
   Leu Ala Ala Leu Pro His Ser Cys Leu
   1 5
<210> 449
   <211> 11
   <212> PRT
   <213> Homo sapiens
<400> 449
   Tyr Ser Ile Ile Thr Pro Asn Ile Leu Arg Leu
   1 5 10
<210> 450
   <211> 10
   <212> PRT
   <213> Homo sapiens
<400> 450
   Ala Leu Pro Ser Arg Ile Leu Leu Trp Lys
   1 5 10
<210> 451
   <211> 12
   <212> PRT
   <213> Homo sapiens
<400> 451
   Val Lys Gly Phe Tyr Pro Ser Asp Ile Ala Val Glu
   1 5 10
<210> 452
   <211> 9
   <212> PRT
   <213> Homo sapiens
<400> 452
   Phe Leu Leu Asp Leu Ser Arg Ser Val
   1 5
<210> 453
   <211> 9
   <212> PRT
   <213> Homo sapiens
<400> 453
   Ile Ile Tyr Lys Gly Gly Thr Ser Arg
   1 5
<210> 454
   <211> 9
   <212> PRT
   <213> Homo sapiens
<400> 454
   Ile Val Ala Asp His Val Ala Ser Tyr
   1 5
<210> 455
   <211> 11
   <212> PRT
   <213> Homo sapiens
<400> 455
   Glu Val Gly Gly Glu Ala Leu Gly Arg Leu Leu
   1 5 10
<210> 456
   <211> 10
   <212> PRT
   <213> Homo sapiens
<400> 456
   Arg Thr Gly Pro Pro Met Gly Ser Arg Phe
   1 5 10
<210> 457
   <211> 9
   <212> PRT
   <213> Homo sapiens
<400> 457
   Arg Gln Ile Gln Glu Ser Val Thr Phe
   1 5
<210> 458
   <211> 9
   <212> PRT
   <213> Homo sapiens
<400> 458
   Arg Val Ala Pro Glu Glu His Pro Val
   1 5
<210> 459
   <211> 9
   <212> PRT
   <213> Homo sapiens
<400> 459
   Thr Leu Ala Asp Leu Leu Ala Leu Arg
   1 5
<210> 460
   <211> 12
   <212> PRT
   <213> Homo sapiens
<400> 460
   Arg Val Ala Pro Glu Glu His Pro Val Leu Leu Thr
   1 5 10
<210> 461
   <211> 9
   <212> PRT
   <213> Homo sapiens
<400> 461
   Thr Leu Ala Asp Ile Ile Ala Arg Leu
   1 5
<210> 462
   <211> 10
   <212> PRT
   <213> Homo sapiens
<400> 462
   Arg Trp Glu Asp Gly Ser Pro Leu Asn Phe
   1 5 10
<210> 463
   <211> 8
   <212> PRT
   <213> Homo sapiens
<400> 463
   Tyr Glu Val Ser Gln Leu Lys Asp
   1 5
<210> 464
   <211> 10
   <212> PRT
   <213> Homo sapiens
<400> 464
   Tyr Arg Asp Ile Pro Glu Leu Gln Gly Phe
   1 5 10
<210> 465
   <211> 10
   <212> PRT
   <213> Homo sapiens
<400> 465
   Tyr Val Asp Gly Thr Gln Phe Val Arg Phe
   1 5 10
<210> 466
   <211> 9
   <212> PRT
   <213> Homo sapiens
<400> 466
   Ser Leu Leu Asp Glu Phe Tyr Lys Leu
   1 5
<210> 467
   <211> 9
   <212> PRT
   <213> Homo sapiens
<400> 467
   His Gly Ile Asp Pro Thr Gly Thr Tyr
   1 5
<210> 468
   <211> 13
   <212> PRT
   <213> Homo sapiens
<400> 468
   Ser Leu Asp Lys Phe Leu Ala Ser Val Ser Thr Val Leu
   1 5 10
<210> 469
   <211> 10
   <212> PRT
   <213> Homo sapiens
<400> 469
   Ser Ile Gly Glu Arg Asp Leu Ile Phe His
   1 5 10
<210> 470
   <211> 9
   <212> PRT
   <213> Homo sapiens
<400> 470
   Ser Ile Thr Ser Val Phe Ile Thr Lys
   1 5
<210> 471
   <211> 11
   <212> PRT
   <213> Homo sapiens
<400> 471
   Phe Gly Glu His Leu Leu Glu Ser Asp Leu Phe
   1 5 10
<210> 472
   <211> 9
   <212> PRT
   <213> Homo sapiens
<400> 472
   Phe Leu Asp Pro Ile Lys Ala Tyr Leu
   1 5
<210> 473
   <211> 11
   <212> PRT
   <213> Homo sapiens
<400> 473
   Phe Leu Ala Asp Pro Ser Ala Phe Val Ala Ala
   1 5 10
<210> 474
   <211> 9
   <212> PRT
   <213> Homo sapiens
<400> 474
   Ile Thr Ala Pro Pro Ser Arg Val Leu
   1 5
<210> 475
   <211> 10
   <212> PRT
   <213> Homo sapiens
<400> 475
   Val Leu Asp Glu Leu Lys Asn Met Lys Cys
   1 5 10
<210> 476
   <211> 9
   <212> PRT
   <213> Homo sapiens
<400> 476
   Leu Leu Gly Pro Arg Leu Val Leu Ala
   1 5
<210> 477
   <211> 9
   <212> PRT
   <213> Homo sapiens
<400> 477
   Ile Ile Met Pro His Asn Ile Tyr Leu
   1 5
<210> 478
   <211> 8
   <212> PRT
   <213> Homo sapiens
<400> 478
   Leu Val Arg Met Val Leu Asn Gly
   1 5
<210> 479
   <211> 10
   <212> PRT
   <213> Homo sapiens
<400> 479
   Arg Leu Tyr Gly Pro Ser Ser Val Ser Phe
   1 5 10
<210> 480
   <211> 9
   <212> PRT
   <213> Homo sapiens
<400> 480
   Phe Glu Ala Pro Ile Lys Leu Val Phe
   1 5
<210> 481
   <211> 9
   <212> PRT
   <213> Homo sapiens
<400> 481
   Ile Gln Pro Gly Ala Val Lys Val Tyr
   1 5
<210> 482
   <211> 9
   <212> PRT
   <213> Homo sapiens
<400> 482
   Val Leu Ala Glu Val Pro Thr Gln Leu
   1 5
<210> 483
   <211> 9
   <212> PRT
   <213> Homo sapiens
<400> 483
   Ile Met Arg Ala Gly Met Ser Ser Leu
   1 5
<210> 484
   <211> 12
   <212> PRT
   <213> Homo sapiens
<400> 484
   Val Glu Phe Ser Ser Gly Leu Lys Gly Met Ser Leu
   1 5 10
<210> 485
   <211> 11
   <212> PRT
   <213> Homo sapiens
<400> 485
   Ile Leu Asn Pro Asp Asn Ser Phe Glu Ile Leu
   1 5 10
<210> 486
   <211> 9
   <212> PRT
   <213> Homo sapiens
<400> 486
   Val Ala Leu Glu Phe Ala Leu His Leu
   1 5
<210> 487
   <211> 9
   <212> PRT
   <213> Homo sapiens
<400> 487
   Thr Val Ala Val Pro Leu Val Gly Lys
   1 5
<210> 488
   <211> 9
   <212> PRT
   <213> Homo sapiens
<400> 488
   Thr Leu Ser Asp Leu Arg Val Tyr Leu
   1 5
<210> 489
   <211> 9
   <212> PRT
   <213> Homo sapiens
<400> 489
   Thr Leu Ile Asp Ile Met Thr Arg Phe
   1 5
<210> 490
   <211> 9
   <212> PRT
   <213> Homo sapiens
<400> 490
   His Asp Phe Pro Arg Ala Leu Ile Phe
   1 5
<210> 491
   <211> 8
   <212> PRT
   <213> Homo sapiens
<400> 491
   Gly Ser His Ser Met Arg Tyr Phe
   1 5
<210> 492
   <211> 10
   <212> PRT
   <213> Homo sapiens
<400> 492
   Ser Leu Met Asp His Thr Ile Pro Glu Val
   1 5 10
<210> 493
   <211> 11
   <212> PRT
   <213> Homo sapiens
<400> 493
   Ser Gly Val His Thr Phe Pro Ala Val Leu Gln
   1 5 10
<210> 494
   <211> 9
   <212> PRT
   <213> Homo sapiens
<400> 494
   Phe Leu Val Thr Val Ile His Thr Leu
   1 5
<210> 495
   <211> 8
   <212> PRT
   <213> Homo sapiens
<400> 495
   Thr Asp Gly Lys Val Phe Gln Phe
   1 5
<210> 496
   <211> 9
   <212> PRT
   <213> Homo sapiens
<400> 496
   Tyr Asp Leu Leu Arg Asn Thr Asn Phe
   1 5
<210> 497
   <211> 9
   <212> PRT
   <213> Homo sapiens
<400> 497
   Ile Leu Tyr Pro Lys Thr Leu Phe Leu
   1 5
<210> 498
   <211> 8
   <212> PRT
   <213> Homo sapiens
<400> 498
   Met Arg Tyr Val Ala Ser Tyr Leu
   1 5
<210> 499
   <211> 9
   <212> PRT
   <213> Homo sapiens
<400> 499
   Phe Ile Trp Glu Asn Ile His Thr Leu
   1 5
<210> 500
   <211> 9
   <212> PRT
   <213> Homo sapiens
<400> 500
   Arg Glu Leu Pro Ala Trp Val Ser Phe
   1 5
<210> 501
   <211> 9
   <212> PRT
   <213> Homo sapiens
<400> 501
   Gln Asp Leu Asn Arg Ile Phe Pro Leu
   1 5
<210> 502
   <211> 8
   <212> PRT
   <213> Homo sapiens
<400> 502
   Arg Asp Ser Ile Val Ala Glu Leu
   1 5
<210> 503
   <211> 9
   <212> PRT
   <213> Homo sapiens
<400> 503
   Ala Asp Val Leu Lys Val Glu Val Phe
   1 5
<210> 504
   <211> 8
   <212> PRT
   <213> Homo sapiens
<400> 504
   Tyr Asp Ser Ile Ile Tyr Arg Met
   1 5
<210> 505
   <211> 9
   <212> PRT
   <213> Homo sapiens
<400> 505
   Ala Met Asn Pro Val Glu His Pro Phe
   1 5
<210> 506
   <211> 9
   <212> PRT
   <213> Homo sapiens
<400> 506
   Ser Glu Leu Ile Arg Asn Val Thr Leu
   1 5
<210> 507
   <211> 9
   <212> PRT
   <213> Homo sapiens
<400> 507
   Gln Asp Val Ala Arg Val Leu Gly Phe
   1 5
<210> 508
   <211> 9
   <212> PRT
   <213> Homo sapiens
<400> 508
   Ser Asp His Ile His Ile Ile Ala Leu
   1 5
<210> 509
   <211> 9
   <212> PRT
   <213> Homo sapiens
<400> 509
   Ala Asp Ser Leu Arg Leu Gln Gln Leu
   1 5
<210> 510
   <211> 8
   <212> PRT
   <213> Homo sapiens
<400> 510
   Leu Leu Asp Ile Arg Ser Glu Tyr
   1 5

<210> 511
   <211> 9
   <212> PRT
   <213> Homo sapiens
<400> 511
   Val Leu Phe Gly Leu Leu Arg Glu Val
   1 5
<210> 512
   <211> 9
   <212> PRT
   <213> Homo sapiens
<400> 512
   Val Ala Val Gly Arg Ala Leu Tyr Tyr
   1 5
<210> 513
   <211> 9
   <212> PRT
   <213> Homo sapiens
<400> 513
   Met Arg Phe Leu Ala Ala Thr Phe Leu
   1 5
<210> 514
   <211> 9
   <212> PRT
   <213> Homo sapiens
<400> 514
   Tyr Thr Asp Pro Glu Val Phe Lys Tyr
   1 5
<210> 515
   <211> 9
   <212> PRT
   <213> Homo sapiens
<400> 515
   His Asp Phe Leu Lys Tyr Asp Phe Phe
   1 5
<210> 516
   <211> 9
   <212> PRT
   <213> Homo sapiens
<400> 516
   Ala Ile Asp Gln Leu His Leu Glu Tyr
   1 5
<210> 517
   <211> 9
   <212> PRT
   <213> Homo sapiens
<400> 517
   Ser Asp Leu Glu Arg Val Thr Ser Leu
   1 5
<210> 518
   <211> 9
   <212> PRT
   <213> Homo sapiens
<400> 518
   Thr Leu Leu Pro Leu Arg Val Phe Leu
   1 5
<210> 519
   <211> 10
   <212> PRT
   <213> Homo sapiens
<400> 519
   Tyr Ser Ile Ile Thr Pro Asn Ile Leu Arg
   1 5 10
<210> 520
   <211> 9
   <212> PRT
   <213> Homo sapiens
<400> 520
   Phe Glu Leu Gln Arg Asn Phe Gln Leu
   1 5
<210> 521
   <211> 9
   <212> PRT
   <213> Homo sapiens
<400> 521
   Leu Asp Leu Gln Arg Asn Tyr Ile Phe
   1 5
<210> 522
   <211> 9
   <212> PRT
   <213> Homo sapiens
<400> 522
   Arg Arg Leu Asp Pro Ile Pro Gln Leu
   1 5
<210> 523
   <211> 12
   <212> PRT
   <213> Homo sapiens
<400> 523
   Ser Leu Pro Ile Lys Glu Ser Glu Ile Ile Asp Phe
   1 5 10
<210> 524
   <211> 9
   <212> PRT
   <213> Homo sapiens
<400> 524
   Thr Glu Leu Leu Arg Tyr Tyr Met Leu
   1 5
<210> 525
   <211> 10
   <212> PRT
   <213> Homo sapiens
<400> 525
   Phe Ile Tyr His Gly Glu Val Pro Gln Ala
   1 5 10
<210> 526
   <211> 9
   <212> PRT
   <213> Homo sapiens
<400> 526
   Ala Glu Met Leu Arg Ser Ile Ser Phe
   1 5
<210> 527
   <211> 10
   <212> PRT
   <213> Homo sapiens
<400> 527
   Arg Leu Gln Glu Asp Pro Pro Val Gly Val
   1 5 10
<210> 528
   <211> 9
   <212> PRT
   <213> Homo sapiens
<400> 528
   Ala Glu Leu Glu Arg Ala Ala Ala Leu
   1 5
<210> 529
   <211> 8
   <212> PRT
   <213> Homo sapiens
<400> 529
   Tyr Thr Asp Lys Ile Asp Arg Tyr
   1 5
<210> 530
   <211> 9
   <212> PRT
   <213> Homo sapiens
<400> 530
   Phe Leu Leu Pro Asp Val Ile Arg Ile
   1 5
<210> 531
   <211> 9
   <212> PRT
   <213> Homo sapiens
<400> 531
   Val Glu Leu Pro His Ile Asn Leu Leu
   1 5
<210> 532
   <211> 9
   <212> PRT
   <213> Homo sapiens
<400> 532
   Val Met Leu Asp Val Pro Ile Arg Leu
   1 5
<210> 533
   <211> 9
   <212> PRT
   <213> Homo sapiens
<400> 533
   Ser Leu Leu Glu Asn Leu Glu Lys Ile
   1 5
<210> 534
   <211> 9
   <212> PRT
   <213> Homo sapiens.
<400> 534
   Tyr Ala Asp Pro Val Asn Ala His Tyr
   1 5
<210> 535
   <211> 9
   <212> PRT
   <213> Homo sapiens
<400> 535
   Ala Glu Leu Leu Arg Gly Leu Ser Leu
   1 5
<210> 536
   <211> 9
   <212> PRT
   <213> Homo sapiens
<400> 536
   Thr Thr Glu Val His Pro Glu Leu Tyr
   1 5
<210> 537
   <211> 9
   <212> PRT
   <213> Homo sapiens
<400> 537
   Arg Glu Thr Asn Leu Asp Ser Leu Pro
   1 5
<210> 538
   <211> 9
   <212> PRT
   <213> Homo sapiens
<400> 538
   Glu Leu Glu Asp Ser Thr Leu Arg Tyr
   1 5
<210> 539
   <211> 8
   <212> PRT
   <213> Homo sapiens
<400> 539
   Phe Leu Asp Ile Tyr Ile Phe Leu
   1 5
<210> 540
   <211> 9
   <212> PRT
   <213> Homo sapiens
<400> 540
   Thr Tyr Thr Asp Arg Val Phe Phe Leu
   1 5
<210> 541
   <211> 10
   <212> PRT
   <213> Homo sapiens
<400> 541
   Ser Pro His Leu Ala Asn Tyr Phe Tyr Phe
   1 5 10
<210> 542
   <211> 9
   <212> PRT
   <213> Homo sapiens
<400> 542
   Ser Pro Arg Leu Pro Val Gly Gly Phe
   1 5
<210> 543
   <211> 10
   <212> PRT
   <213> Homo sapiens
<400> 543
   Lys Leu Leu Asp Lys Val Gln Ala Tyr Ser
   1 5 10
<210> 544
   <211> 9
   <212> PRT
   <213> Homo sapiens
<400> 544
   Ala Tyr Gln His Leu Phe Tyr Leu Leu
   1 5
<210> 545
   <211> 10
   <212> PRT
   <213> Homo sapiens
<400> 545
   Lys Tyr Ile Leu Leu Met Asp Ile Ile Ala
   1 5 10
<210> 546
   <211> 9
   <212> PRT
   <213> Homo sapiens
<400> 546
   Arg Tyr Ser Ser Met Ala Ala Ser Phe
   1 5
<210> 547
   <211> 10
   <212> PRT
   <213> Homo sapiens
<400> 547
   Ser Pro Arg Ala Ala Glu Pro Val Gln Leu
   1 5 10
<210> 548
   <211> 9
   <212> PRT
   <213> Homo sapiens
<400> 548
   Ile Tyr Thr Ser Ser Val Asn Arg Leu
   1 5
<210> 549
   <211> 9
   <212> PRT
   <213> Homo sapiens
<400> 549
   Leu Tyr Pro Gln Phe Met Phe His Leu
   1 5
<210> 550
   <211> 9
   <212> PRT
   <213> Homo sapiens
<400> 550
   Arg Tyr Ile Pro Thr Ala Ala Ala Phe
   1 5
<210> 551
   <211> 9
   <212> PRT
   <213> Homo sapiens
<400> 551
   Glu Tyr Ile Val Lys Lys Ile Pro Val
   1 5
<210> 552
   <211> 9
   <212> PRT
   <213> Homo sapiens
<400> 552
   Ser Arg Val Glu Ala Val Tyr Val Leu
   1 5
<210> 553
   <211> 9
   <212> PRT
   <213> Homo sapiens
<400> 553
   Met Pro Arg Gly Val Val Val Thr Leu
   1 5
<210> 554
   <211> 9
   <212> PRT
   <213> Homo sapiens
<400> 554
   Leu Pro Lys Pro Pro Gly Arg Gly Val
   1 5
<210> 555
   <211> 9
   <212> PRT
   <213> Homo sapiens
<400> 555
   Arg Leu Trp Gly Glu Pro Val Asn Leu
   1 5
<210> 556
   <211> 9
   <212> PRT
   <213> Homo sapiens
<400> 556
   Arg Leu Leu Asp Val Leu Ala Pro Leu
   1 5
<210> 557
   <211> 9
   <212> PRT
   <213> Homo sapiens
<400> 557
   Leu Tyr Ile Leu Ser Ser His Asp Ile
   1 5
<210> 558
   <211> 9
   <212> PRT
   <213> Homo sapiens
<400> 558
   Thr Pro Met Gly Pro Gly Arg Thr Val
   1 5
<210> 559
   <211> 14
   <212> PRT
   <213> Homo sapiens
<400> 559
   Gly Pro Pro Gly Thr Gly Lys Thr Asp Val Ala Val Gln Ile
   1 5 10
<210> 560
   <211> 10
   <212> PRT
   <213> Homo sapiens
<400> 560
   Asn Glu Ile Glu Asp Thr Phe Arg Gln Phe
   1 5 10
<210> 561
   <211> 10
   <212> PRT
   <213> Homo sapiens
<400> 561
   Glu Glu Ile Asp Leu Arg Ser Val Gly Trp
   1 5 10
<210> 562
   <211> 9
   <212> PRT
   <213> Homo sapiens
<400> 562
   Lys Tyr Gln Lys Gly Phe Ser Leu Trp
   1 5
<210> 563
   <211> 9
   <212> PRT
   <213> Homo sapiens
<400> 563
   Val Tyr Pro Asp Gly Ile Arg His Ile
   1 5
<210> 564
   <211> 9
   <212> PRT
   <213> Homo sapiens
<400> 564
   Lys Phe Ile Asp Thr Thr Ser Lys Phe
   1 5
<210> 565
   <211> 9
   <212> PRT
   <213> Homo sapiens
<400> 565
   Phe Leu Asp Ile Leu Asn Thr Leu Ile
   1 5
<210> 566
   <211> 11
   <212> PRT
   <213> Homo sapiens
<400> 566
   Lys Tyr Ile Thr Gln Gly Gln Leu Leu Gln Phe
   1 5 10
<210> 567
   <211> 10
   <212> PRT
   <213> Homo sapiens
<400> 567
   Lys Tyr Leu Ser Val Gln Gly Gln Leu Phe
   1 5 10
<210> 568
   <211> 9
   <212> PRT
   <213> Homo sapiens
<400> 568
   Arg Tyr Phe Asp Glu Pro Val Glu Leu
   1 5

<210> 569
   <211> 9
   <212> PRT
   <213> Homo sapiens
<400> 569
   Lys Tyr Asp Glu Ile Phe Tyr Asn Leu
   1 5
<210> 570
   <211> 9
   <212> PRT
   <213> Homo sapiens
<400> 570
   Ser Tyr Ile Glu His Ile Phe Glu Ile
   1 5
<210> 571
   <211> 10
   <212> PRT
   <213> Homo sapiens
<400> 571
   Lys Phe Ile Asp Pro Ile Tyr Gln Val Trp
   1 5 10
<210> 572
   <211> 11
   <212> PRT
   <213> Homo sapiens
<400> 572
   Leu Gly Tyr Thr Glu Gly Ala Leu Leu Ala Leu
   1 5 10
<210> 573
   <211> 9
   <212> PRT
   <213> Homo sapiens
<400> 573
   Lys Tyr Pro Ser Pro Phe Phe Val Phe
   1 5
<210> 574
   <211> 10
   <212> PRT
   <213> Homo sapiens
<400> 574
   Glu Tyr Pro Asp Arg Ile Met Asn Thr Phe
   1 5 10
<210> 575
   <211> 9
   <212> PRT
   <213> Homo sapiens
<400> 575
   Val Tyr Ile Ser Glu His Glu His Phe
   1 5
<210> 576
   <211> 9
   <212> PRT
   <213> Homo sapiens
<400> 576
   Lys Tyr Phe Leu Lys Pro Glu Val Leu
   1 5
<210> 577
   <211> 9
   <212> PRT
   <213> Homo sapiens
<400> 577
   Gly Pro Ala Leu Gly Arg Ser Phe Leu
   1 5
<210> 578
   <211> 10
   <212> PRT
   <213> Artificial Sequence
<223> abgeleitet von Melan-A, Position 26-35, modifiziert durch einen Aminosäureaustausch des an Position 27 befindlichen Alanin durch Leucin
<400> 578
   Glu Leu Ala Gly Ile Gly Ile Leu Thr Val
   1 5 10
<210> 579
   <211> 9
   <212> PRT
   <213> Human immunodeficiency virus type 1
<400> 579
   Ile Leu Lys Glu Pro Val His Gly Val
   1 5
<210> 580
   <211> 9
   <212> PRT
   <213> Influenza virus
<400> 580
   Gly Ile Leu Gly Phe Val Phe Thr Leu
   1 5
<210> 581
   <211> 9
   <212> PRT
   <213> Human cytomegalovirus
<400> 581
   Asn Leu Val Pro Met Val Ala Thr Val
   1 5
<210> 582
   <211> 10
   <212> PRT
   <213> Human herpesvirus 4
<400> 582
   Leu Leu Asp Phe Val Arg Phe Met Gly Val
   1 5 10
<210> 583
   <211> 9
   <212> PRT
   <213> Human herpesvirus 4
<400> 583
   Gly Leu Cys Thr Leu Val Ala Met Leu
   1 5
<210> 584
   <211> 9
   <212> PRT
   <213> Human herpesvirus 4
<400> 584
   Cys Leu Gly Gly Leu Leu Thr Met Val
   1 5
<210> 585
   <211> 9
   <212> PRT
   <213> Homo sapiens
<400> 585
   Ala Pro Arg Thr Val Ala Leu Thr Ala
   1 5